# EUROPEAN PATENT APPLICATION

(11) **EP 1 477 803 A1**
(43) Date of publication of application: **17.11.2004**
(21) Application number: 03090153.2
(22) Date of filing: 23.05.2003
(51) Int. Cl.: G01N 33/48, G01N 33/50

(54) **Serum protein profiling for the diagnosis of epithelial cancers**

(30) Priority: 15.05.2003 EP 03090141
(71) Applicant: Europroteome AG, 16761 Hennigsdorf (DE)
(72) Inventor: Ebert, Matthias, 39116 Magdeburg (DE); Wiemar, Jan, 69124 Heidelberg (DE); Meuer, Jörn, 13189 Berlin (DE)
(74) Representative: Ziebig, Marlene K., Dr.

(57) **Abstract**

The present invention provides biomolecules and the use of these biomolecules for the differential diagnosis of epithelial cancers or an acute and chronic inflammation of the epithelium. In particular the present invention provides methods for detecting biomolecules within a test sample as well as a database comprising of mass profiles of biomolecules specific for healthy subjects, subjects having a precancerous lesion, subjects having an epithelial cancer or a metastasised epithelial cancer or subjects having an acute and chronic inflammation of the epithelium. Furthermore, the present invention provides methods for the characterization of said biomolecules using gas phase ion spectrometry. In addition, the present invention provides methods for the identification of said biomolecules provided that they are proteins or polypeptides. The invention further provides kits for the differential diagnosis of epithelial cancers or an acute and chronic inflammation of the epithelium.

## Description

The present invention provides biomolecules and the use of these biomolecules for the differential diagnosis of epithelial cancers or an acute and chronic inflammation of the epithelium. In specific embodiments, the biomolecules are characterised by mass profiles generated by contacting a test and/or biological sample with an anion exchange surface under specific binding conditions and detecting said biomolecules using gas phase ion spectrometry. The biomolecules used according to the invention are preferably proteins or polypeptides. Furthermore, test and/or biological samples are blood serum samples and are of human origin.

### BACKGROUND TO THE INVENTION

Despite major efforts made by the medical and pharmaceutical communities to provide effective therapies for patients suffering from a variety of epithelial cancers, the prognosis of such diseases still remains a great challenge for clinicians and oncologists. For example, colorectal cancer is the fourth most common cancer and the second most common cause of cancer death in the US. Approximately 130.000 new cases and 55.000 deaths per year are reported. Worldwide approximately 875.000 individuals have developed colorectal cancer in 1996, which accounts for approx. 8.5% of all new cases of cancer worldwide (Ref: Potter JD, J Natl Cancer Inst 1999;91:916-932).

The reasons for the poor prognoses of a number of epithelial cancers are combinatorial: the proper diagnosis of such diseases in their advanced stages combined with inadequate therapeutic options. Thus apart from surgery, which may be curative in early stages of such cancers, all other treatment modalities including chemotherapy and radiation are disappointing. The identification of early stages of epithelial cancers and the screening of high-risk individuals, therefore present the current strategies that would greatly improve the overall prognosis of such diseases. Unfortunately, despite intensive research over the last decades, no valid serum markers for epithelial cancers have been identified.

With the development of new technologies in genomic and proteomic analysis of human cancers, there has been great hope that these techniques might not only contribute to our understanding of these diseases, but may also help to develop diagnostic markers that could assist the practicing clinician in the management of epithelial cancers such as breast, gastrointestinal and other epithelial cancers. The standard method of proteome analysis has been two dimensional (2D) gel electrophoresis, which is an invaluable tool for the separation and identification of proteins. This method is also an effective tool for the identification of aberrantly expressed proteins in a variety of tissue samples. Unfortunately, the analysis of data generated by 2D-gel electrophoresis is labour-intensive and requires large quantities of material for protein analysis, thereby rendering it impractical for routine clinical use.

Through the introduction of mass spectrometry (MS), this tool has been achieved. This approach was further improved through the introduction of MALDI-TOF (matrix-assisted laser desorption ionization/time of flight), which is a mass spectrometry technique that allows the simultaneous analysis of multiple proteins in one sample. In combination with tandem mass spectrometry micro-sequencing, differentially expressed proteins can be easily identified. A further modification of MALDI-TOF is SELDI (surface enhanced laser desorption ionization) in which small amounts of proteins are directly bound to a biochip, carrying spots with different types of chromatographic material, including those with hydrophobic, hydrophilic, cation-exchanging and anion-exchanging characteristics. This approach has been proven to be very useful to identify proteins and protein patterns (Serum protein fingerprinting) in various biological fluids, including serum, urine or pancreatic juice.

Recently, Ciphergen Biosystems, Inc. have developed diagnostic tools based on such an approach for the diagnosis of human breast (WO0223200) and prostate cancers (WO0125791). Using this approach, new markers specific for breast and prostate cancer have been identified, providing the medical community with an improved method for the non-invasive diagnoses of breast or prostate cancer in patients. However, a major drawback of these diagnostic tools is the inability to determine if a patient has one type of a cancer over another (e.g. breast vs. colon cancer or lung cancer), as well as the inability to differentiate between an acute inflammatory disease that exhibits symptoms similar to those stemming from a specific cancer or a cancer itself (e.g. pancreatitis vs. pancreatic cancer). As a result of this, patients may still be provided with inadequate treatment for their particular disease, a difficulty currently being battled by many clinicians and oncologists.

Despite Ciphergen's efforts in identifying new markers specific for certain epithelial cancers, there is still a need to develop a non-invasive diagnostic tool that is able to provide the practicing clinician with a method of determining if a patient is suffering from a specific epithelial cancer (e.g. colon cancer) or an acute and chronic inflammation of the epithelium (e.g. pancreatitis or colitis), and to identify specific cancers at early stages of development.

The present invention addresses this difficulty with the development of a non-invasive diagnostic tool for the differential diagnosis of a variety of epithelial cancers, as well as the differential diagnosis of acute inflammatory diseases over their cancerous counterparts (e.g. pancreatitis vs. pancreatic cancer).

### SUMMARY OF THE INVENTION

The present invention relates to methods for the differential diagnosis of epithelial cancers or an acute and chronic inflammation of the epithelium by detecting one or more differentially expressed biomolecules within a test sample of a given subject, comparing results with samples from healthy subjects, subjects having a precancerous lesion, subjects having an epithelial cancer, subjects having a metastasised epithelial cancer, or subjects having an acute and chronic inflammation of the epithelium, wherein the comparison allows for the differential diagnosis of a subject as healthy, having a precancerous lesion, having an epithelial cancer, having a metastasised epithelial cancer or an acute and chronic inflammation of the epithelium.

The present invention provides a method for the differential diagnosis of an epithelial cancer and/or an acute and chronic inflammation of the epithelium, *in vitro,* comprising obtaining a test sample from a subject, contacting test sample with a biologically active surface under specific binding conditions, allowing for biomolecules present within the test sample to bind to the biologically active surface, detecting one or more bound biomolecules using mass spectrometry thereby generating a mass profile of said test sample, transforming data into a computer-readable form, and comparing said mass profile against a database containing mass profiles specific for healthy subjects, subjects having precancerous lesions, subjects having epithelial cancer, subjects having metastasised epithelial cancers, or subjects having an acute and chronic inflammation of the epithelium, wherein the comparison allows for the differential diagnosis of a subject as healthy, having a precancerous lesion, having an epithelial cancer, having a metastasised epithelial cancer or an acute and chronic inflammation of the epithelium.

In one embodiment the invention provides a database comprising of mass profiles of biological samples from healthy subjects, subjects having a precancerous lesion, subjects having an epithelial cancer, subjects having a metastasised epithelial cancer, or subjects having an acute and chronic inflammation of the epithelium.

Within the same embodiment the database is generated by obtaining biological samples from healthy subjects, subjects having a precancerous lesion, subjects having an epithelial cancer, subjects having a metastasised epithelial cancer, and subjects having an acute and chronic inflammation of the epithelium, contacting said biological samples with a biologically active surface under specific binding conditions, allowing the biomolecules within the biological sample to bind to said biologically active surface, detecting one or more bound biomolecules using mass spectrometry thereby generating a mass profile of said biological samples, transforming data into a computer-readable form, and applying a mathematical algorithm to classify the mass profiles as specific for healthy subjects, subjects having precancerous lesions, subjects having epithelial cancer, subjects having metastasised epithelial cancers, and subjects having an acute and chronic inflammation of the epithelium.

In specific embodiments, the present invention provides biomolecules having a molecular mass selected from the group consisting of 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ± 70 Da, 15959 Da ± 80 Da, 16164 Da ± 81 Da, 17279 Da ± 86 Da, 17406 Da ± 87 Da, 17630 Da ± 88 Da, or 18133 Da ± 91 Da. The biomolecules having said molecular masses are detected by contacting a test and/or biological sample with a biologically active surface comprising an adsorbent under specific binding conditions and further analysed by gas phase ion spectrometry. Preferably the adsorbent used is comprised of positively charged quaternary ammonium groups (anion exchange surface).

In specific embodiments, the invention provides specific binding conditions for the detection of biomolecules within a sample. In preferred embodiments, a sample is diluted 1:5 in a denaturation buffer consisting of 7 M urea, 2 M thiourea, 4% CHAPS, 1% DTT, and 2% Ampholine, and then diluted again 1:10 in binding buffer consisting of 0.1 M Tis-HCl, 0.02% Triton X-100 at a pH 8.5 at 0 to 4°C. The treated sample is then contacted with a biologically active surface comprising of positively charged (cationic) quaternary ammonium groups (anion exchanging), incubated for 120 minutes at 20 to 24°C, and the bound biomolecules are detected using gas phase ion spectrometry.

In an alternative embodiment, the invention provides a method for the differential diagnosis of an epithelial cancer and/or an acute and chronic inflammation of the epithelium comprising detecting of one or more differentially expressed biomolecules within a sample. This method comprises obtaining a test sample from a subject, contacting said sample with a binding molecule specific for a differentially expressed polypeptide, detecting an interaction between the binding molecule and its specific polypeptide, wherein the detection of an interaction indicates the presence or absence of said polypeptide, thereby allowing for the differential diagnosis of a subject as healthy, having a precancerous lesion, having an epithelial cancer, having a metastasised epithelial cancer and/or an acute and chronic inflammation of the epithelium. Preferably, binding molecules are antibodies specific for said polypeptides.

The biomolecules related to the invention, having a molecular mass selected from the group consisting of 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ± 70 Da, 15959 Da ± 80 Da, 16164 Da ± 81 Da, 17279 Da ± 86 Da, 17406 Da ± 87 Da, 17630 Da ± 88 Da, or 18133 Da ± 91 Da, and may include, but are not limited to, molecules comprising nucleotides, amino acids, sugars, fatty acids, steroids, nucleic acids, polynucleotides (DNA or RNA), polypeptides, proteins, antibodies, carbohydrates, lipids, and combinations thereof (e.g., glycoproteins, ribonucleoproteins, lipoproteins). Preferably said biomolecules are proteins, polypeptides, or fragments thereof.

In yet another embodiment, the invention provides a method for the identification of biomolecules within a sample, provided that the biomolecules are proteins, polypeptides or fragments thereof, comprising: chromatography and fractionation, analysis of fractions for the presence of said differentially expressed proteins and/or fragments thereof, using a biologically active surface, further analysis using mass spectrometry to obtain amino acid sequences encoding said proteins and/or fragments thereof, and searching amino acid sequence databases of known proteins to identify said differentially expressed proteins by amino acid sequence comparison. Preferably the method of chromatography is high performance liquid chromatography (HPLC) or fast protein liquid chromatography (FPLC). Furthermore, the mass spectrometry used is selected from the group of matrix-assisted laser desorption ionization/time of flight (MALDI-TOF), surface enhanced laser desorption ionisation/time of flight (SELDI-TOF), liquid chromatography, MS-MS, or ESI-MS.

Furthermore, the invention provides kits for the differential diagnosis of an epithelial cancer and/or an acute and chronic inflammation of the epithelium.

The test or biological samples used according to the invention may be of blood, blood serum, plasma, nipple aspirate, urine, semen, seminal fluid, seminal plasma, prostatic fluid, excreta, tears, saliva, sweat, biopsy, ascites, cerebrospinal fluid, milk, lymph, or tissue extract origin. Preferably, the test and/or biological samples are blood serum samples, and are isolated from subjects of mammalian origin, preferably of human origin.

An epithelial cancer of the invention may be a breast, lung, gastrointestinal, prostate, ovarian, cervical, endometrial, bladder and/or other cancer of epithelial origin, and may be of various stages and/or grades.

### DESCRIPTION OF FIGURES

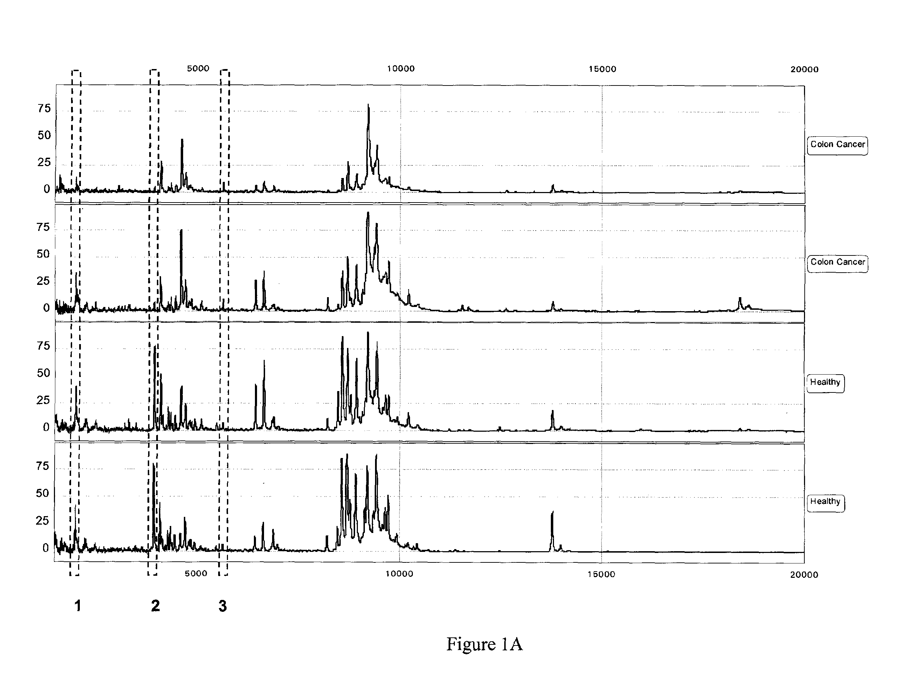

**Figure 1A.** Colon cancer. Comparison of protein mass spectra processed on the anion exchange surface of a SAX2 ProteinChip array comprised of cationic quaternary ammonium groups. Protein mass spectra obtained from sera of patients with **colon** cancer and healthy individuals are shown. Scattered boxes indicate differentially expressed proteins with high diagnostic significance. Three representative differentially expressed proteins (m/z=2020 Da, m/z=3947 Da, m/z=5653 Da) possessing highest importance within the overall classificator (ensemble of decision trees) for differential an acute and chronic inflammation of the epithelium diagnosis, based on their frequency of appearance within the ensemble of trees are highlighted. The X-axis shows the mass/charge (m/z) ratio, which is equivalent to the apparent molecular mass of the corresponding biomolecule. The Y-axis shows the normalized relative signal intensity of the peak in the examined serum samples.

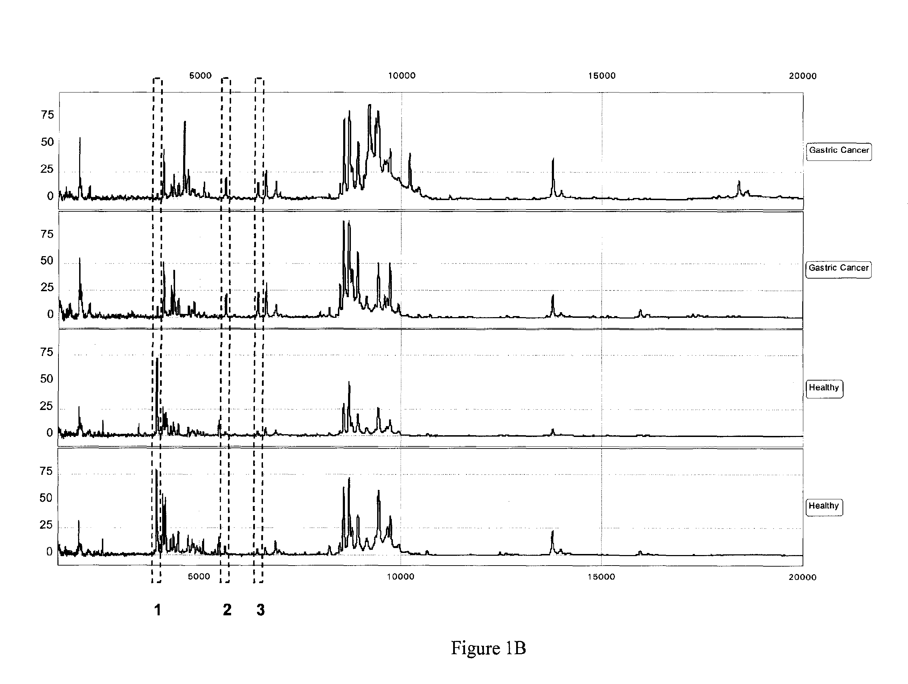

**Figure 1B.** Gastric cancer. Comparison of protein mass spectra processed on the anion exchange surface of a SAX2 ProteinChip array comprised of cationic quaternary ammonium groups. Protein mass spectra obtained from sera of patients with **gastric** cancer and healthy individuals are shown. Scattered boxes indicate differentially expressed proteins with high diagnostic significance. Three representative differentially expressed proteins (m/z=3946 Da, m/z=5650 Da, m/z=6449 Da) possessing highest importance within the overall classificator (ensemble of decision trees) for differential an acute and chronic inflammation of the epithelium diagnosis, based on their frequency of appearance within the ensemble of trees are highlighted. The X-axis shows the mass/charge (m/z) ratio, which is equivalent to the apparent molecular mass of the corresponding biomolecule. The Y-axis shows the normalized relative signal intensity of the peak in the examined serum samples.

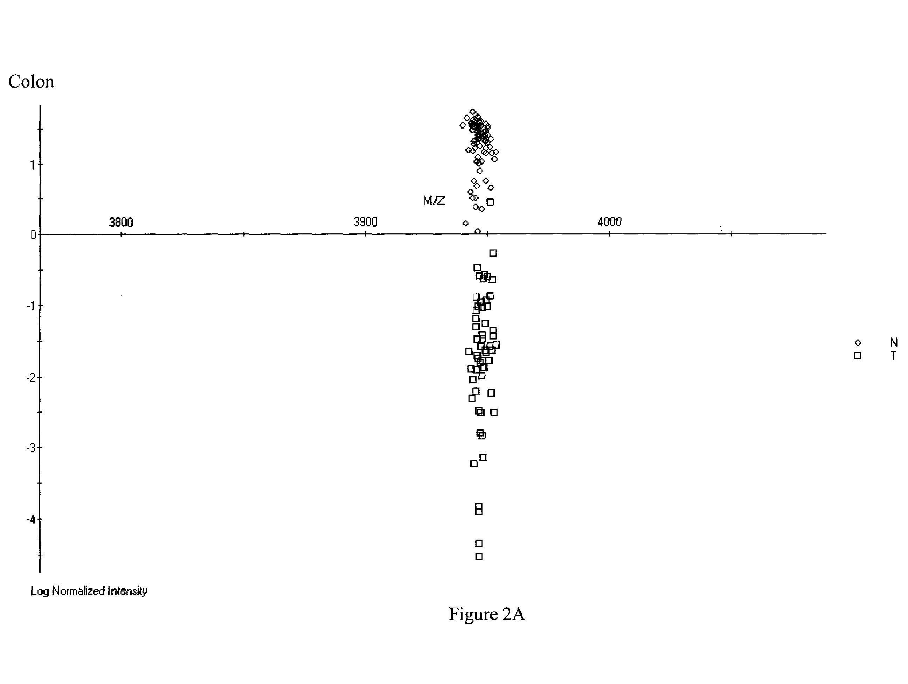

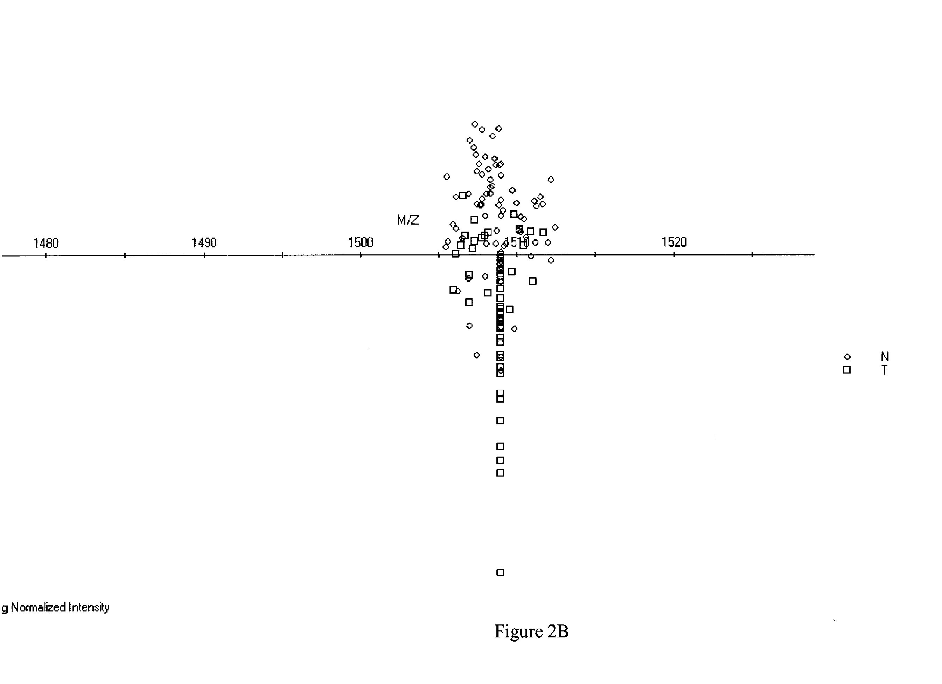

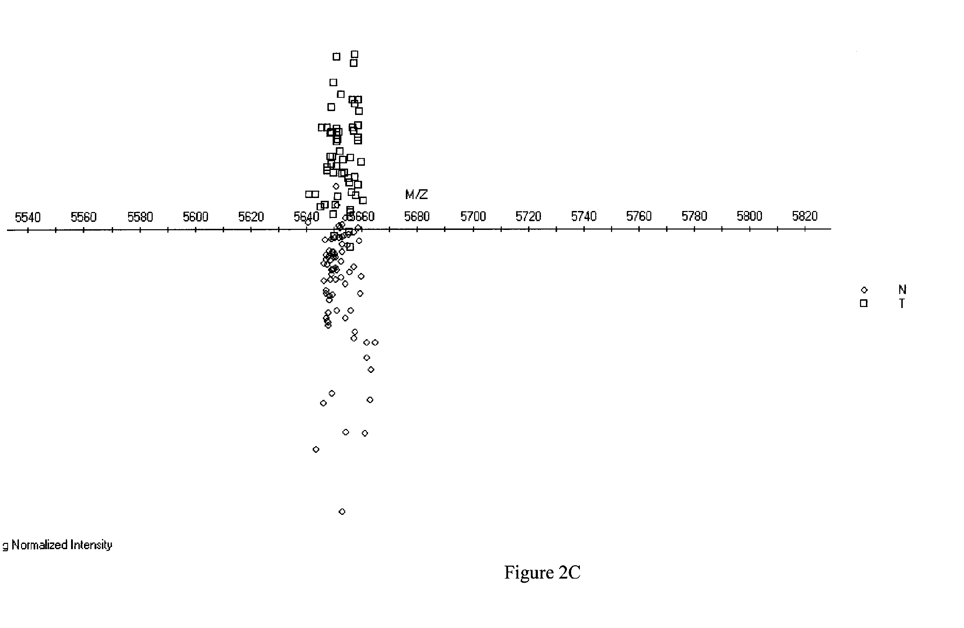

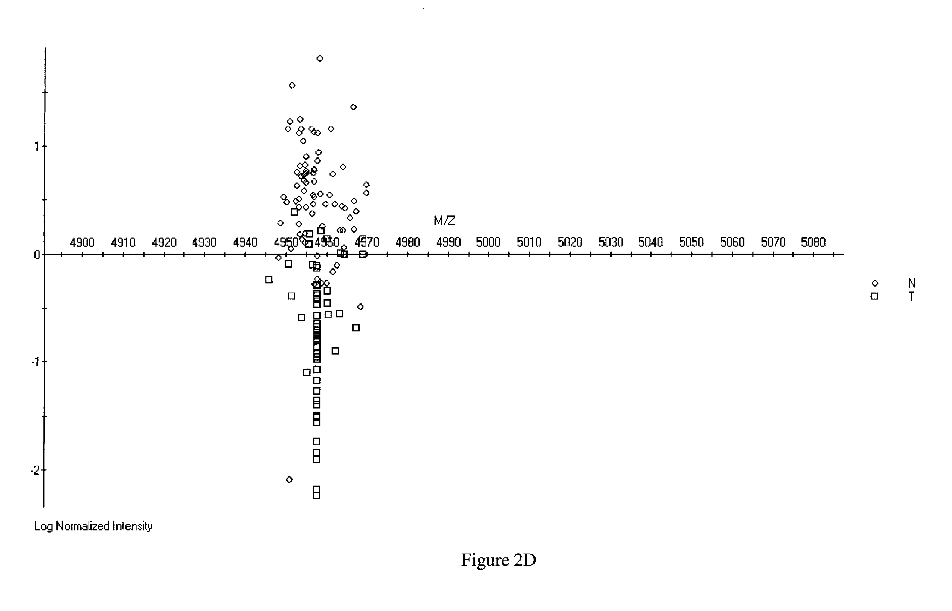

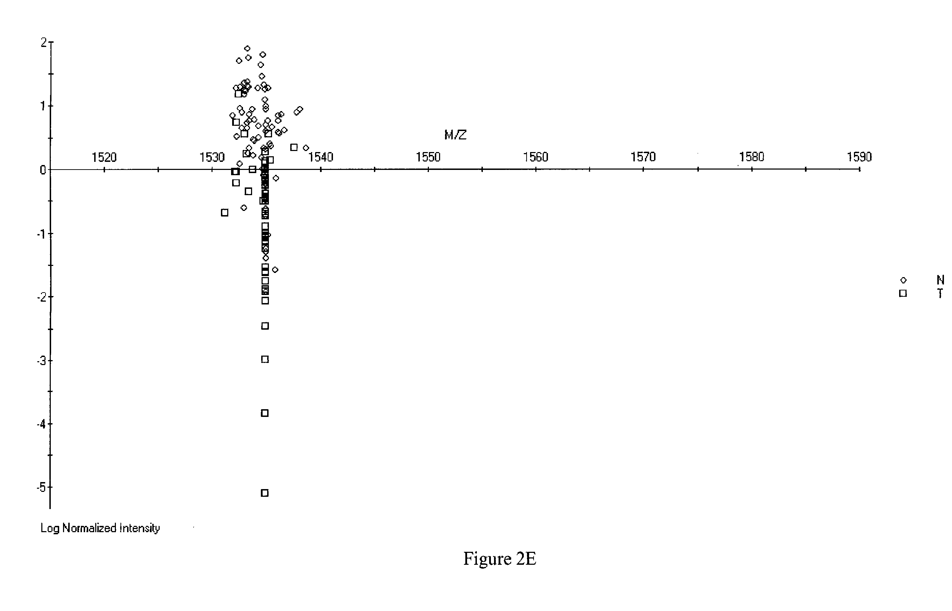

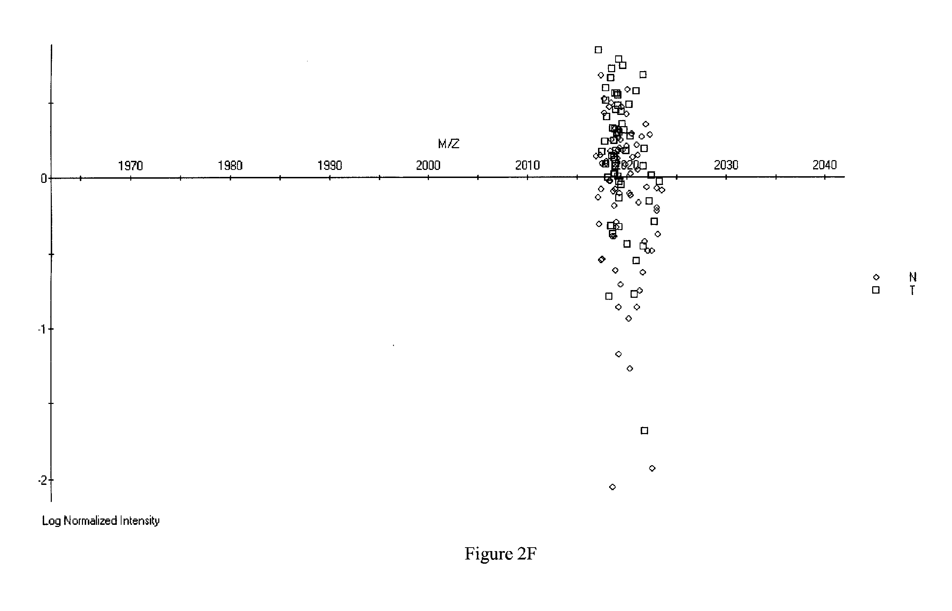

**Figure 2A - F.** Colon cancer. Scatter plots of clusters (peaks, variables) m/z = 3947 Da, 1509 Da, 5653 Da, 4958 Da, 1535 Da, 2020 Da. The X-axis shows the mass/charge (m/z) ratio, which is equivalent to the apparent molecular mass of the corresponding biomolecule. The Y-axis shows the log₂ of the normalized relative signal intensity of the peak in the examined serum samples, whereas the value of the highest peak intensity within the cluster is set to 100% and. □ T (An acute and chronic inflammation of the epithelium): Colon cancer patients' serum samples. ○ N (Normal): Healthy patients' serum samples.
**Figure 2A**. Colon cancer. Scatter plot of cluster (peak, variable) m/z = 3947 Da.
**Figure 2B**. Colon cancer. Scatter plot of cluster (peak, variable) m/z = 1509 Da.
**Figure 2C**. Colon cancer. Scatter plot of cluster (peak, variable) m/z = 5653 Da.
**Figure 2D**. Colon cancer. Scatter plot of cluster (peak, variable) m/z = 4958 Da.
**Figure 2E**. Colon cancer. Scatter plot of cluster (peak, variable) m/z = 1535 Da.
**Figure 2F.** Colon cancer. Scatter plot of cluster (peak, variable) m/z = 2020 Da.

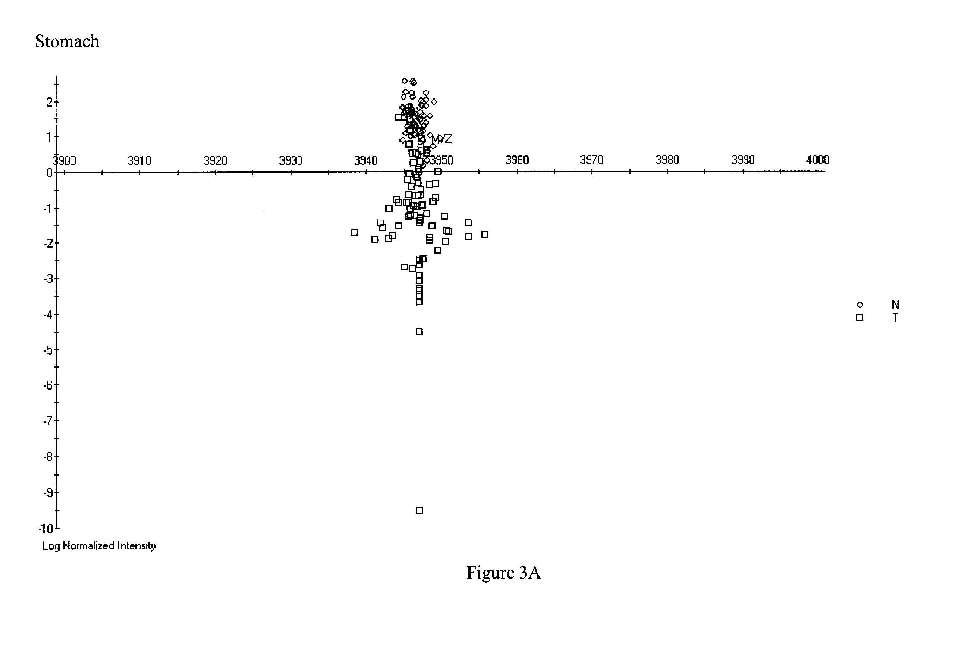

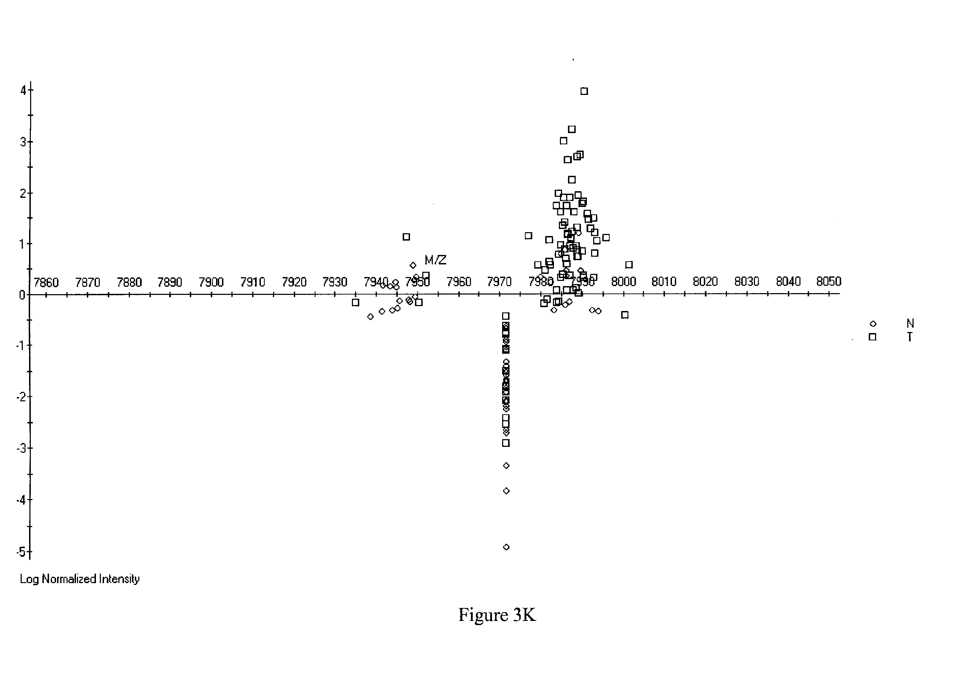

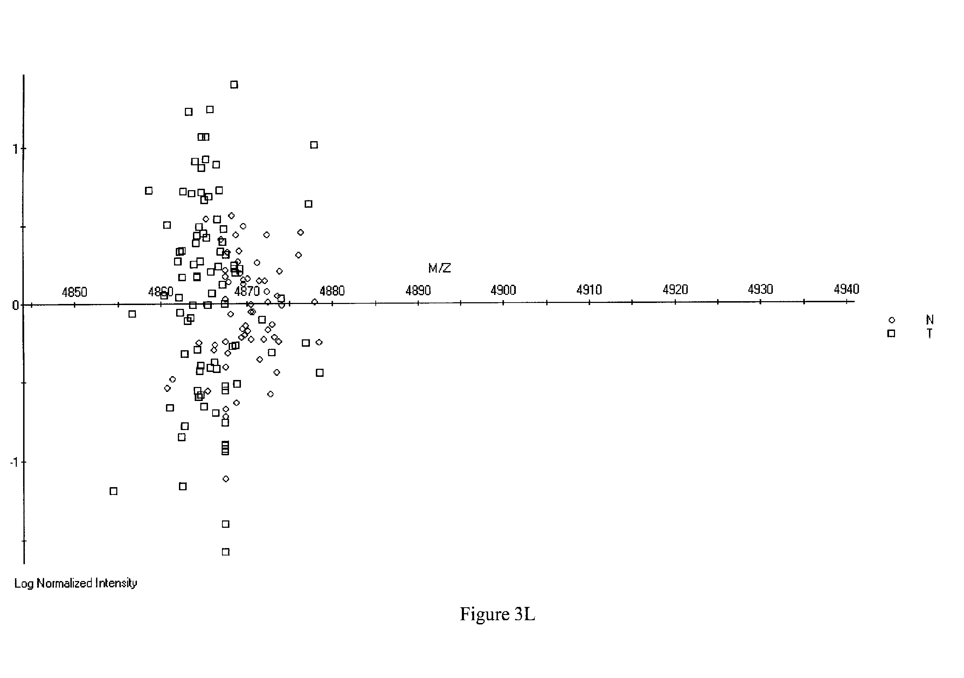

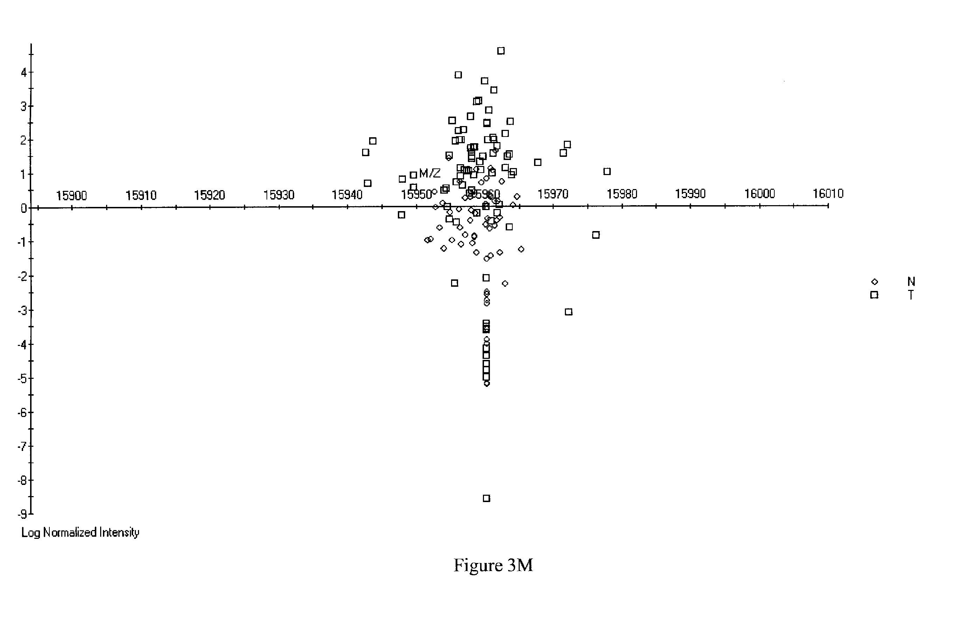

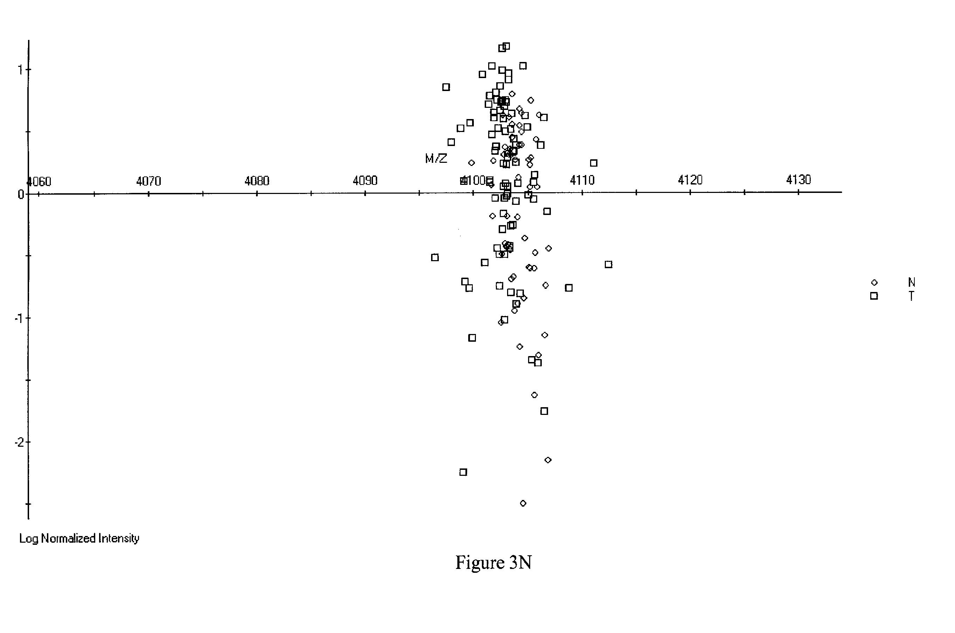

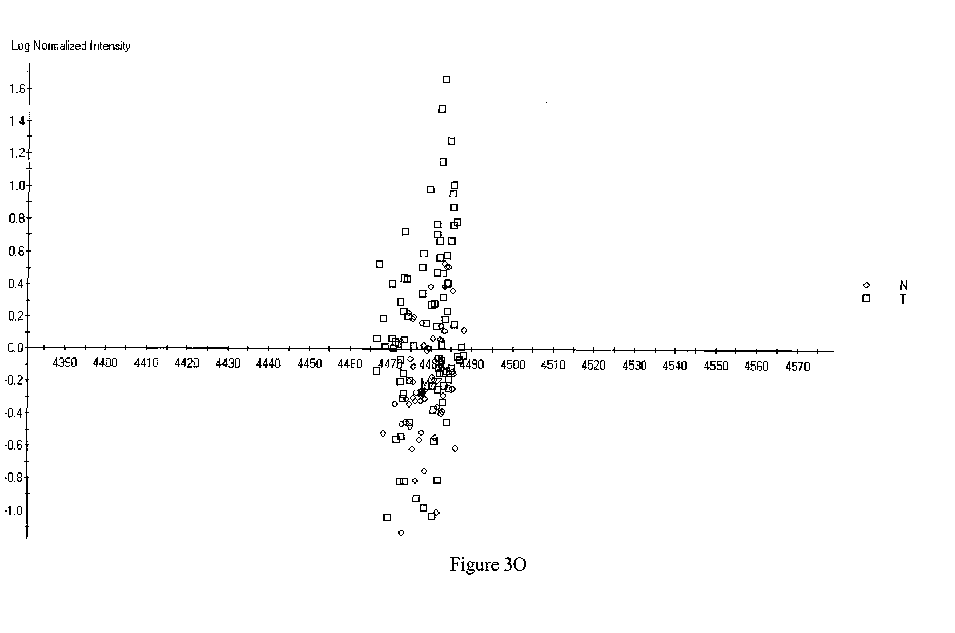

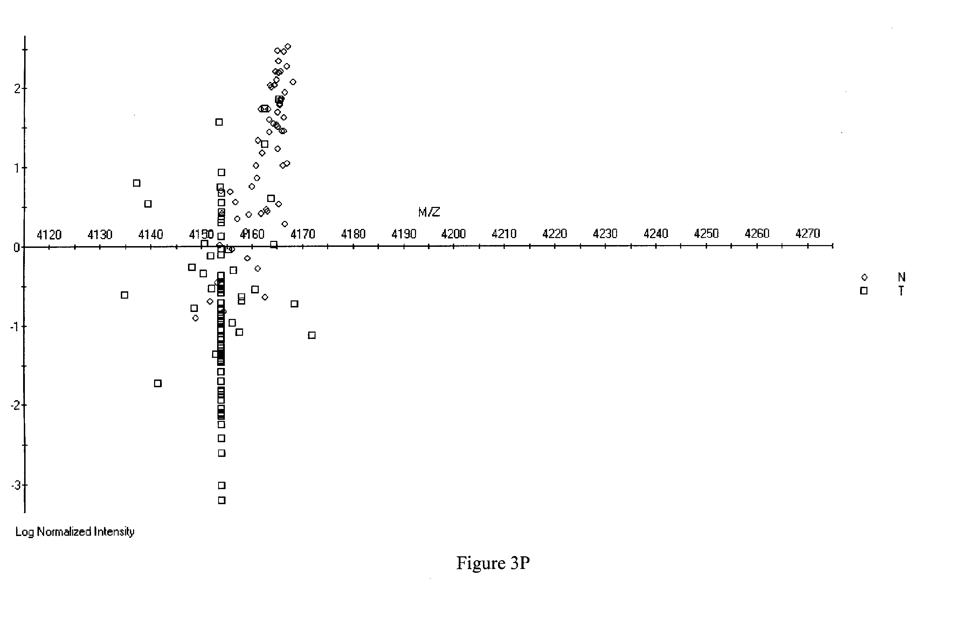

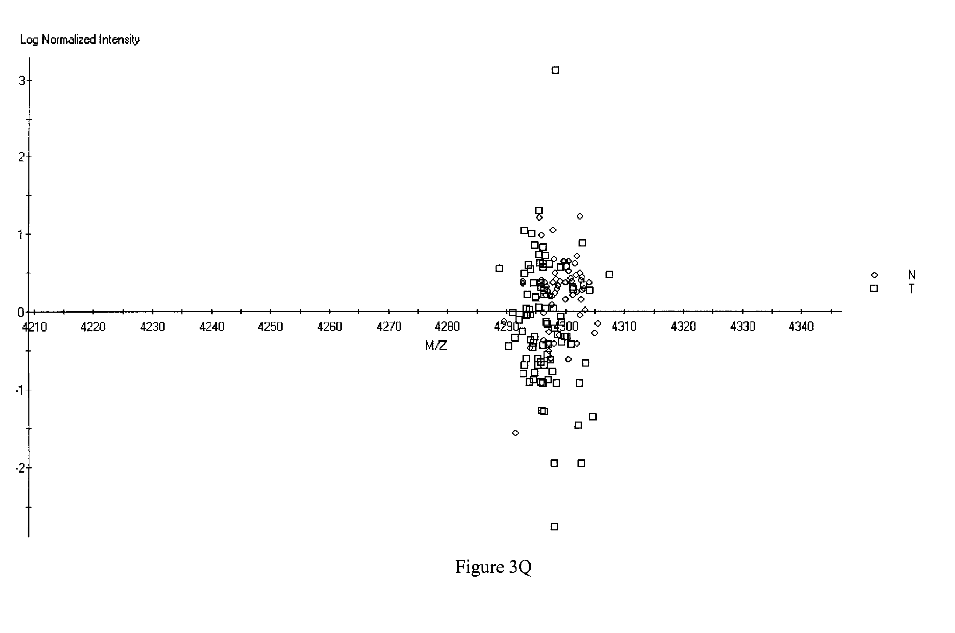

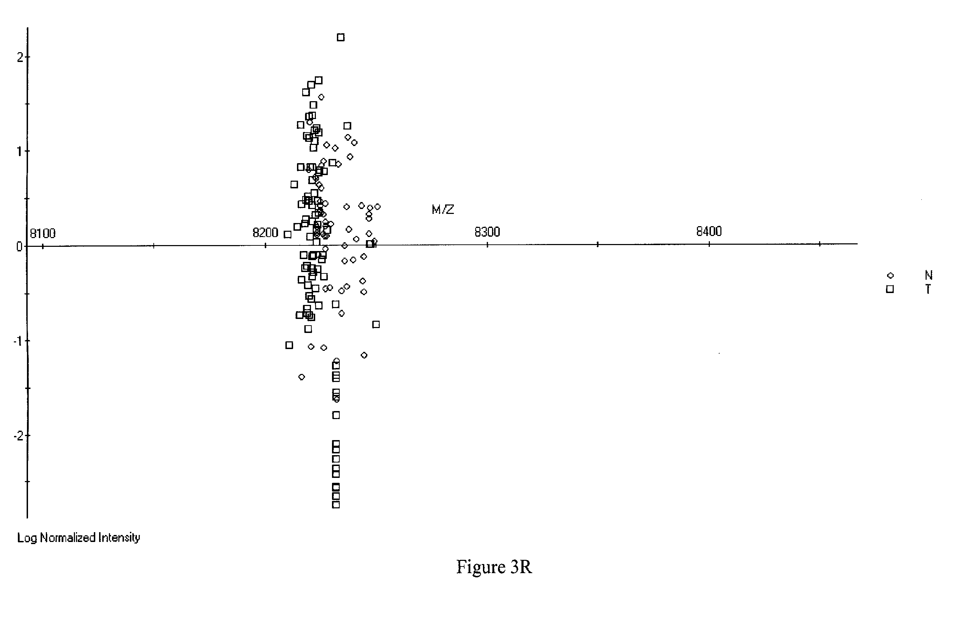

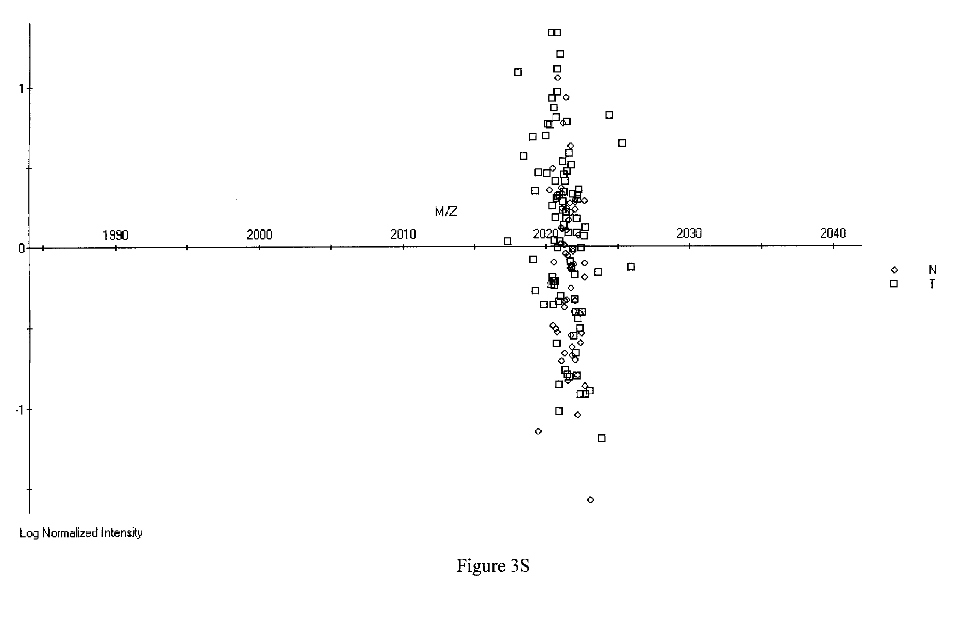

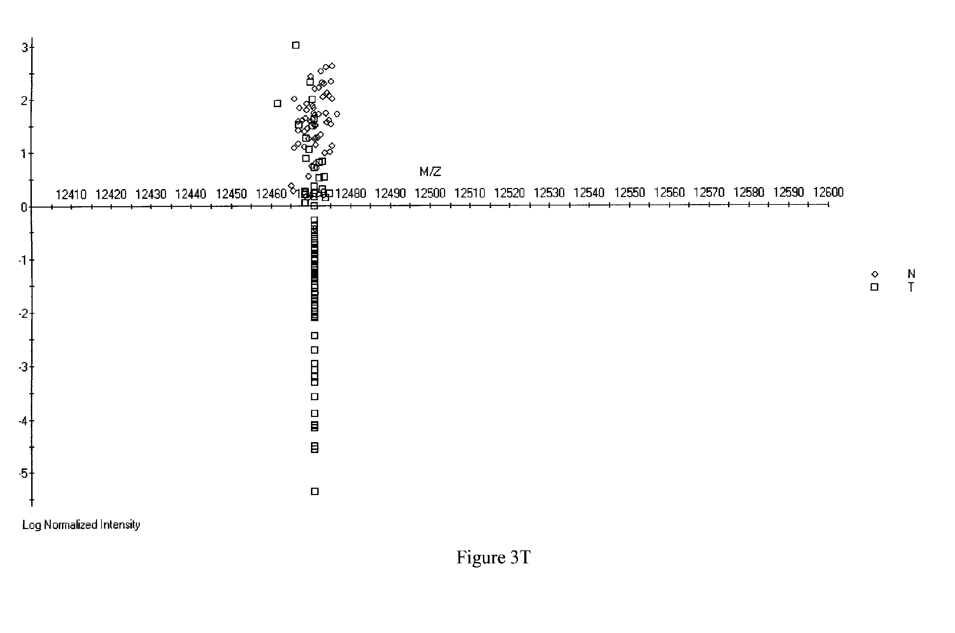

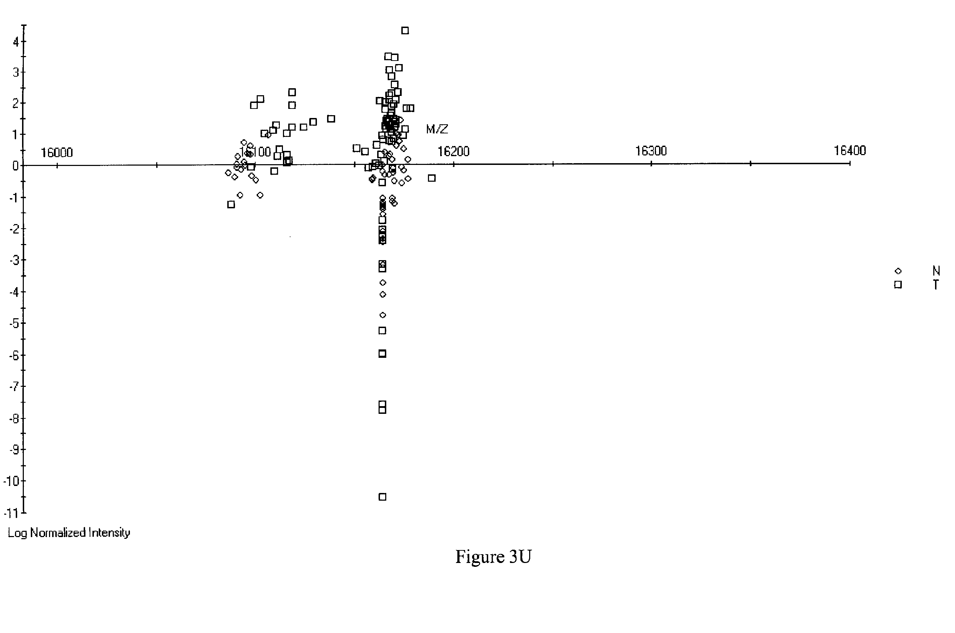

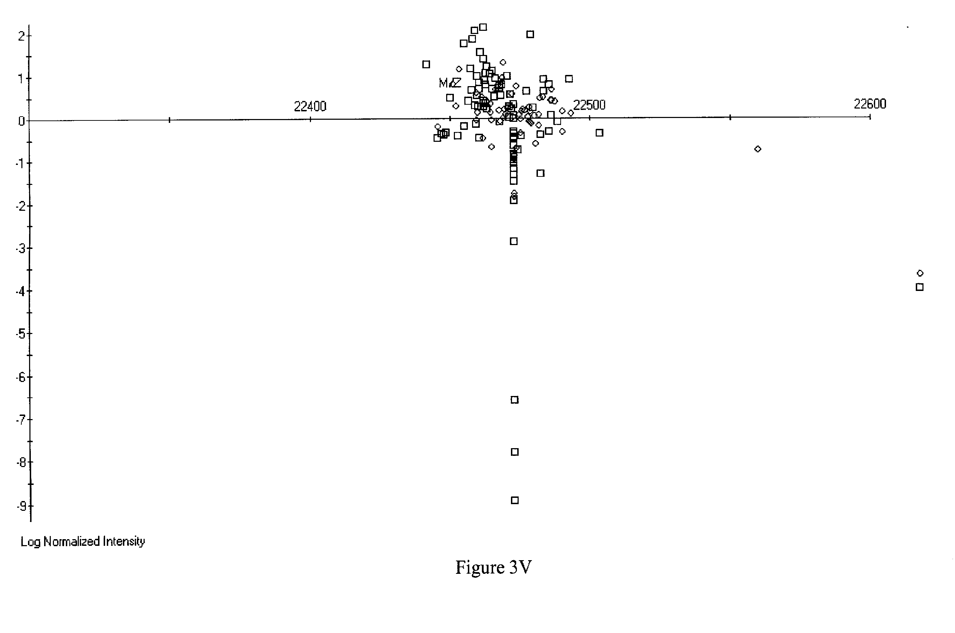

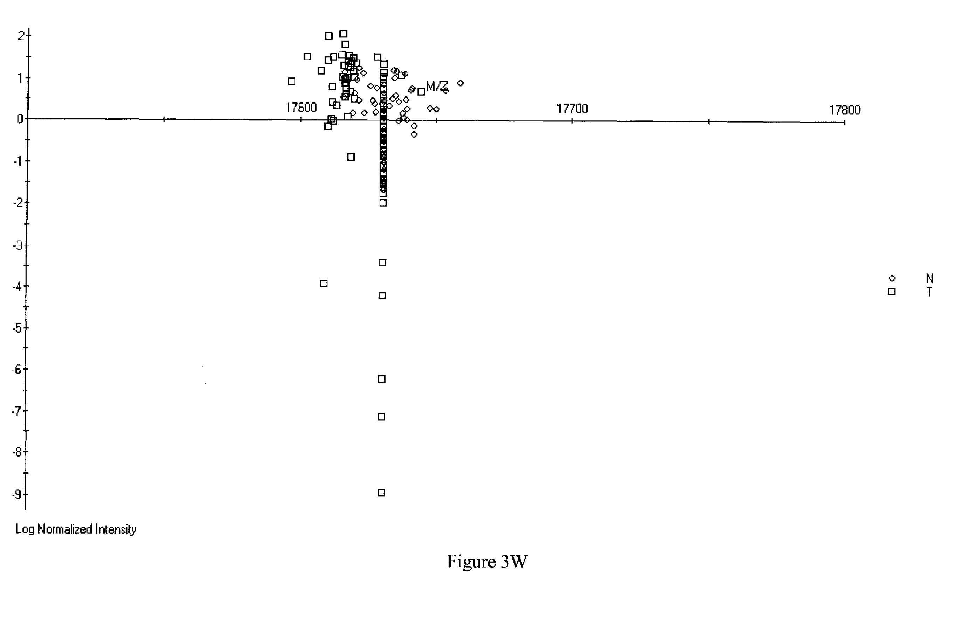

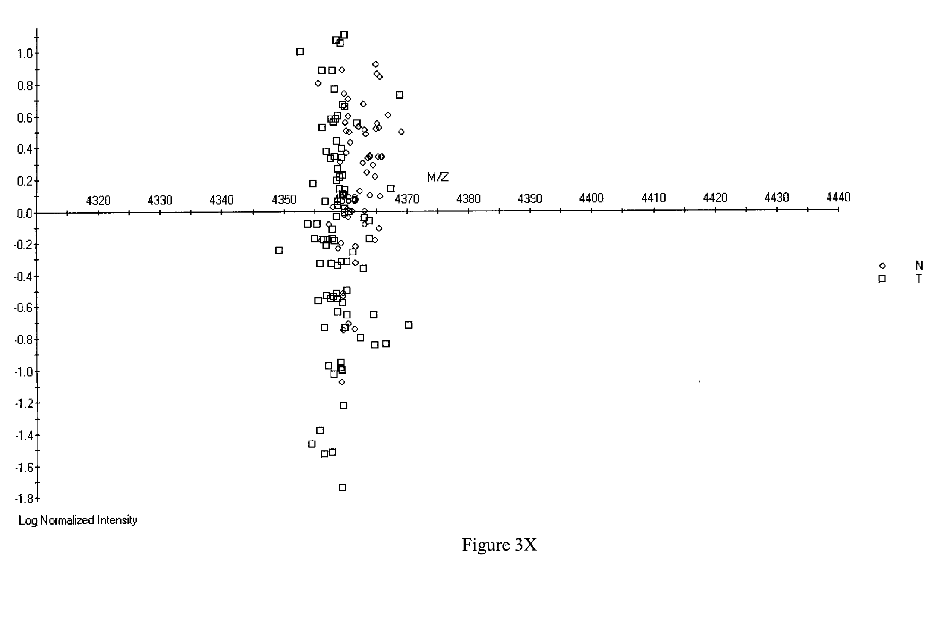

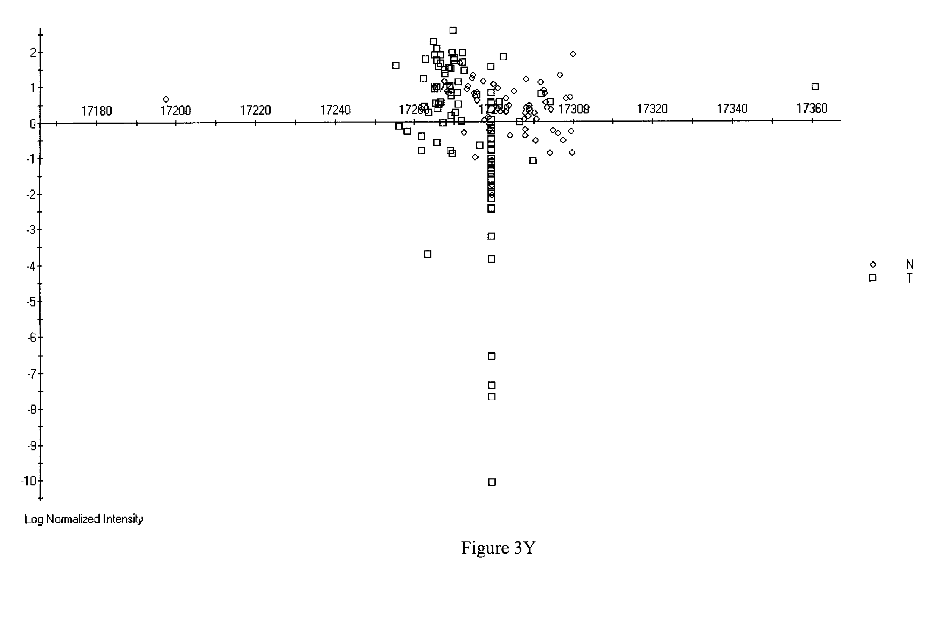

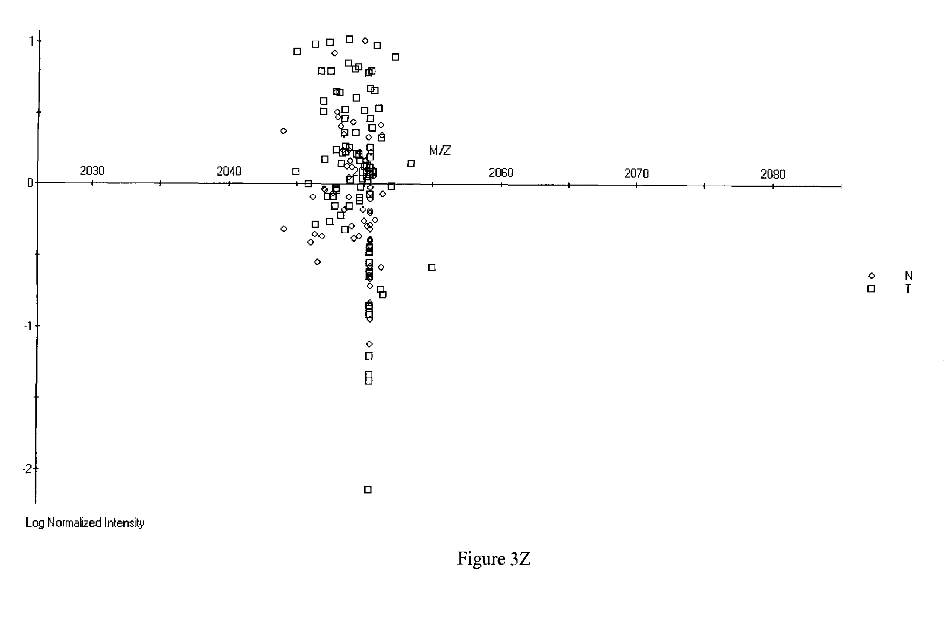

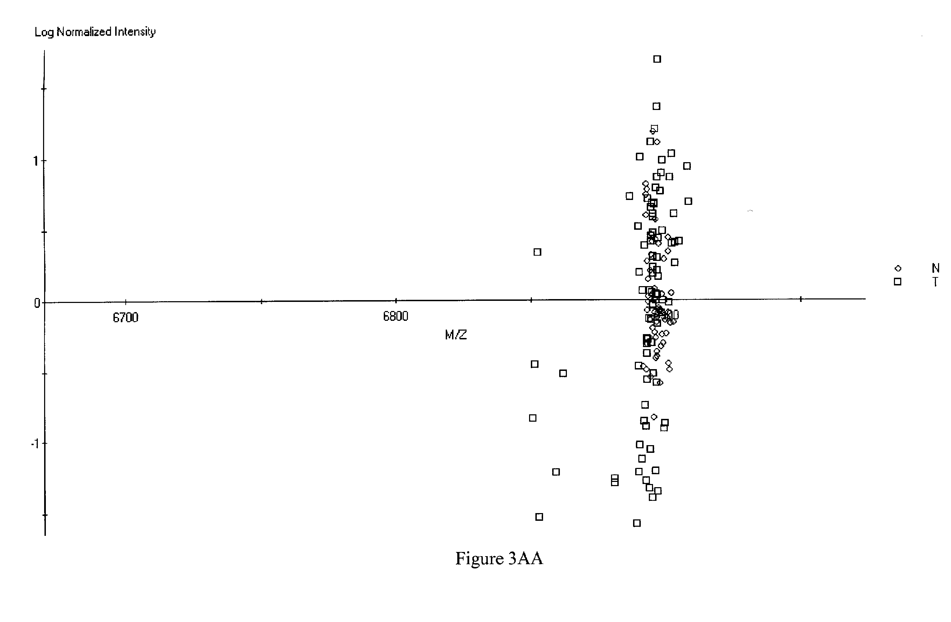

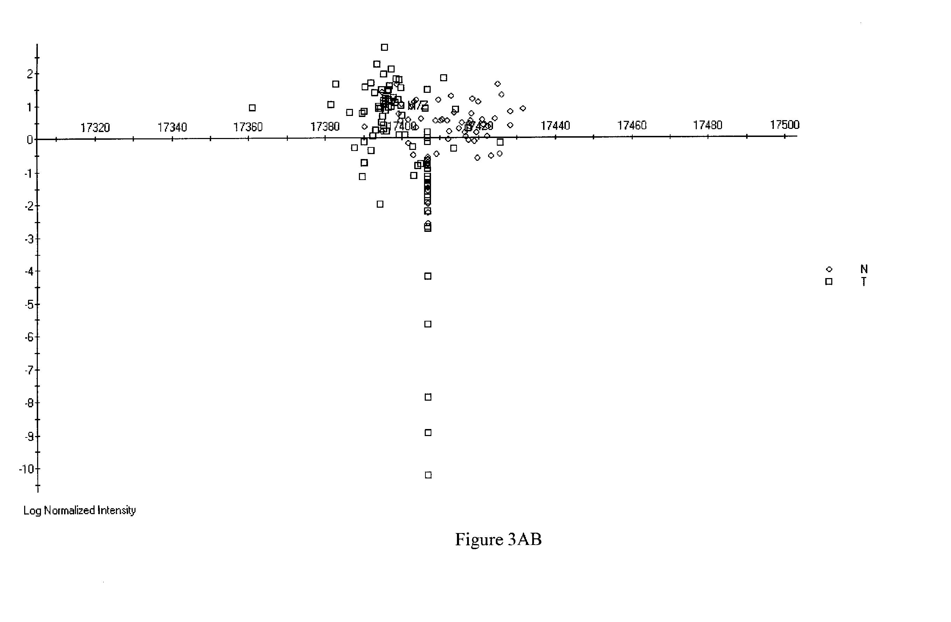

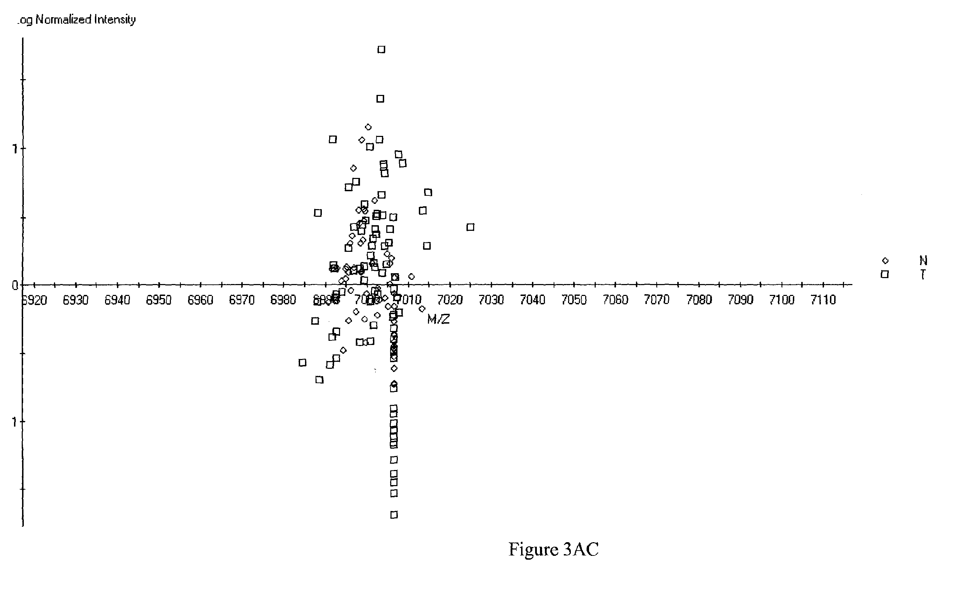

**Figure 3A - AC.** Gastric cancer. Scatter plots of clusters (peaks, variables) m/z = 3947 Da, 5492 Da, 5650 Da, 8711 Da, 1516 Da, 10665 Da, 18133 Da, 6450 Da, 13996 Da, 7971 Da, 4867 Da, 15960 Da, 4104 Da, 4477 Da, 4154 Da, 4298 Da, 8232 Da, 2022 Da, 12471 Da, 16164 Da, 22473 Da, 17630 Da, 4360 Da, 17279 Da, 2050 Da, 6881 Da, 17406 Da, 7006 Da. The X-axis shows the mass/charge (m/z) ratio, which is equivalent to the apparent molecular mass of the corresponding biomolecule. The Y-axis shows the log₂ of the normalized relative signal intensity of the peak in the examined serum samples, whereas the value of the highest peak intensity within the cluster is set to 100%. □ T (An acute and chronic inflammation of the epithelium): Colon cancer patients' serum samples. o N (Normal): Healthy patients' serum samples.
**Figure 3A.** Gastric cancer. Scatter plot of cluster (peak, variable) m/z = 3947 Da.

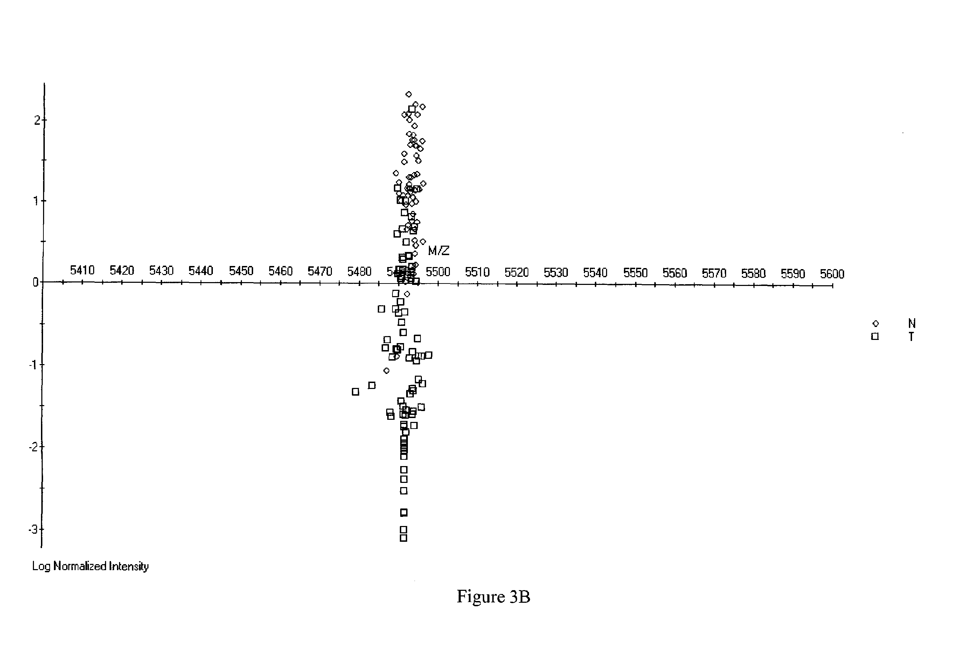

**Figure 3B.** Gastric cancer. Scatter plot of cluster (peak, variable) m/z = 5492 Da.

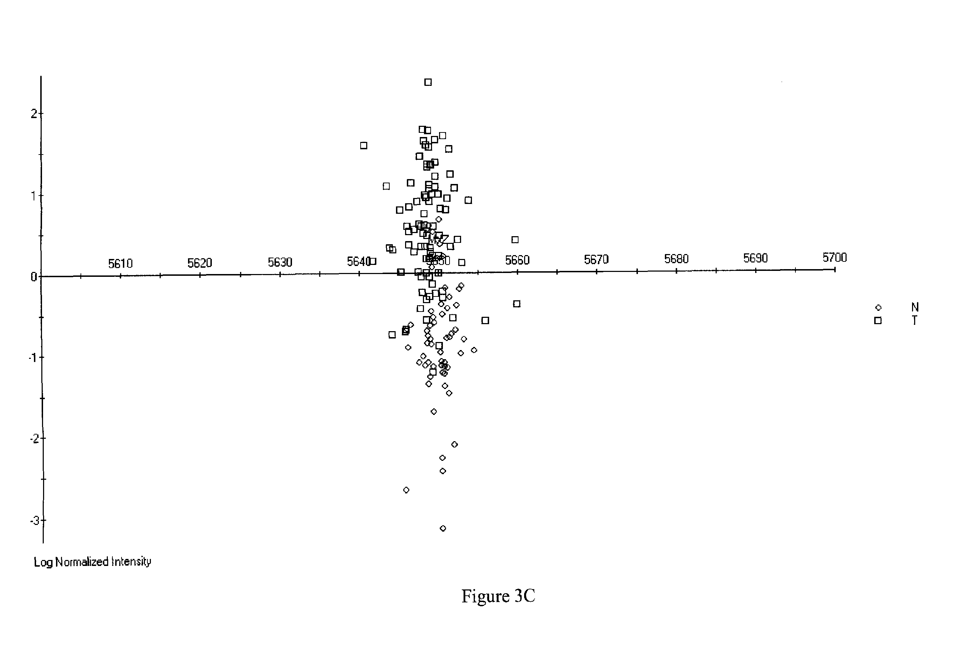

**Figure 3C.** Gastric cancer. Scatter plot of cluster (peak, variable) m/z = 5650 Da.

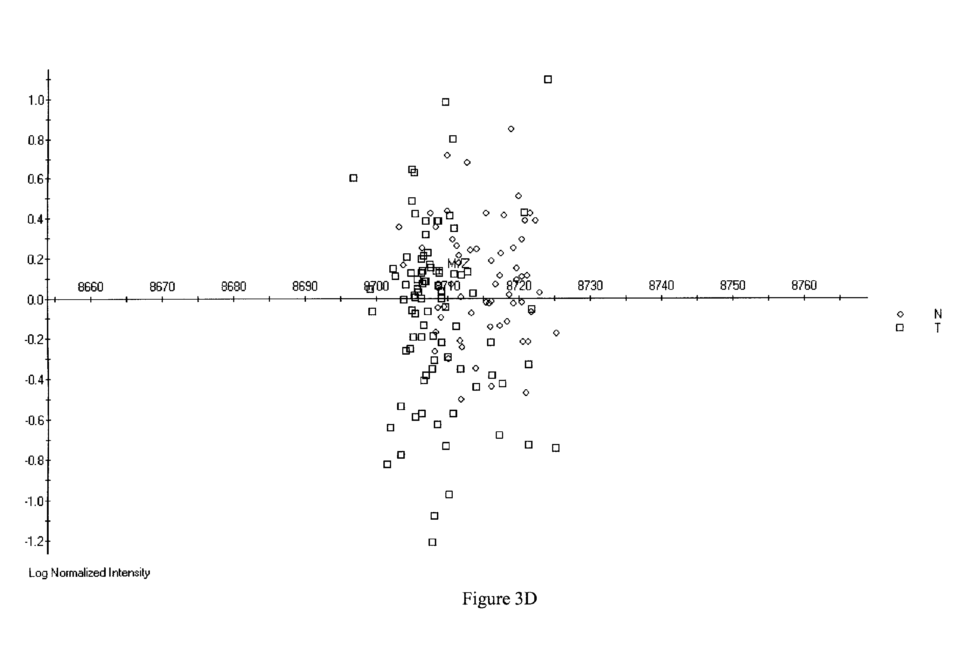

**Figure 3D.** Gastric cancer. Scatter plot of cluster (peak, variable) m/z = 8711 Da.

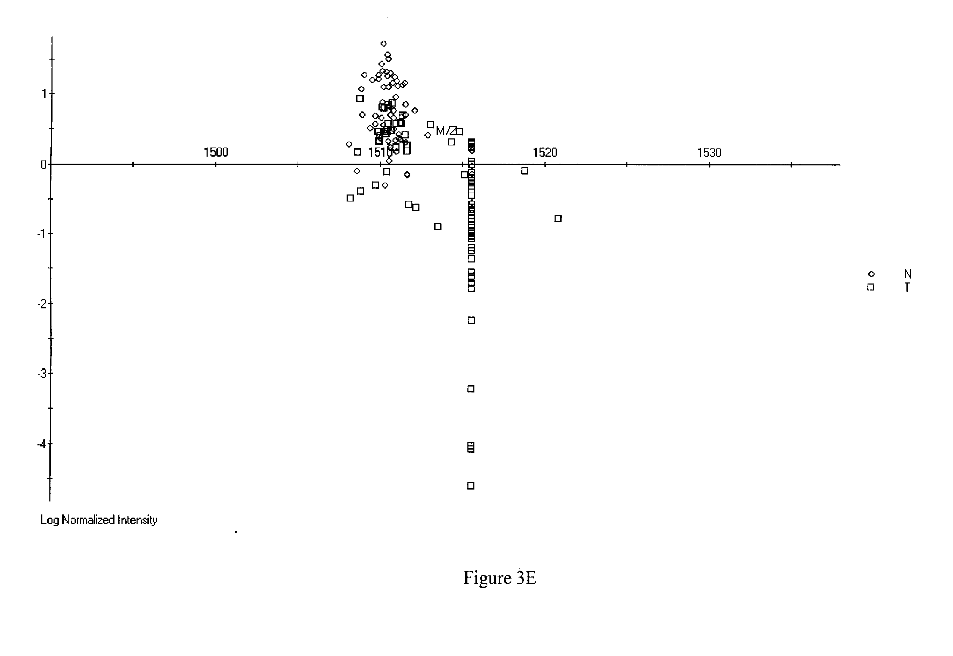

**Figure 3E.** Gastric cancer. Scatter plot of cluster (peak, variable) m/z = 1516 Da.

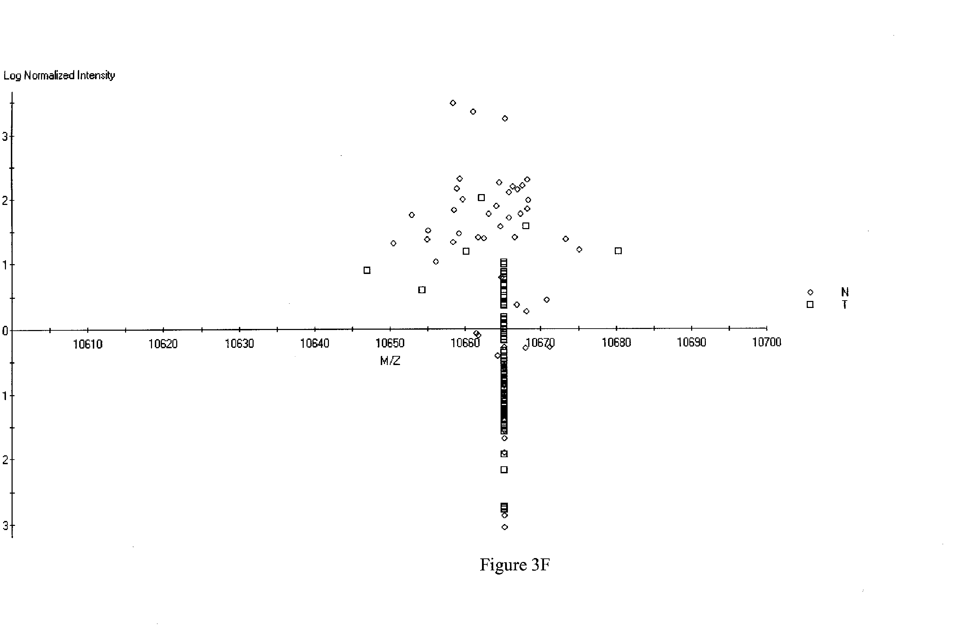

**Figure 3F.** Gastric cancer. Scatter plot of cluster (peak, variable) m/z = 10665 Da.

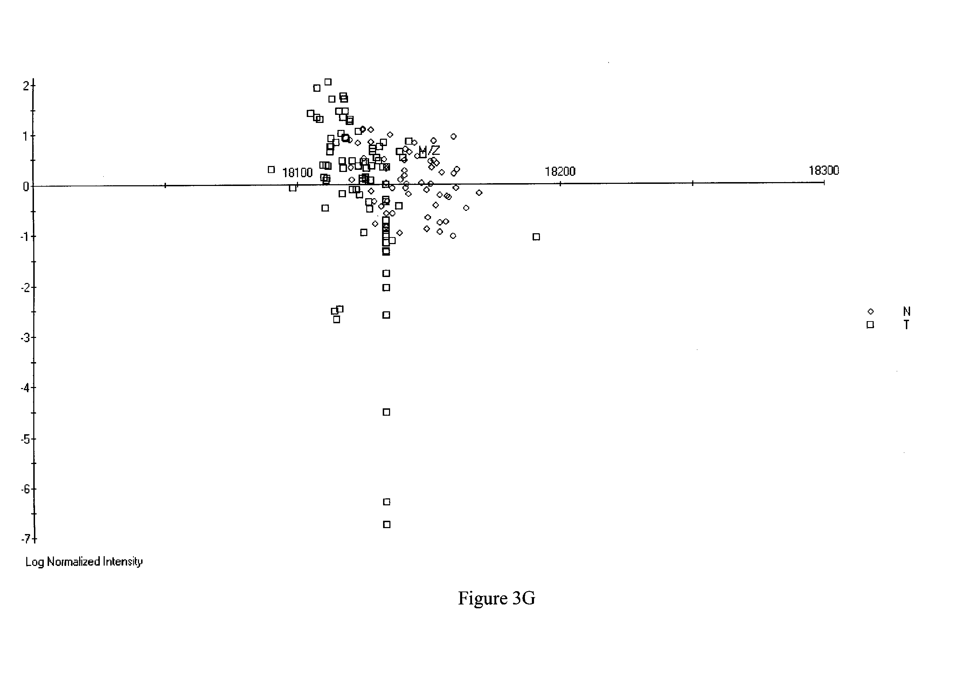

**Figure 3G.** Gastric cancer. Scatter plot of cluster (peak, variable) m/z = 18133 Da.

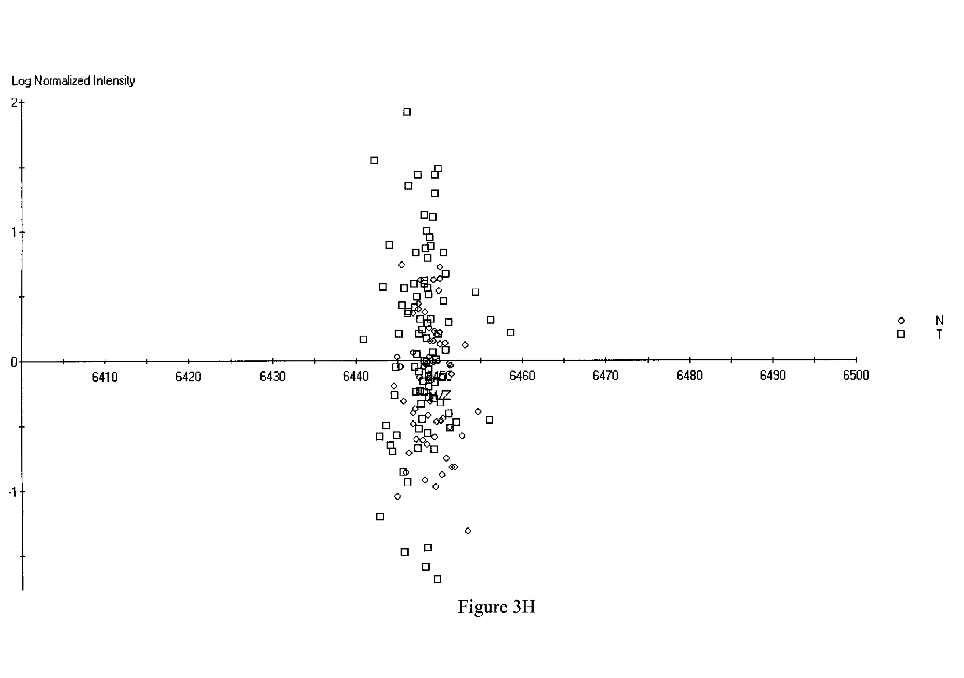

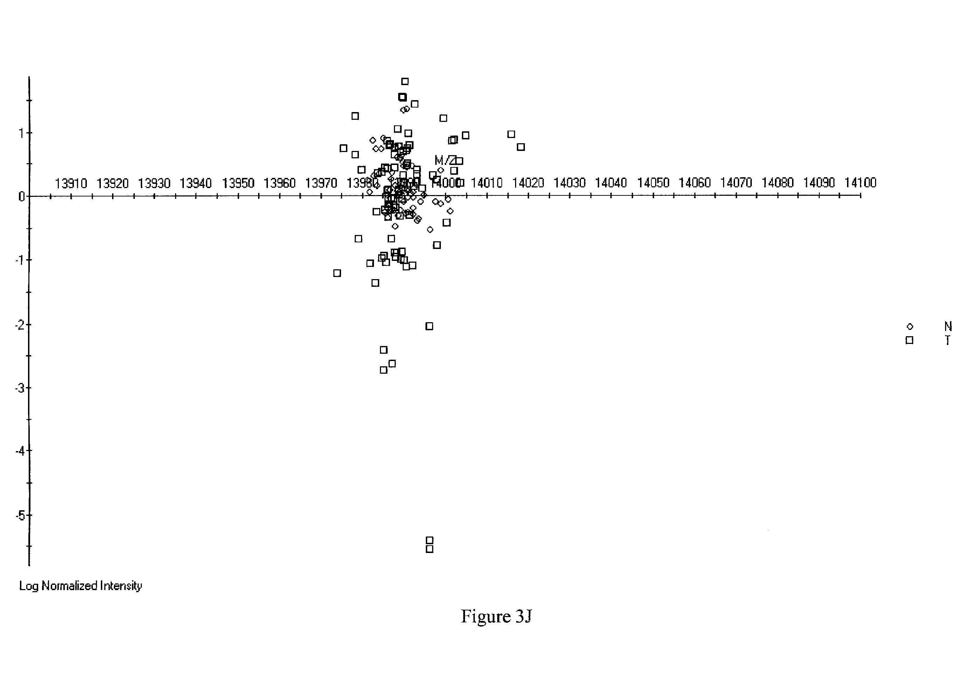

**Figure 3H.** Gastric cancer. Scatter plot of cluster (peak, variable) m/z = 6450 Da.
**Figure 3J.** Gastric cancer. Scatter plot of cluster (peak, variable) m/z = 13996 Da.
**Figure 3K.** Gastric cancer. Scatter plot of cluster (peak, variable) m/z = 7971 Da.
**Figure 3L.** Gastric cancer. Scatter plot of cluster (peak, variable) m/z = 4867 Da.
**Figure 3M.** Gastric cancer. Scatter plot of cluster (peak, variable) m/z = 15960 Da.
**Figure 3N.** Gastric cancer. Scatter plot of cluster (peak, variable) m/z = 4104 Da.
**Figure 3O.** Gastric cancer. Scatter plot of cluster (peak, variable) m/z = 4477 Da.
**Figure 3P.** Gastric cancer. Scatter plot of cluster (peak, variable) m/z = 4154 Da.
**Figure 3Q.** Gastric cancer. Scatter plot of cluster (peak, variable) m/z = 4298 Da.
**Figure 3R.** Gastric cancer. Scatter plot of cluster (peak, variable) m/z = 8232 Da.
**Figure 3S.** Gastric cancer. Scatter plot of cluster (peak, variable) m/z = 2022 Da.
**Figure 3T.** Gastric cancer. Scatter plot of cluster (peak, variable) m/z = 12471 Da.
**Figure 3U.** Gastric cancer. Scatter plot of cluster (peak, variable) m/z = 16164 Da.
**Figure 3V.** Gastric cancer. Scatter plot of cluster (peak, variable) m/z = 22473 Da.
**Figure 3W.** Gastric cancer. Scatter plot of cluster (peak, variable) m/z = 17630 Da.
**Figure 3X.** Gastric cancer. Scatter plot of cluster (peak, variable) m/z = 4360 Da.
**Figure 3Y.** Gastric cancer. Scatter plot of cluster (peak, variable) m/z = 17279 Da.
**Figure 3Z.** Gastric cancer. Scatter plot of cluster (peak, variable) m/z = 2050 Da.
**Figure 3AA.** Gastric cancer. Scatter plot of cluster (peak, variable) m/z = 6881 Da.
**Figure 3AB.** Gastric cancer. Scatter plot of cluster (peak, variable) m/z = 17406 Da.
**Figure 3AC.** Gastric cancer. Scatter plot of cluster (peak, variable) m/z = 7001 Da.

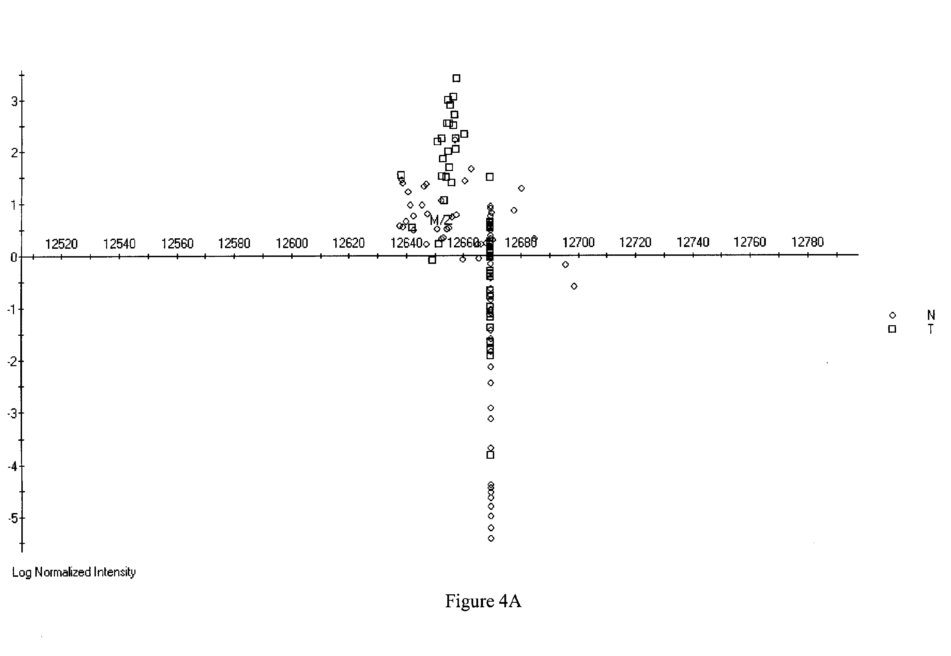

**Figure 4A** - **D.** Scatter plots of clusters (peaks, variables) m/z = 4968 Da, 5474 Da, 12669 Da, 12504 Da, belonging to differentially expressed proteins not included in the colon and stomach classifier. The X-axis shows the mass/charge (m/z) ratio, which is equivalent to the apparent molecular mass of the corresponding biomolecule. The Y-axis shows the log₂ of the normalized relative signal intensity of the peak in the examined serum samples, whereas the value of the highest peak intensity within the cluster is set to 100%. □ T (An acute and chronic inflammation of the epithelium): Cancer patients' serum samples. o N (Normal): Healthy patients' serum samples.
**Figure 4A.** Colon cancer. Scatter plot of cluster (peak, variable) m/z = 12669 Da.

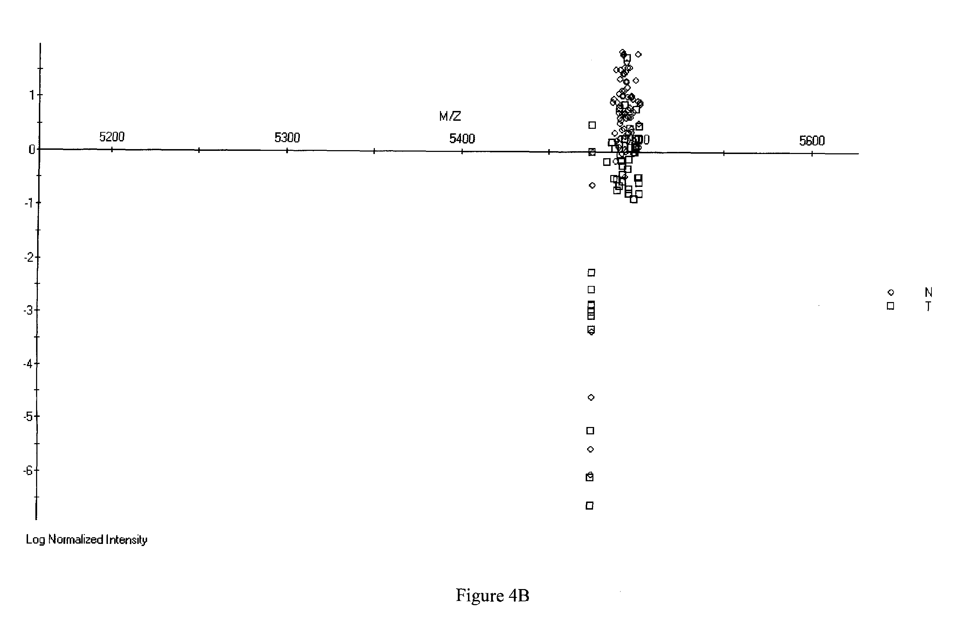

**Figure 4B.** Colon cancer. Scatter plot of cluster (peak, variable) m/z = 5474 Da.

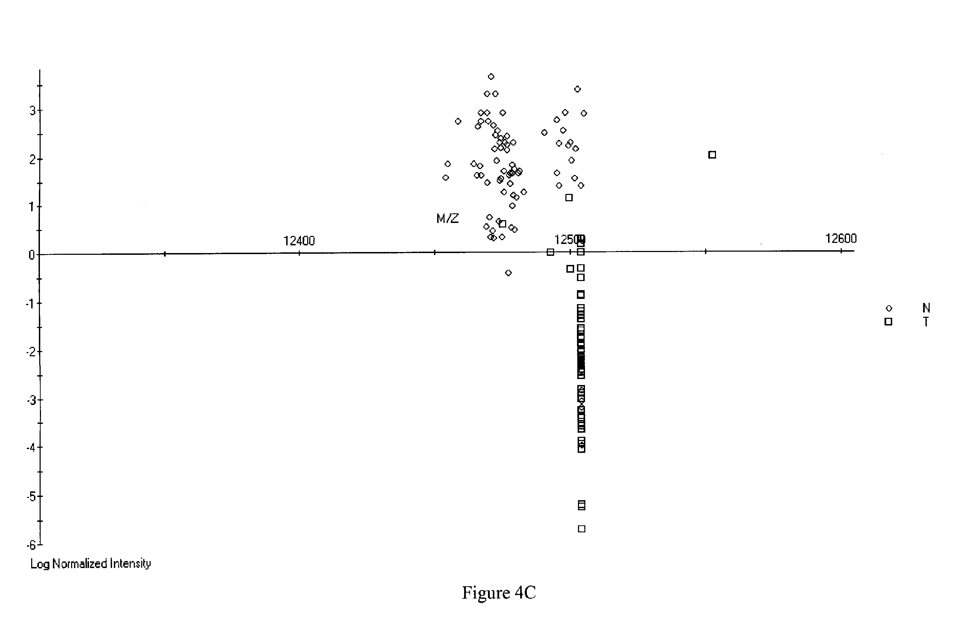

**Figure 4C.** Colon cancer. Scatter plot of cluster (peak, variable) m/z = 12504 Da.
**Figure 4D.** Colon cancer. Scatter plot of cluster (peak, variable) m/z = 4968 Da.

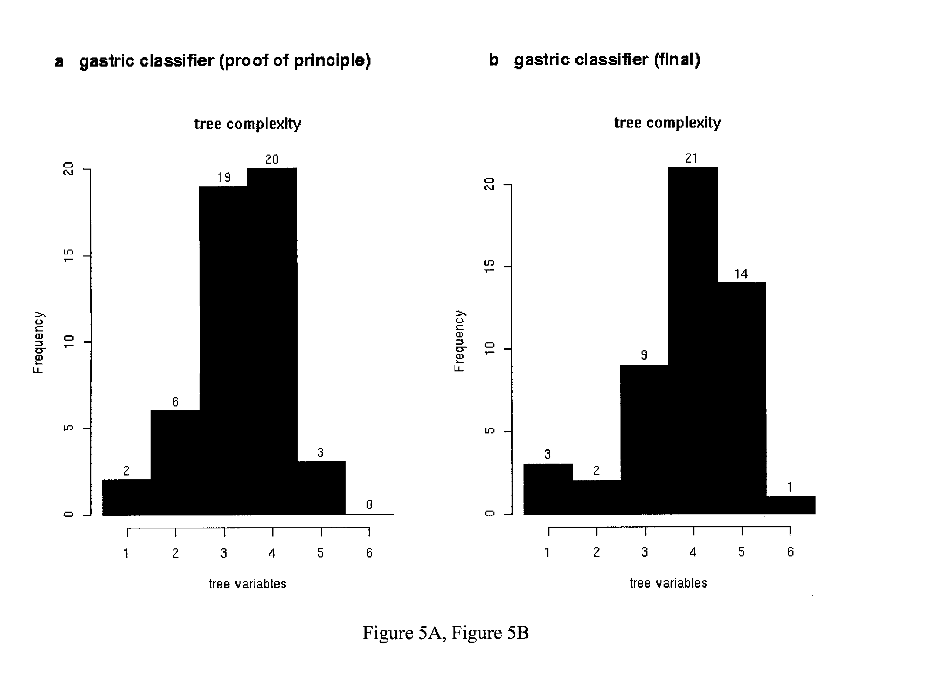

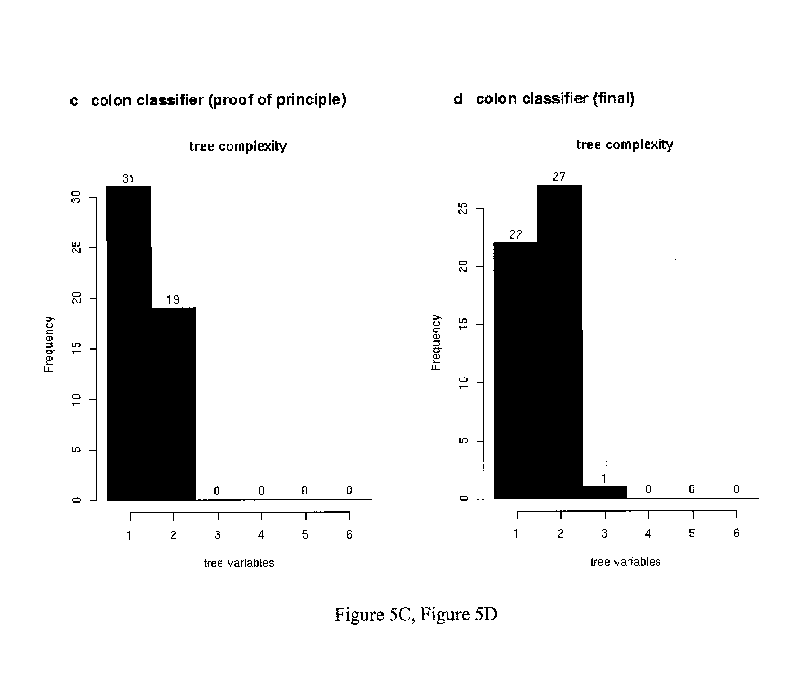

**Figure 5.** Distribution of classifier complexity. The four histograms visualize the distribution of the number of decision tree variables (peaks, clusters) for the gastric cancer classifier (A, B) and the colon cancer classifier (C, D).

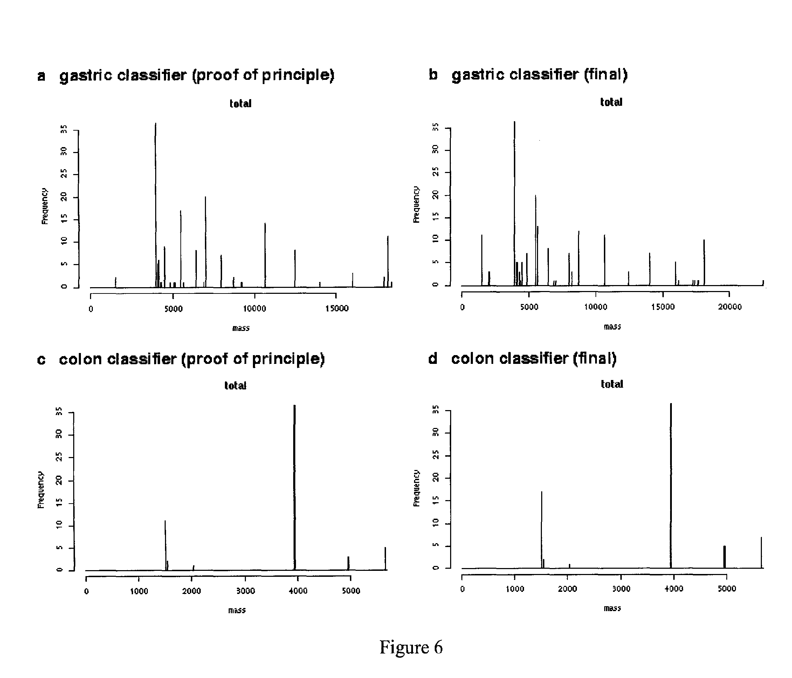

**Figure 6.** Variable importance. The four histograms visualize how often a variable (mass) is employed in the gastric cancer classifiers (A, B) and the colon cancer classifiers (C, D).

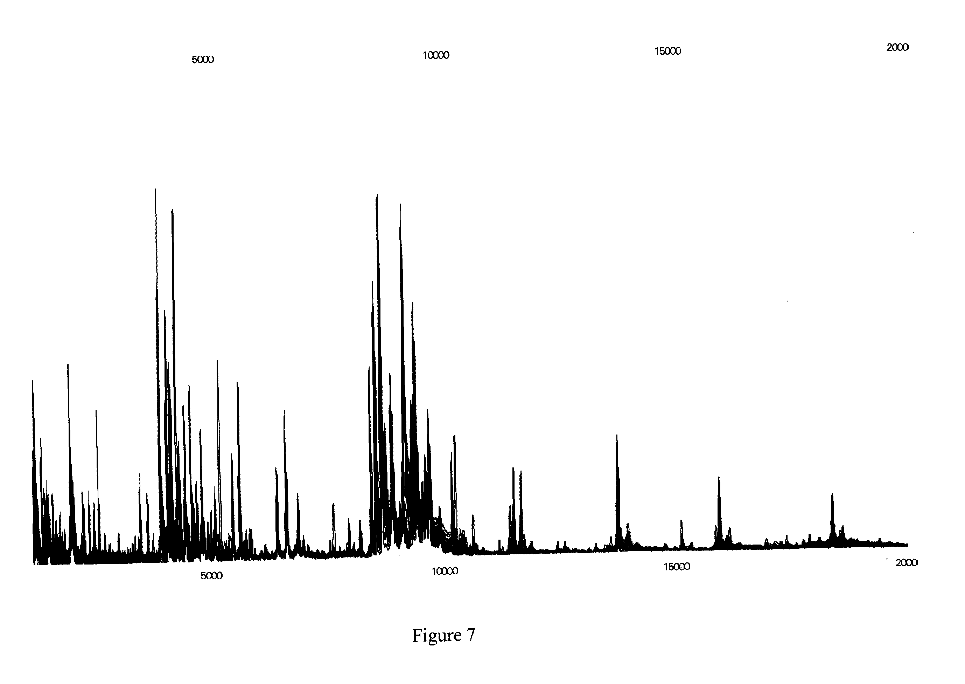

**Figure 7.** Overlay of protein mass spectra processed on SAX2 ProteinChip surface. Protein mass spectra obtained in sera of patients with gastric cancer (blue) and non-cancer individuals (red) are superimposed. Differential expression and variations in intensity indicate potential biomarkers. Three biomarkers (2020, 8483 and 13779 Da) were chosen by the Biomarker Pattern Software for the generation of a decision tree algorithm.

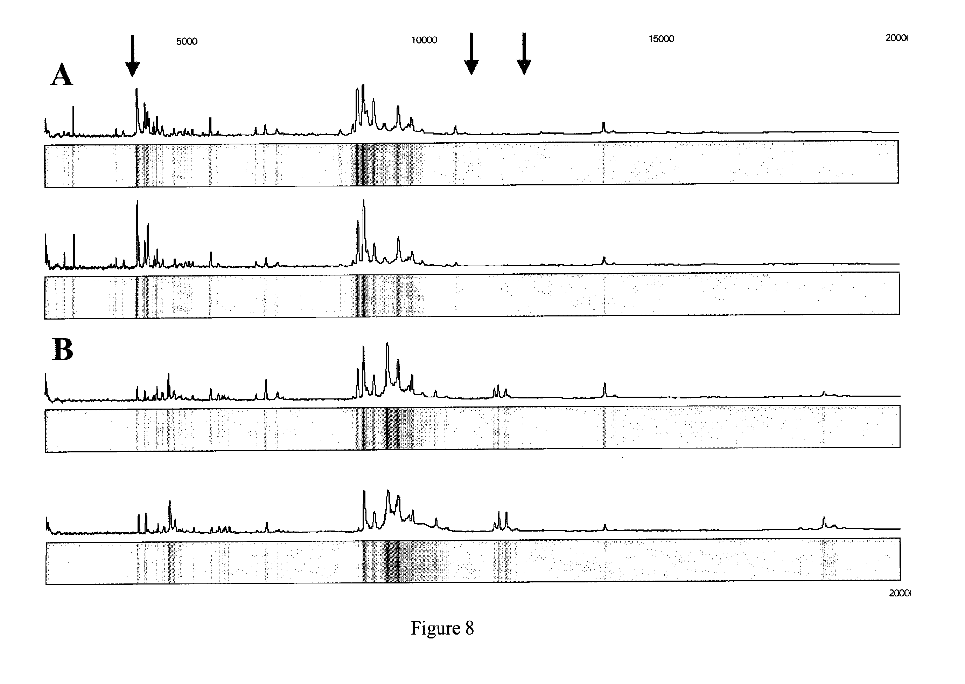

**Figure 8.** Representative protein mass spectra from two patients without gastric cancer (**A**) and two patients with gastric cancer (**B**), depicted as mass spectra and respective gel views. Arrows indicate the position of the three biomarkers used for the decision tree algorithm

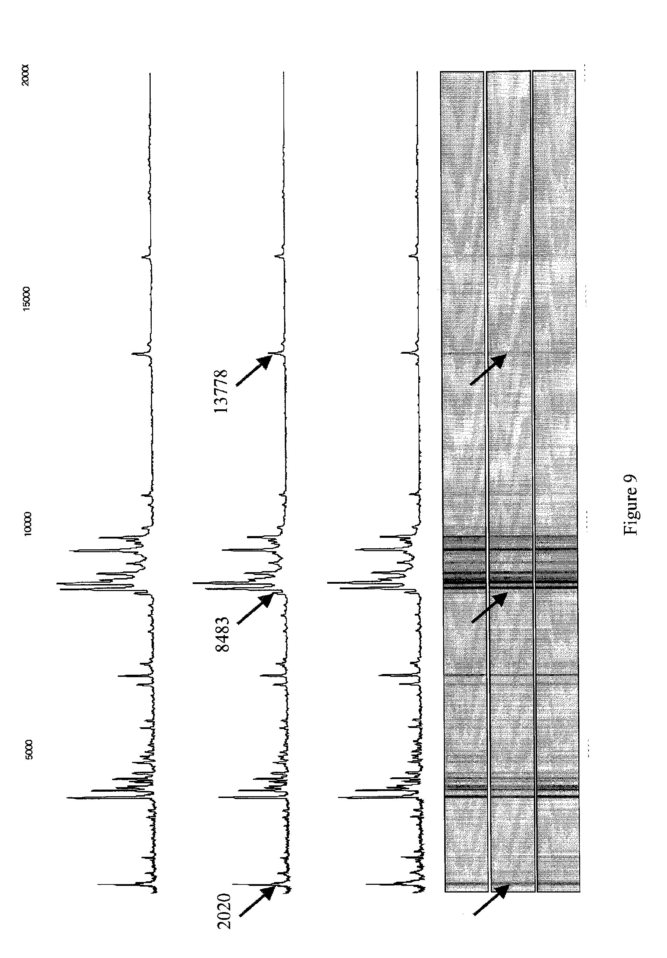

**Figure 9.** Representative protein mass spectra from a patient without gastric cancer processed on different ProteinChip arrays at different days in order to demonstrate reproducibility of the protein mass spectra. Upper panel, mass spectra; lower panel, respective gel views. Arrows indicate the position of the three proteins that were used to determine the variance in intensity and mass.

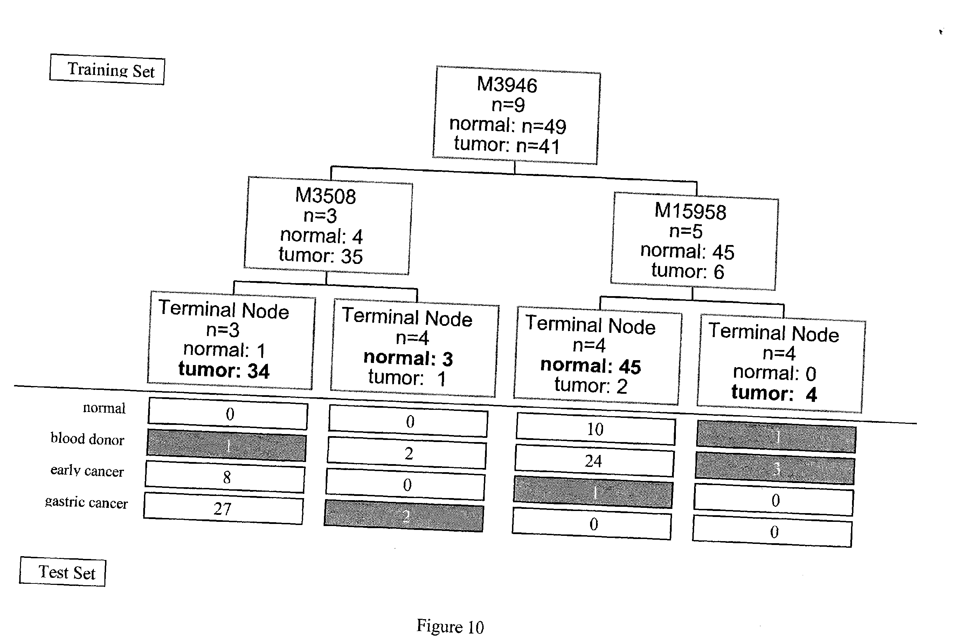

**Figure 10.** Diagram of the decision tree algorithm. The numbers in each box indicate the total number of samples, together with the number of cancer and non-cancer individuals. Upper panel, train set. Lower panel: 5 different test sets. Grey boxes indicate misclassified cases.

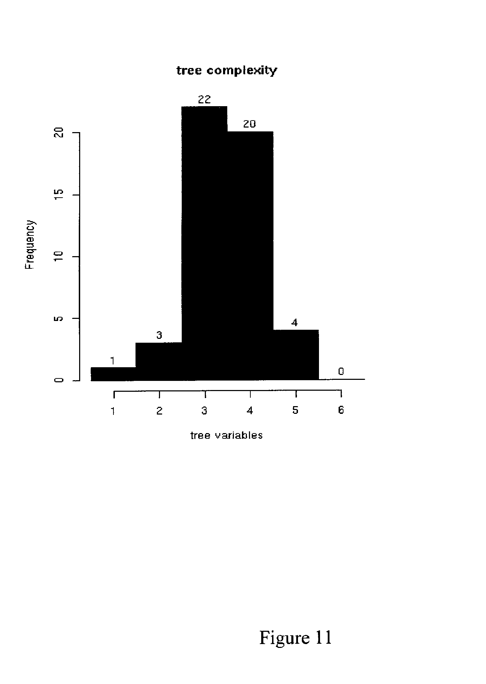

**Figure 11.** Distribution of decision tree complexity. For each of the 50 bootstrap samples of the training data, the number of decision tree variables is set appropriately. 3 and 4 variables per decision tree are typical.

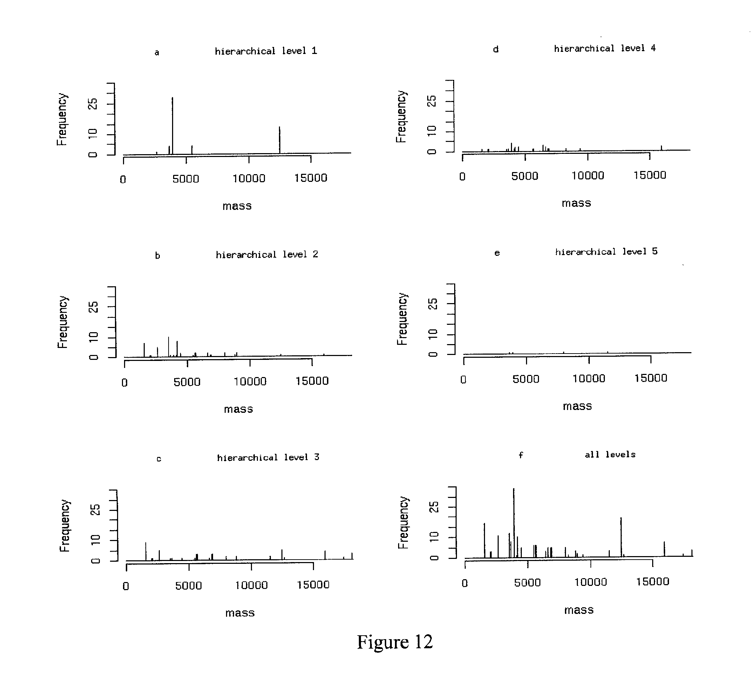

**Figure 12.** Variable importance. The frequency of variable selection is presented in histogram form for each hierarchical level (a-e) and for all hierarchical levels taken together (f).

### DESCRIPTION OF THE INVENTION

It is to be understood that the present invention is not limited to the particular materials and methods described or equipment, as these may vary. It is also to be understood that the terminology used herein is for the purpose of describing particular embodiments only, and is not intended to limit the scope of the present invention, which will be limited only by the appended claims.

It should be noted that as used herein and in the appended claims, the singular forms "a," "an," and "the" include plural reference unless the context clearly dictates otherwise. Thus, for example, a reference to "a host cell" includes a plethora of such host cells, and a reference to "an antibody" is a reference to one or more antibodies and derivatives thereof known to those skilled in the art, and so forth.

Unless defined otherwise, all technical and scientific terms used herein have the same meanings as commonly understood by one of ordinary skill in the art. Although any materials and methods, or equipment comparable to those specifically described herein can be used to practice or test the present invention, the preferred equipment, materials and methods are described below. All publications mentioned herein are cited for the purpose of describing and disclosing protocols, reagents, and current state of the art technologies that might be used in connection with the invention. Nothing herein is to be construed as an admission that the invention is not entitled to precede such disclosure by virtue of prior invention.

### Definitions

The term "biomolecule" refers to a molecule produced by a cell or living organism. Such molecules include, but are not limited to, molecules comprising nucleotides, amino acids, sugars, fatty acids, steroids, nucleic acids, polynucleotides, polypeptides, proteins, carbohydrates, lipids, and combinations thereof (e.g., glycoproteins, ribonucleoproteins, lipoproteins). Furthermore, the terms "nucleotide" or polynucleotide" refer to a nucleotide, oligonucleotide, polynucleotide, or any fragment thereof. These phrases also refer to DNA or RNA of genomic or synthetic origin which may be single-stranded or double-stranded and may represent the sense, or the antisense strand, to peptide polynucleotide sequences (i.e. peptide nucleic acids; PNAs), or to any DNA-like or RNA-like material.

The term "fragment" refers to a portion of a polypeptide (parent) sequence that comprises at least 10 consecutive amino acid residues and retains a biological activity and/or some functional characteristics of the parent polypeptide e.g. antigenicity or structural domain characteristics.

The terms "biological sample" and "test sample" refer to all biological fluids and excretions isolated from any given subject. In the context of the invention such samples include, but are not limited to, blood, blood serum, plasma, nipple aspirate, urine, semen, seminal fluid, seminal plasma, prostatic fluid, excreta, tears, saliva, sweat, biopsy, ascites, cerebrospinal fluid, milk, lymph, or tissue extract samples.

The term "specific binding" refers to the binding reaction between a biomolecule and a specific "binding molecule". Related to the invention are binding molecules that include, but are not limited to, proteins, peptides, nucleotides, nucleic acids, hormones, amino acids, sugars, fatty acids, steroids, polynucleotides, carbohydrates, lipids, or a combination thereof (e.g. glycoproteins, ribonucleoproteins, lipoproteins). Furthermore, a binding reaction is considered to be specific when the interaction between said molecules is substantial. In the context of the invention, a binding reaction is considered substantial when the reaction that takes place between said molecules is at least two times the background. Moreover, the term "specific binding conditions" refers to reaction conditions that permit the binding of said molecules such as pH, salt, detergent and other conditions known to those skilled in the art.

The term "interaction" relates to the direct or indirect binding or alteration of biological activity of a biomolecule.

The term "differential diagnosis" refers to a diagnostic decision between a healthy and different disease states, including various stages of a specific disease. A subject is diagnosed as healthy or to be suffering from a specific disease, or a specific stage of a disease based on a set of hypotheses that allow for the distinction between healthy and one or more stages of the disease. The choice between healthy and one or more stages of disease depends on a significant difference between each hypothesis. Under the same principle, a "differential diagnosis" may also refer to a diagnostic decision between one disease type as compared to another (e.g. stomach cancer vs. colon cancer).

The term "epithelial cancer" refers to a cancer that arises from epithelial cell origin and may include, but is not limited to, breast, lung, gastrointestinal, prostate, ovarian, cervical, endometrial cancers, bladder and/or other cancers of epithelial origin. Within the context of the invention epithelial cancers may be at different stages (e.g. ductal carcinoma in situ (DCIS)), as well as varying degrees of grading. In the context of the invention, an epithelial cancer may also be referred to as a neoplasm of epithelial origin.

The term "gastrointestinal cancer" refers to a cancer state associated with the gastrointestinal system of any given subject. In the context of the invention gastrointestinal cancers include, but are not limited to oesophageal, stomach, small intestine, colon, rectal, pancreatic, liver, gallbladder, and biliary tract cancers. Within the context of the invention gastrointestinal cancers may be at different stages, as well as varying degrees of grading.

The term "neoplasm" can be used interchangeably with "an acute and chronic inflammation of the epithelium" and refers to any new and abnormal growth, specifically a new growth of tissue in which the growth is uncontrolled and progressive.

The term "healthy individual" refers to a subject possessing good health. Such a subject demonstrates an absence of a disease, preferably an epithelial cancer or an acute and chronic inflammation of the epithelium. Moreover, subjects demonstrate an absence of breast, lung, gastrointestinal, prostate, ovarian, cervical, endometrial, and/or other cancers of epithelial origin.

The term "precancerous lesion" refers to a biological change within a cell and/or tissue such that said cell and/or tissue becomes susceptible to the development of a cancer. More specifically, a precancerous lesion is a preliminary stage of cancer (i.e. Dysplasia). Causes of precancerous lesions may include, but are not limited to, genetic predisposition and exposure to cancer-causing agents (carcinogens); such cancer causing agents include agents that cause genetic damage and induce neoplastic transformation of a cell. Furthermore, the phrase "neoplastic transformation of a cell" refers an alteration in normal cell physiology and includes, but is not limited to, self-sufficiency in growth signals, insensitivity to growth-inhibitory (anti-growth) signals, evasion of programmed cell death (apoptosis), limitless replicative potential, sustained angiogenesis, and tissue invasion and metastasis.

The term "Dysplasia" refers to morphological alterations within a tissue, which are characterised by a loss in the uniformity of individual cells, as well as a loss in their architectural orientation. Furthermore, dysplastic cells also exhibit a variation in size and shape.

The phrase "differentially present" refers to differences in the quantity of a biomolecule (of a particular apparent molecular mass) present in a sample from a subject as compared to a comparable sample. For example, a biomolecule is present at an elevated level, a decreased level or absent in samples of subjects having an epithelial cancer compared to samples of subjects who do not have a cancer of epithelial origin. Therefore in the context of the invention, the term "differentially present biomolecule" refers to the quantity biomolecule (of a particular apparent molecular mass) present within a sample taken from a subject having a disease or cancer of epithelial origin as compared to a comparable sample taken from a healthy subject. Within the context of the invention, a biomolecule is differentially present between two samples if the quantity of said biomolecule in one sample is statistically significantly different from the quantity of said biomolecule in another sample.

The term "diagnostic assay" can be used interchangeably with "diagnostic method" and refers to the detection of the presence or nature of a pathologic condition. Diagnostic assays differ in their sensitivity and specificity. Within the context of the invention the sensitivity of a diagnostic assay is defmed as the percentage of diseased subjects who test positive for an epithelial cancer or an acute and chronic inflammation of the epithelium and are considered "true positives". Subjects having an epithelial cancer or an acute and chronic inflammation of the epithelium but not detected by the diagnostic assay are considered "false negatives". Subjects who are not diseased and who test negative in the diagnostic assay are considered "true negatives". Furthermore, the term specificity of a diagnostic assay, as used herein, is defined as 1 minus the false positive rate, where the "false positive rate" is defined as the proportion of those subjects devoid of an epithelial cancer or an acute and chronic inflammation of the epithelium but who test positive in said assay.

The term "adsorbent" refers to any material that is capable of accumulating (binding) a biomolecule. The adsorbent typically coats a biologically active surface and is composed of a single material or a plurality of different materials that are capable of binding a biomolecule. Such materials include, but are not limited to, anion exchange materials, cation exchange materials, metal chelators, polynucleotides, oligonucleotides, peptides, antibodies, metal chelators etc.

The term "biologically active surface" refers to any two- or three-dimensional extension of a material that biomolecules can bind to, or interact with, due to the specific biochemical properties of this material and those of the biomolecules. Such biochemical properties include, but are not limited to, ionic character (charge), hydrophobicity, or hydrophilicity.

The term "binding molecule" refers to a molecule that displays an affinity for another molecule. With in the context of the invention such molecules may include, but are not limited to nucleotides, amino acids, sugars, fatty acids, steroids, nucleic acids, polypeptides, carbohydrates, lipids, and combinations thereof (e.g. glycoproteins, ribonucleoproteins, lipoproteins). Preferably, such binding molecules are antibodies.

The term "solution" refers to a homogeneous mixture of two or more substances. Solutions may include, but are not limited to buffers, substrate solutions, elution solutions, wash solutions, detection solutions, standardisation solutions, chemical solutions, solvents, etc. Furthermore, other solutions known to those skilled in the art are also included herein.

The term "mass profile" refers to a mass spectrum as a characteristic property of a given sample or a group of samples, especially when compared to the mass profile of a second sample or group of samples in any way different from the first sample or group of sample. In the context of the invention, the mass profile is obtained by treating the biological sample as follows. The sample is diluted it 1:5 in a denaturation buffer consisting of 7 M urea, 2 M thiourea, 4% CHAPS, 1% DTT, and 2% ampholine and subsequently diluted 1:10 in binding buffer consisting of 0.1 M Tis-HCl, 0.02% Triton X-100 at pH 8.5. Thus pre-treated sample is applied to a biologically active surface comprising positively charged quaternary ammonium groups (anion exchange surface) and incubated for 120 minutes. The biomolecules bound to the surface are analysed by gas phase ion spectrometry as described in another section. All but the dilution steps are performed at 20 to 24°C. Dilution steps are performed at 0 to 4°C.

The phrase "apparent molecular mass" refers to the molecular mass value in Dalton (Da) of a biomolecule as it may appear in a given method of investigation, e.g. size exclusion chromatography, gel electrophoresis, or mass spectrometry.

The term "chromatography" refers to any method of separating biomolecules within a given sample such that the original native state of a given biomolecule is retained. Separation of a biomolecule from other biomolecules within a given sample for the purpose of enrichment, purification and/or analysis, may be achieved by methods including, but not limited to, size exclusion chromatography, ion exchange chromatography, hydrophobic and hydrophilic interaction chromatography, metal affinity chromatography, wherein "metal" refers to metal ions (e.g. nickel, copper, gallium, or zinc) of all chemically possible valences, or ligand affinity chromatography wherein "ligand" refers to binding molecules, preferably proteins, antibodies, or DNA. Generally, chromatography uses biologically active surfaces as adsorbents to selectively accumulate certain biomolecules.

The term "mass spectrometry" refers to a method comprising employing an ionization source to generate gas phase ions from a biological entity of a sample presented on a biologically active surface and detecting the gas phase ions with a mass spectrometer.

The phrase "laser desorption mass spectrometry" refers to a method comprising the use of a laser as an ionization source to generate gas phase ions from a biomolecule presented on a biologically active surface and detecting the gas phase ions with a mass spectrometer.

The term "mass spectrometer" refers to a gas phase ion spectrometer that includes an inlet system, an ionisation source, an ion optic assembly, a mass analyser, and a detector.

Within the context of the invention, the terms "detect", "detection" or "detecting" refer to the identification of the presence, absence, or quantity of a biomolecule.

The term "energy absorbing molecule" or "EAM" refers to a molecule that absorbs energy from an energy source in a mass spectrometer thereby enabling desorption of a biomolecule from a biologically active surface. Cinnamic acid derivatives, sinapinic acid and dihydroxybenzoic acid are frequently used as energy-absorbing molecules in laser desorption of biomolecules. See U.S. Pat. No. 5,719,060 (Hutchens & Yip) for a further description of energy absorbing molecules.

The term "training set" refers to a subset of the respective entire available data set. This sunset is typically randomly selected, and is solely used for the purpose of classifier construction.

The term "test set" refers to a subset of the entire available data set consisting of those entries not included in the training set. Test data is applied to evaluate classifier performance.

The term "decision tree" refers to a flow-chart-like tree structure employed for classification. Decision trees consist of repeated splits of a data set into subsets. Each split consists of a simple rule applied to one variable, e.g., "if value of 'variable 1' larger than 'threshold 1' then go left else go right". Accordingly, the given feature space is partitioned into a set of rectangles with each rectangle assigned to one class.

The terms "ensemble", "tree ensemble" or "ensemble classifier" can be used interchangeably and refer to a classifier that consists of many simpler elementary classifiers, e.g., an ensemble of decision trees is a classifier consisting of decision trees. The result of the ensemble classifier is obtained by combining all the results of its constituent classifiers, e.g., by majority voting that weights all constituent classifiers equally. Majority voting is especially reasonable in the case of bagging, where constituent classifiers are then naturally weighted by the frequency with which they are generated.

The term "competitor" refers to a variable (in our case: mass) that can be used as an alternative splitting rule in a decision tree. In each step of decision tree construction, only the variable yielding best data splitting is selected. Competitors are non-selected variables with similar but lower performance than the selected variable. They point into the direction of alternative decision trees.

The term "surrogate" refers to a splitting rule that closely mimics the action of the primary split. A surrogate is a variable that can substitute a selected decision tree variable, e.g. in the case of missing values. Not only must a good surrogate split the parent node into descendant nodes similar in size and composition to the primary descendant nodes. In addition, the surrogate must also match the primary split on the specific cases that go to the left child and right child nodes.

The terms "peak" and "signal" may be used interchangeably and refer to any signal which is generated by a biomolecule when under investigation using a specific method, for example chromatography, mass spectrometry, or any type of spectroscopy like Ultraviolet/Visible Light (UV/Vis) spectroscopy, Fourier Transformed Infrared (FTIR) spectroscopy, Electron Paramagnetic Resonance (EPR) spectroscopy, or Nuclear Mass Resonance (NMR) spectroscopy.

Within the context of the invention, the terms "peak" and "signal" refer to the signal generated by a biomolecule of a certain molecular mass hitting the detector of a mass spectrometer, thus generating a signal intensity which correlates with the amount or concentration of said biomolecule of a given sample. A "peak" and "signal" is defined by two values: an apparent molecular mass value and an intensity value generated as described. The mass value is an elemental characteristic of a biological entity, whereas the intensity value accords to a certain amount or concentration of a biological entity with the corresponding apparent molecular mass value, and thus "peak" and "signal" always refer to the properties of this biological entity.

The term "cluster" refers to a signal or peak present in a certain set of mass spectra or mass profiles obtained from different samples belonging to two or more different groups (e.g. cancer and non cancer). Within the set, signals belonging to cluster can differ in their intensities, but not in the apparent molecular masses.

The term "variable" refers to a cluster which is subjected to a statistical analysis aiming towards a classification of samples into two or more different sample groups (e.g. cancer and non cancer) by using decision trees, wherein the sample feature relevant for classification is the intensity value of the variables in the analysed samples.

### Detailed Description of the invention

### a) Diagnostics

The present invention relates to methods for the differential diagnosis of epithelial cancers or an acute and chronic inflammation of the epithelium by detecting one or more differentially expressed biomolecules within a test sample of a given subject, comparing results with samples from healthy subjects, subjects having a precancerous lesion, subjects having an epithelial cancer, subjects having a metastasised epithelial cancer, or subjects having an acute and chronic inflammation of the epithelium, wherein the comparison allows for the differential diagnosis of a subject as healthy, having a precancerous lesion, having an epithelial cancer, having a metastasised epithelial cancer or an acute and chronic inflammation of the epithelium.

In one aspect of the invention, a method for the differential diagnosis of an epithelial cancer or an acute and chronic inflammation of the epithelium comprises obtaining a test sample from a given subject, contacting said sample with an adsorbent present on a biologically active surface under specific binding conditions, allowing the biomolecules within the test sample to bind to said adsorbent, detecting one or more bound biomolecules using a detection method, wherein the detection method generates a mass profile of said sample, transforming mass profile data into a computer-readable form comparing the mass profile of said sample with a database containing mass profiles from comparable samples specific for healthy subjects, subjects having a precancerous lesion, subjects having an epithelial cancer, subjects having a metastasised epithelial cancer, or subjects having an acute and chronic inflammation of the epithelium. A comparison of mass profiles allows for the medical practitioner to determine if a subject is healthy, has a precancerous lesion, an epithelial cancer, a metastasised epithelial cancer or an acute and chronic inflammation of the epithelium based on the presence, absence or quantity of specific biomolecules.

In more than one embodiment, a single biomolecule or a combination of more than one biomolecule selected from the group having an apparent molecular mass of 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ± 70 Da, 15959 Da ± 80 Da, 16164 Da ± 81 Da, 17279 Da ± 86 Da, 17406 Da ± 87 Da, 17630 Da ± 88 Da, or 18133 Da ± 91 Da may be detected within a given sample. Detection of a single or a combination of more than one biomolecule of the invention is based on specific sample pre-treatment conditions, the pH of binding conditions, and the type of biologically active surface used for the detection of biomolecules. For example, prior to the detection of the biomolecules described herein, a given sample is pre-treated by diluting 1:5 in a denaturation buffer consisting of 7 M urea, 2 M thiourea, 4% CHAPS, 1% DTT, and 2% ampholine. The denatured sample is then diluted 1:10 in a specific binding buffer (0.1 M Tis-HCl, 0.02% Triton X-100, pH 8.5), applied to a biologically active surface comprising of positively-charged quaternary ammonium groups (cationic) and incubated using specific buffer conditions (0.1 M Tis-HCl, 0.02% Triton X-100, pH 8.5) to allow for binding of said biomolecules to the above-mentioned biologically active surface.

According to the invention, a biomolecule with the molecular mass of 1516 Da ± 8 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 1535 Da ± 8 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 2020 Da ± 10 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 2022 Da ± 10 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 2050 Da ± 10 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 3946 Da ± 20 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4104 Da ± 21 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4154 Da ± 21 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4298 Da ± 21 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4360 Da ± 22 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4477 Da ± 22 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4867 Da ± 24 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4958 Da ± 25 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4968 Da ± 25 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 5474 Da ± 27 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 5491 Da ± 27 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 5650 Da ± 28 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 6449 Da ± 32 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 6876 Da ± 34 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 7001 Da ± 35 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 7969 Da ± 40 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 8232 Da ± 41 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 8711 Da ± 44 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 10665 Da ± 53 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 12471 Da ± 62 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 12504 Da ± 63 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 12669 Da ± 63 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 13989 Da ± 70 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 15959 Da ± 80 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 16164 Da ± 81 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 17279 Da ± 86 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 17406 Da ± 87 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 17630 Da ± 88 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 18133 Da ± 91 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

A biomolecule of the invention may include any molecule that is produced by a cell or living organism, and may have any biochemical property (e.g. phosphorylated proteins, positively charged molecules, negatively charged molecules, hydrophobicity, hydrophilicity), but preferably biochemical properties that allow binding of the biomolecule to a biologically active surface comprising positively charged quaternary ammonium groups after denaturation in 7 M urea, 2 M thiourea, 4% CHAPS, 1% DTT, and 2% Ampholine and dilution in 0.1 M Tis-HCl, 0.02% Triton X-100 at pH 8.5 at 0 to 4°C followed by incubation on said biologically active surface for 120 minutes at 20 to 24°C. Such molecules include, but are not limited to, molecules comprising nucleotides, amino acids, sugars, fatty acids, steroids, nucleic acids, polynucleotides (DNA or RNA), polypeptides, proteins, antibodies, carbohydrates, lipids, and combinations thereof (e.g., glycoproteins, ribonucleoproteins, lipoproteins). Preferably a biomolecule may be a nucleotide, polynucleotide, peptide, protein or fragments thereof. Even more preferred are peptide or protein biomolecules or fragments thereof.

The methods for detecting these biomolecules have many applications. For example, a single biomolecule or a combination of more than one biomolecule selected from the group having an apparent molecular mass of 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ± 70 Da, 15959 Da ± 80 Da, 16164 Da ± 81 Da, 17279 Da ± 86 Da, 17406 Da ± 87 Da, 17630 Da ± 88 Da, or 18133 Da ± 91 Da can be measured to differentiate between healthy subjects, subjects having a precancerous lesion, subjects having an epithelial cancer, subjects having a metastasized epithelial cancer or subjects with a disease of epithelial origin, and thus are useful as an aid in the diagnosis of an epithelial cancer and/or a disease of epithelial origin within a subject. Alternatively, said biomolecules may be used to diagnose a subject as healthy.

For example, a biomolecule having the apparent molecular mass of about e.g. 3946 Da is present only in biological samples from patients having a metastasised epithelial cancer. Mass profiling of two test samples from different subjects, X and Y, reveals the presence of a biomolecule with the apparent molecular mass of about 3946 Da in a sample from test subject X, and the absence of said biomolecule in test sample from subject Y. The medical practitioner is able to diagnose subject X as having a metastasised epithelial cancer and subject Y as not having a metastasised epithelial cancer. In yet another example, three biomolecules having the apparent molecular mass of about 7969 Da, 12471 Da and 18133 Da are present in varying quantities in samples specific for precancerous lesions and "early" epithelial cancers. The biomolecule having the apparent molecular mass of 7969 Da is more present in samples specific for precancerous lesions than for "early" epithelial cancers. A biomolecule having an apparent molecular mass of 12471 Da is detected in samples from subjects having "early" epithelial cancers but not in those having a precancerous lesion, whereas the biomolecule having the molecular mass of 18133 Da is present in about the same quantity in both sample types. Such biomolecules are not present in samples from healthy subjects, only those of apparent molecular mass of 6449 Da and 15959 Da. Analysis of a test sample reveals the presence of biomolecules having the molecular mass of 18133 Da, 7969 Da and 12471 Da. Comparison of the quantity of the biomolecules within said sample reveals that the biomolecule with an apparent molecular mass of 7969 Da is present at lower levels than those found in samples from subjects having a precancerous lesion. The medical practitioner is able to diagnose the test subject as having an "early" epithelial cancer. These examples are solely used for the purpose of clarification and are not intended to limit the scope of this invention,

In another aspect of the invention, an immunoassay can be used to determine the presence or absence of a biomolecule within a test sample of a subject. First, the presence or absence of a biomolecule within a sample can be detected using the various immunoassay methods known to those skilled in the art (i.e. ELISA, western blots). If a biomolecule is present in the test sample, it will form an antibody-marker complex with an antibody that specifically binds a biomolecule under suitable incubation conditions. The amount of an antibody-biomolecule complex can be determined by comparing to a standard.

The invention provides a method for the differential diagnosis of an epithelial cancer and/or an acute and chronic inflammation of the epithelium comprising detecting of one or more differentially expressed biomolecules within a sample. This method comprises obtaining a test sample from a subject, contacting said sample with a binding molecule specific for a differentially expressed polypeptide, detecting an interaction between the binding molecule and its specific polypeptide, wherein the detection of an interaction indicates the presence or absence of said polypeptide, thereby allowing for the differential diagnosis of a subject as healthy, having a precancerous lesion, having an epithelial cancer, having a metastasised epithelial cancer and/or an acute and chronic inflammation of the epithelium. Binding molecules include, but are not limited to, proteins, peptides, nucleotides, nucleic acids, hormones, amino acids, sugars, fatty acids, steroids, polynucleotides, carbohydrates, lipids, or a combination thereof (e.g. glycoproteins, ribonucleoproteins, lipoproteins), compounds or synthetic molecules. Preferably, binding molecules are antibodies specific for biomolecules selected from the group of having an apparent molecular mass of 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ± 70 Da, 15959 Da ± 80 Da, 16164 Da ± 81 Da, 17279 Da ± 86 Da, 17406 Da ± 87 Da, 17630 Da ± 88 Da, or 18133 Da ± 91 Da.

In another aspect of the invention, a method for detecting the differential presence of one or more biomolecules selected from the group having an apparent molecular mass of 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ±70 Da, 15959 Da ± 80 Da, 16164 Da ± 81 Da, 17279 Da ± 86 Da, 17406 Da ± 87 Da, 17630 Da ± 88 Da, or 18133 Da ± 91 Da in a test sample of a subject involves contacting the test sample with a compound or agent capable of detecting said biomolecule such that the presence of said biomolecule is directly and/or indirectly labelled. For example a fluorescently labelled secondary antibody can be used to detect a primary antibody bound to its specific biomolecule. Furthermore, such detection methods can be used to detect a variety of biomolecules within a test sample both *in vitro* as well as *in vivo.*

In more than one embodiment of the invention, the test sample used for the differential diagnosis of an epithelial cancer and/or an acute and chronic inflammation of the epithelium of a subject may be of blood, blood serum, plasma, nipple aspirate, urine, semen, seminal fluid, seminal plasma, prostatic fluid, excreta, tears, saliva, sweat, biopsy, ascites, cerebrospinal fluid, milk, lymph, or tissue extract origin. Preferably, test samples are of blood, blood serum, plasma, urine, excreta, prostatic fluid, biopsy, ascites, lymph or tissue extract origin. More preferred are blood, blood serum, plasma, urine, excreta, biopsy, lymph or tissue extract samples. Even more preferred are blood serum, urine, excreta or biopsy samples. Overall preferred are blood serum samples.

Furthermore, test samples used for the methods of the invention are isolated from subjects of mammalian origin, preferably of primate origin. Even more preferred are subjects of human origin.

In addition, the methods for the methods of the invention of healthy subjects, subjects having a precancerous lesion, subjects having an epithelial cancer, subjects having a metastasized epithelial cancer or subjects having an acute and chronic inflammation of the epithelium described herein may be combined with other diagnostic methods to improve the outcome of the differential diagnosis. Other diagnostic methods are known to those skilled in the art.

### b) Database

In another aspect of the invention, a database comprising of mass profiles specific for healthy subjects, subjects having a precancerous lesion, subjects having an epithelial cancer, subjects having a metastasised epithelial cancer, or subjects having an acute and chronic inflammation of the epithelium is generated by contacting biological samples isolated from above-mentioned subjects with an adsorbent on a biologically active surface under specific binding conditions, allowing the biomolecules within said sample to bind said adsorbent, detecting one or more bound biomolecules using a detection method wherein the detection method generates a mass profile of said sample, transforming the mass profile data into a computer-readable form and applying a mathematical algorithm to classify the mass profile as specific for healthy subjects, subjects having a precancerous lesion, subjects having an epithelial cancer, subjects having a metastasised epithelial cancer, or subjects having an acute inflammatory disease of epithelial origin.

According to the invention, the classification of said mass profiles is performed using the "CART" decision tree approach (classification and regression trees; Breiman et al., 1984) and is known to those skilled in the art. Furthermore, bagging of classifiers is applied to overcome typical instabilities of forward variable selection procedures, thereby increasing overall classifier performance (Breiman, 1994).

In more than one embodiment, one or more biomolecules selected from the group having an apparent molecular mass of 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ± 70 Da, 15959 Da ± 80 Da, 16164 Da ± 81 Da, 17279 Da ± 86 Da, 17406 Da ± 87 Da, 17630 Da ± 88 Da, or 18133 Da ± 91 Da may be detected within a given biological sample. Detection of said biomolecules of the invention is based on specific sample pre-treatment conditions, the pH of binding conditions, and the type of biologically active surface used for the detection of biomolecules.

Within the context of the invention, biomolecules within a given sample are bound to an adsorbent on a biologically active surface under specific binding conditions, for example, the biomolecules within a given sample are applied to a biologically active surface comprising positively-charged quaternary ammonium groups (cationic) and incubated with 0.1 M Tris-HCl, 0.02% Triton X-100 at a pH of 8.5 to allow for specific binding. Biomolecules that bind to said biologically active surface under these conditions are negatively charged molecules. It should be noted that although the biomolecules of the invention are bound to a cationic adsorbent comprising of positively-charged quaternary ammonium groups, the biomolecules are capable of binding other types of adsorbents, as described in another section using binding conditions known to those skilled in the art. Accordingly, some embodiments of the invention are not limited to the use of cationic adsorbents

According to the invention, a biomolecule with the molecular mass of 1516 Da ± 8 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 1535 Da ± 8 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 2020 Da ± 10 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 2022 Da ± 10 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 2050 Da ± 10 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 3946 Da ± 20 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4104 Da ± 21 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4154 Da ± 21 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4298 Da ± 21 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4360 Da ± 22 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4477 Da ± 22 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4867 Da ± 24 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4958 Da ± 25 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4968 Da ± 25 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 5474 Da ± 27 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 5491 Da ± 27 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 5650 Da ± 28 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 6449 Da ± 32 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 6876 Da ± 34 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 7001 Da ± 35 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 7969 Da ± 40 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 8232 Da ± 41 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 8711 Da ± 44 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 10665 Da ± 53 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 12471 Da ± 62 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 12504 Da ± 63 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 12669 Da ± 63 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 13989 Da ± 70 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 15959 Da ± 80 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 16164 Da ± 81 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 17279 Da ± 86 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 17406 Da ± 87 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 17630 Da ± 88 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 18133 Da ± 91 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

In one embodiment of the invention, biological samples used to generate a database of mass profiles for healthy subjects, subjects having a precancerous lesion, subjects having an epithelial cancer, subjects having a metastasised epithelial cancer or subjects having an acute and chronic inflammation of the epithelium, may be of blood, blood serum, plasma, nipple aspirate, urine, semen, seminal fluid, seminal plasma, prostatic fluid, excreta, tears, saliva, sweat, biopsy, ascites, cerebrospinal fluid, milk, lymph, or tissue extract origin. Preferably, biological samples are of blood, blood serum, plasma, urine, excreta, prostatic fluid, biopsy, ascites, lymph or tissue extract origin. More preferred are blood, blood serum, plasma, urine, excreta, biopsy, lymph or tissue extract samples. Even more preferred are blood serum, urine, excreta or biopsy samples. Overall preferred are blood serum samples.

Furthermore, the biological samples related to the invention are isolated from subjects considered to be healthy, having a precancerous lesion, having an epithelial cancer, having a metastasised epithelial cancer or having an acute and chronic inflammation of the epithelium. Said subjects are of mammalian origin, preferably of primate origin. Even more preferred are subjects of human origin.

A subject of the invention that is said to have a precancerous lesion, displays preliminary stages of cancer (i.e. Dysplasia), wherein a cell and/or tissue has become susceptible to the development of a cancer as a result of either a genetic predisposition, exposure to a cancer-causing agent (carcinogen) or both.

A genetic pre-disposition may include a predisposition for an autosomal dominant inherited cancer syndrome which is generally indicated by a strong family history of uncommon cancer and/or an association with a specific marker phenotype (e.g. familial adenomatous polyps of the colon), a familial cancer wherein an evident clustering of cancer is observed but the role of inherited predisposition may not be clear (e.g. breast cancer, ovarian cancer, or colon cancer), or an autosomal recessive syndrome characterised by chromosomal or DNA instability. Whereas, cancer-causing agents include agents that cause genetic damage and induce neoplastic transformation of a cell. Such agents fall into three categories: 1) chemical carcinogens such as alkylating agents, polycyclic aromatic hydrocarbons, aromatic amines, azo dyes, nitrosamines and amides, asbestos, vinyl chloride, chromium, nickel, arsenic, and naturally occurring carcinogens (e.g. aflotoxin B1); 2) radiation such as ultraviolet (UV) and ionisation radiation including electromagnetic (e.g. x-rays, γ-rays) and particulate radiation (e.g. α and β particles, protons, neutrons); 3) viral and microbial carcinogens such as human Papillomavirus (HPV), Epstein-Barr virus (EBV), hepatitis B virus (HBV), human T-cell leukaemia virus type 1 (HTLV-1), or *Helicobacter pylori.*

Alternatively, a subject within the invention that is said to have an epithelial cancer possesses a cancer that arises from epithelial cell origin. Such cancers may include, but are not limited to, breast, lung, gastrointestinal, prostate, ovarian, cervical, endometrial cancers, bladder and/or other cancers of epithelial origin. In addition, gastrointestinal cancers can be further sub-divided according to the location of the cancer within the gastrointestinal tract. For example, gastrointestinal cancers include, but are not limited to, oesophageal, stomach, small intestine, colon, rectal, pancreatic, gallbladder, liver, and biliary tract cancers. An epithelial cancer related to the invention may also be referred to as a neoplasm of epithelial origin.

Within the context of the invention, cancers of epithelial origin may also be of various stages, wherein the staging is based on the size of the primary lesion, its extent of spread to regional lymph nodes, and the presence or absence of blood-borne metastases (metastatic epithelial cancers) [e.g. ductal carcinoma in situ (DCIS)]. The various stages of a cancer may be identified using staging systems known to those skilled in the art [e.g. Union Internationale Contre Cancer (UICC) system or American Joint Committee on Cancer (AJC)]. Also included are different grades of said cancers, wherein the grade of a cancer is based on the degree of differentiation of an acute and chronic inflammation of the epithelium cells and the number of mitoses within the an acute and chronic inflammation of the epithelium as a correlation to a neoplasm's aggression.

The invention pertains to breast, lung, gastrointestinal, prostate, ovarian, cervical, endometrial or bladder cancer, and any stage and/or grade thereof. Preferred cancers of the invention are breast, lung, gastrointestinal, prostate, ovarian cancer, and any stage and/or grade thereof. More preferred are breast, lung, gastrointestinal cancer and any stage and/or grade thereof. Even more preferred are gastrointestinal cancers and any stage and/or grade thereof.

Furthermore, other cancers of epithelial origin known to those skilled in the art also within the context of the invention.

Healthy individuals, as related to certain embodiments of the invention, are those that possess good health, and demonstrate an absence of an epithelial cancer or an acute and chronic inflammation of the epithelium. Moreover, subjects demonstrate an absence of breast, lung, gastrointestinal, prostate, ovarian, cervical, endometrial, and/or other cancers of epithelial origin.

### c) Biomolecules

The differential expression of biomolecules in samples from healthy subjects, subjects having precancerous lesions, subjects having an epithelial cancer, subjects having metastasised epithelial cancer, and subjects having an acute and chronic inflammation of the epithelium, allows for the differential diagnosis of an acute inflammatory disease or a cancer of epithelial origin in a subject.

Biomolecules are said to be specific for a particular clinical state (e.g. healthy, precancerous lesion, epithelial cancer, metastasised epithelial cancer, an acute and chronic inflammation of the epithelium) when they are present at different levels within samples taken from subjects in one clinical state as compared to samples taken from subjects from other clinical states (e.g. in subjects with a precancerous lesion vs. in subjects with metastasised epithelial cancers). Biomolecules may be present at elevated levels, at decreased levels, or altogether absent within a sample taken from a subject in a particular clinical state (e.g. healthy, precancerous lesion, epithelial cancer, metastasised epithelial cancer, an acute and chronic inflammation of the epithelium). For example, biomolecules A and B are found at elevated levels in samples isolated from healthy subjects as compared to samples isolated from subjects having a precancerous lesion, an epithelial cancer, a metastatic epithelial cancer or an acute and chronic inflammation of the epithelium. Whereas, biomolecules X, Y, Z are found at elevated levels and/or more frequently in samples isolated from subjects having precancerous lesions as opposed to subjects in good health, having an epithelial cancer, a metastasised epithelial cancer or an acute and chronic inflammation of the epithelium. Biomolecules A and B are said to be specific for healthy subjects, whereas biomolecules X, Y, Z are specific for subjects having a precancerous lesion.

Accordingly, the differential presence of one or more biomolecules found in a test sample compared to samples from healthy subjects, subjects with a precancerous lesion, an epithelial cancer, a metastasized epithelial cancer, or an acute and chronic inflammation of the epithelium, or the mere detection of one or more biomolecules in the test sample provides useful information regarding probability of whether a subject being tested has a precancerous lesion, epithelial cancer, a metastasized epithelial cancer or an acute and chronic inflammation of the epithelium. The probability that a subject being tested has a precancerous lesion, an epithelial cancer, a metastasized epithelial cancer or an acute and chronic inflammation of the epithelium depends on whether the quantity of one or more biomolecules in a test sample taken from said subject is statistically significantly different from the quantity of one or more biomolecules in a biological sample taken from healthy subjects, subjects having a precancerous lesion, an epithelial cancer, a metastasised epithelial cancer, or an acute and chronic inflammation of the epithelium.

A biomolecule of the invention may be any molecule that is produced by a cell or living organism, and may have any biochemical property (e.g. phosphorylated proteins, positively charged molecules, negatively charged molecules, hydrophobicity, hydrophilicity), but preferably biochemical properties that allow binding of the biomolecule to a biologically active surface comprising positively charged quaternary ammonium groups after denaturation in 7 M urea, 2 M thiourea, 4% CHAPS, 1% DTT, and 2% Ampholine and dilution in 0.1 M Tis-HCl, 0.02% Triton X-100 at pH 8.5 at 0 to 4°C followed by incubation on said biologically active surface for 120 minutes at 20 to 24°C. Such molecules include, but are not limited to, molecules comprising nucleotides, amino acids, sugars, fatty acids, steroids, nucleic acids, polynucleotides (DNA or RNA), polypeptides, proteins, antibodies, carbohydrates, lipids, and combinations thereof (e.g., glycoproteins, ribonucleoproteins, lipoproteins). Preferably a biomolecule may be a nucleotide, polynucleotide, peptide, protein or fragments thereof. Even more preferred are peptide or protein biomolecules.

The biomolecules of the invention can be detected based on specific sample pre-treatment conditions, the pH of binding conditions, the type of biologically active surface used for the detection of biomolecules within a given sample and their molecular mass. For example, prior to the detection of the biomolecules described herein, a given sample is pre-treated by diluting 1:5 in a denaturation buffer consisting of 7 M urea, 2 M thiourea, 4% CHAPS, 1% DTT, and 2% ampholine. The denatured sample is then diluted 1:10 in 0.1 M Tis-HCl, 0.02% Triton X-100, pH 8.5, applied to a biologically active surface comprising positively-charged quaternary ammonium groups (cationic) and incubated using specific buffer conditions (0.1 M Tis-HCl, 0.02% Triton X-100, pH 8.5) to allow for binding of said biomolecules to the above-mentioned biologically active surface. It should be noted that although the biomolecules of the invention are detected using a cationic adsorbent positively charged quaternary ammonium groups, as well as specific pre-treatment and binding conditions, the biomolecules are capable of binding other types of adsorbents, as described below, using alternative pre-treatment and binding conditions known to those skilled in the art. Accordingly, some embodiments of the invention are not limited to the use of cationic adsorbents.

The biomolecules of the invention include biomolecules having a molecular mass selected from the group consisting of 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ± 70 Da, 15959 Da ± 80 Da, 16164 Da ± 81 Da, 17279 Da ± 86 Da, 17406 Da ± 87 Da, 17630 Da ± 88 Da, or 18133 Da ± 91 Da.

According to the invention, a biomolecule with the molecular mass of 1516 Da ± 8 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 1535 Da ± 8 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 2020 Da ± 10 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 2022 Da ± 10 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 2050 Da ± 10 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 3946 Da ± 20 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4104 Da ± 21 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4154 Da ± 21 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4298 Da ± 21 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4360 Da ± 22 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4477 Da ± 22 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4867 Da ± 24 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4958 Da ± 25 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 4968 Da ± 25 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 5474 Da ± 27 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 5491 Da ± 27 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 5650 Da ± 28 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 6449 Da ± 32 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 6876 Da ± 34 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 7001 Da ± 35 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 7969 Da ± 40 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 8232 Da ± 41 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 8711 Da ± 44 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 10665 Da ± 53 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 12471 Da ± 62 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 12504 Da ± 63 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 12669 Da ± 63 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 13989 Da ± 70 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 15959 Da ± 80 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 16164 Da ± 81 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 17279 Da ± 86 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 17406 Da ± 87 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 17630 Da ± 88 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

According to the invention, a biomolecule with the molecular mass of 18133 Da ± 91 Da is detected by diluting the biological sample 1:5 in a denaturation buffer consisting of 7 M urea, 2 M thiourea, 4% CHAPS, 1% DTT, and 2% Ampholine, and then 1:10 in binding buffer consisting of 0.1 M Tis-HCl, 0.02% Triton X-100 at pH 8.5 at 0 to 4°C, applying thus treated sample to a biologically active surface comprising positively charged (cationic) quaternary ammonium groups (anion exchanging), incubating for 120 minutes at 20 to 24°C, and subjecting the bound biomolecules to gas phase ion spectrometry as described in another section.

Although said biomolecules were first identified in blood serum samples, their detection is not limited to said sample type. The biomolecules may also be detected in other samples types, such as blood, blood serum, plasma, nipple aspirate, urine, semen, seminal fluid, seminal plasma, prostatic fluid, excreta, tears, saliva, sweat, biopsy, ascites, cerebrospinal fluid, milk, lymph, or tissue extract. Preferably, samples are of blood, blood serum, plasma, urine, excreta, prostatic fluid, biopsy, ascites, lymph or tissue extract origin. More preferred are blood, blood serum, plasma, urine, excreta, biopsy, lymph or tissue extract samples. Even more preferred are blood serum, urine, excreta or biopsy samples. Overall preferred are blood serum samples.

Since the biomolecules can be sufficiently characterized by their mass and biochemical characteristics such as the type of biologically active surface they bind to or the pH of binding conditions, it is not necessary to identify the biomolecules in order to be able to identify them in a sample. It should be noted that molecular mass and binding properties are characteristic properties of these biomolecules and not limitations on the means of detection or isolation. Furthermore, using the methods described herein, or other methods known in the art, the absolute identity of the markers can be determined. This is important when one wishes to develop and/or screen for specific binding molecules, or to develop a an assay for the detection of said biomolecules using specific binding molecules.

### d) Biologically Active Surfaces

In one embodiment of the invention, biologically active surfaces include, but are not restricted to, surfaces that contain adsorbents such as quaternary ammonium groups (anion exchange surfaces), carboxylate groups (cation exchange surfaces), alkyl or aryl chains (hydrophobic interaction, reverse phase chemistry), groups such as nitriloacetic acid that immobilize metal ions such as nickel, gallium, copper, or zinc (metal affinity interaction), or biomolecules such as proteins, preferably antibodies, or nucleic acids, preferably protein binding sequences, covalently bound to the surface via carbonyl diimidazole moieties or epoxy groups (specific affmity interaction). Preferred are adsorbents comprising anion exchange surfaces.

These surfaces may be located on matrices like polysaccharides such as sepharose, e.g. anion exchange surfaces or hydrophobic interaction surfaces, or solid metals, e.g. antibodies coupled to magnetic beads. Surfaces may also include gold-plated surfaces such as those used for Biacore Sensor Chip technology. Other surfaces known to those skilled in the art are also included within the scope of the invention.

Biologically active surfaces are able to adsorb biomolecules like amino acids, sugars, fatty acids, steroids, nucleic acids, polynucleotides, polypeptides, carbohydrates, lipids, and combinations thereof (e.g., glycoproteins, ribonucleoproteins, lipoproteins).

In another embodiment, devices that use biologically active surfaces to selectively adsorb biomolecules may be chromatography columns for Fast Protein Liquid Chromatography (FPLC) and High Pressure Liquid Chromatography (HPLC), where the matrix, e.g. a polysaccharide, carrying the biologically active surface, is filled into vessels (usually referred to as "columns") made of glass, steel, or synthetic materials like polyetheretherketone (PEEK).

In yet another embodiment, devices that use biologically active surfaces to selectively adsorb biomolecules may be metal strips carrying thin layers of the biologically active surface on one or more spots of the strip surface to be used as probes for gas phase ion spectrometry analysis, for example the SAX2 ProteinChip array (Ciphergen Biosystems, Inc.) for SELDI analysis.

### e) Mass Profiling

In one embodiment, the mass profile of a sample may be generated using an array-based assay in which the biomolecules of a given sample are bound by biochemical or affmity interactions to an adsorbent present on a biologically active surface located on a solid platform ("array" or "probe"). After the biomolecules have bound to the adsorbent, they are detected using gas phase ion spectrometry. Biomolecules or other substances bound to the adsorbents on the probes can be analyzed using a gas phase ion spectrometer. This includes, e.g., mass spectrometers, ion mobility spectrometers, or total ion current measuring devices. The quantity and characteristics of the biomolecule can be determined using gas phase ion spectrometry. Other substances in addition to the biomolecule of interest can also be detected by gas phase ion spectrometry.

In one embodiment, a mass spectrometer can be used to detect biomolecules on the probe. In a typical mass spectrometer, a probe with a biomolecule is introduced into an inlet system of the mass spectrometer. The biomolecule is then ionized by an ionization source, such as a laser, fast atom bombardment, or plasma. The generated ions are collected by an ion optic assembly, and then a mass analyzer disperses and analyzes the passing ions. Within the scope of this invention, the ionisation course that ionises the biomolecule is a laser.

The ions exiting the mass analyzer are detected by a ion detector. The ion detector then translates information of the detected ions into mass-to-charge ratios. Detection of the presence of a biomolecule or other substances will typically involve detection of signal intensity. This, in turn, can reflect the quantity and character of a biomolecule bound to the probe.

In another embodiment, the mass profile of a sample may be generated using a liquid-chromatography (LC)-based assay in which the biomolecules of a given sample are bound by biochemical or affinity interactions to an adsorbent located in a vessel made of glass, steel, or synthetic material; known to those skilled in the art as a chromatography column. The biomolecules are eluted from the biologically active surface by washing the vessel with appropriate solutions known to those skilled in the art. Such solutions include but are not limited to, buffers, e.g. Tris (hydroxymethyl) aminomethane hydrochloride (TRIS-HCl), buffers containing salt, e.g. sodium chloride (NaCl), or organic solvents, e.g. acetonitrile. Biomolecule mass profiles are generated by application of the eluting biomolecules of the sample by direct connection via an electrospray device to a mass spectrometer (LC/ESI-MS).

Conditions that promote binding of biomolecules to an adsorbent are known to those skilled in the art (reference) and ordinarily include parameters such as pH, the concentration of salt, organic solvent, or other competitors for binding of the biomolecule to the adsorbent. Within the scope of the invention, incubation temperatures are of at least 0 to 100°C, preferably of at least 4 to 60°C, and most preferably of at least 15 to 30°C. Varying additional parameters, such as incubation time, the concentration of detergent, e.g., 3-[(3-Cholamidopropyl) dimethylammonio]-2-hydroxy-1-propanesulfonate (CHAPS), or reducing agents, e.g. dithiothreitol (DTT), are also known to those skilled in the art. Various degrees of binding can be accomplished by combining the above stated conditions as needed, and will be readily apparent to those skilled in the art.

### f) Methods for detecting biomolecules within a sample

In yet another aspect, the invention relates to methods for detecting differentially present biomolecules in a test sample and/or biological sample. Within the context of the invention, any suitable method can be used to detect one or more of the biomolecules described herein. For example, gas phase ion spectrometry can be used. This technique includes, e.g., laser desorption/ionization mass spectrometry. Preferably, the test and/or biological sample is prepared prior to gas phase ion spectrometry, e.g., pre-fractionation, two-dimensional gel chromatography, high performance liquid chromatography, etc. to assist detection of said biomolecules. Detection of said biomolecules can also be achieved using methods other than gas phase ion spectrometry. For example, immunoassays can be used to detect the biomolecules within a sample.

In one embodiment, the test and/or biological sample is prepared prior to contacting a biologically active surface and is in aqueous form. Examples said samples include, but are not limited to, blood, blood serum, plasma, nipple aspirate, urine, semen, seminal fluid, seminal plasma, prostatic fluid, tears, saliva, sweat, ascites, cerebrospinal fluid, milk, lymph, or tissue extract samples. Furthermore, solid test and/or biological samples, such as excreta or biopsy samples can be solubilised in or admixed with an eluent using methods known to those skilled in the art such that said samples may be easily applied to a biologically active surface. Test and/or biological samples in the aqueous form can be further prepared using specific solutions for denaturation (pre-treatment) like sodium dodecyl sulfate, mercaptoethanol, urea, etc. For example, a test and/or biological sample of the invention can be denatured prior to contacting a biologically active surface comprising of quaternary ammonium groups by diluting said sample 1:5 with a buffer consisting of 7 M urea, 2 M thiourea, 4% CHAPS, 1% DTT and 2% ampholine.

The sample is contacted with a biologically active surface using any techniques including bathing, soaking, dipping, spraying, washing over, or pipetting, etc. Generally, a volume of sample containing from a few atomoles to 100 picomoles of a biomolecule in about 1 to 500 µl is sufficient for detecting binding of the biomolecule to the adsorbent.

The pH value of the solvent in which the sample contacts the biologically active surface is a function of the specific sample and the selected biologically active surface. Typically, a sample is contacted with a biologically active surface under pH values between 0 and 14, preferably between about 4 and 10, more preferably between 4.5 and 9.0, and most preferably, at pH 8.5. The pH value depends on the type of adsorbent present on a biologically active surface and can be adjusted accordingly.

The sample can contact the adsorbent present on a biologically active for a period of time sufficient to allow the marker to bind to the adsorbent. Typically, the sample and the biologically active surface are contacted for a period of between about 1 second and about 12 hours, preferably, between about 30 seconds and about 3 hours, and most preferably for 120 minutes.

The temperature at which the sample contacts the biologically active surface (incubation temperature) is a function of the specific sample and the selected biologically active surface. Typically, the washing solution can be at a temperature of between 0 and 100°C, preferably between 4 and 37°C, and most preferably between 20 and 24°C.

For example, a biologically active surface comprising of quaternary ammonium groups (anion exchange surface) will bind the biomolecules described herein when the pH value is between 6.5 and 9.0. Optimal binding of the biomolecules of the present invention occurs at a pH of 8.5. Furthermore, a sample is contacted with said biologically active surface for 120 min. at a temperature of 20 - 24 °C.

Following contacting a sample or sample solution with a biological surface, it is preferred to remove any unbound biomolecules so that only the bound biomolecules remain on the biologically active surface. Washing unbound biomolecules are removed by methods known to those skilled in the art such as bathing, soaking, dipping, rinsing, spraying, or washing the biologically active surface with an eluent or a washing solution. A microfluidics process is preferably used when a washing solution such as an eluent is introduced to small spots of adsorbents on the biologically active surface. Typically, the washing solution can be at a temperature of between 0 and 100°C, preferably between 4 and 37°C, and most preferably between 20 and 24°C.

Washing solution or eluents used to wash the unbound biomolecules from a biologically active surface include, but are not limited to, organic solutions, aqueous solutions such as buffers wherein a buffer may contain detergents, salts, or reducing agents in appropriate concentrations as those known to those skilled in the art.

Aqueous solutions are preferred for washing biologically active surfaces. Exemplary aqueous solutions include, but not limited to, HEPES buffer, Tris buffer, phosphate buffered saline (PBS), and modifications thereof. The selection of a particular washing solution or an eluent is dependent on other experimental conditions (e. g., types of adsorbents used or biomolecules to be detected), and can be determined by those of skill in the art. For example, if a biologically active surface comprising a quaternary ammonium group as adsorbent (anion exchange surface) is used, then an aqueous solution, such as a Tris buffer, may be preferred. In another example, if a biologically active surface comprising a carboxylate group as adsorbent (cation exchange surface) is used, then an aqueous solution, such as an acetate buffer, may be preferred.

Optionally, an energy absorbing molecule (EAM), e.g. in solution, can be applied to biomolecules or other substances bound on the biologically active surface by spraying, pipetting or dipping. Applying an EAM can be done after unbound materials are washed off of the biologically active surface. Exemplary energy absorbing molecules include, but are not limited to, cinnamic acid derivatives, sinapinic acid and dihydroxybenzoic acid.

Once the biologically active surface is free of any unbound biomolecules, adsorbent-bound biomolecules are detected using gas phase ion spectrometry. The quantity and characteristics of a biomolecule can be determined using said method. Furthermore, said biomolecules can be analyzed using a gas phase ion spectrometer such as mass spectrometers, ion mobility spectrometers, or total ion current measuring devices. Other gas phase ion spectrometers known to those skilled in the art are also included.

In one embodiment, mass spectrometry can be used to detect biomolecules of a given sample present on a biologically active surface. Such methods include, but are not limited to, matrix-assisted laser desorption ionization/time-of-flight (MALDI-TOF), surface-enhanced laser desorption ionization/time-of-flight (SELDI-TOF), liquid chromatography coupled with MS, MS-MS, or ESI-MS. Typically, biomolecules are analysed by introducing a biologically active surface containing said biomolecules, ionizing said biomolecules to generate ions that are collected and analysed.

In a preferred embodiment, the biomolecules present in a sample are detected using gas phase ion spectrometry, and more preferably, using mass spectrometry. In one embodiment, matrix-assisted laser desorption/ionization ("MALDI") mass spectrometry can be used. In MALDI, the sample is typically quasi-purified to obtain a fraction that essentially consists of a marker using separation methods such as two-dimensional gel electrophoresis or high performance liquid chromatography (HPLC).

In another embodiment, surface-enhanced laser desorption/ionization mass spectrometry ("SELDI") can be used. SELDI uses a substrate comprising adsorbents to capture biomolecules, which can then be directly desorbed and ionized from the substrate surface during mass spectrometry. Since the substrate surface in SELDI captures biomolecules, a sample need not be quasi-purified as in MALDI. However, depending on the complexity of a sample and the type of adsorbents used, it may be desirable to prepare a sample to reduce its complexity prior to SELDI analysis.

For example, biomolecules bound to a biologically active surface can be introduced into an inlet system of the mass spectrometer. The biomolecules are then ionized by an ionization source such as a laser, fast atom bombardment, or plasma. The generated ions are then collected by an ion optic assembly, and then a mass analyzer disperses the passing ions. The ions exiting the mass analyzer are detected by a detector and translated into mass-to-charge ratios. Detection of the presence of a biomolecule typically involves detection of its specific signal intensity, and reflects the quantity and character of said biomolecule.

In a preferred embodiment, a laser desorption time-of-flight mass spectrometer is used with the probe of the present invention. In laser desorption mass spectrometry, biomolecules bound to a biologically active surface are introduced into an inlet system. Biomolecules are desorbed and ionized into the gas phase by a laser. The ions generated are then collected by an ion optic assembly. These ions are accelerated through a short high voltage field and let drift into a high vacuum chamber of a time-of-flight mass analyzer. At the far end of the high vacuum chamber, the accelerated ions strike a sensitive detector surface at a different time. Since the time-of-flight is a function of the mass of the ions, the elapsed time between ionization and impact can be used to identify the presence or absence of molecules of a specific mass.

The detection of biomolecules described herein can be enhanced using certain selectivity conditions (e. g., types of adsorbents used or washing solutions). In a preferred embodiment, the same or substantially the same selectivity conditions that were used to discover the biomolecules can be used in the methods for detecting a biomolecule in a sample.

Combinations of the laser desorption time-of-flight mass spectrometer with other components described herein, in the assembly of mass spectrometer that employs various means of desorption, acceleration, detection, measurement of time, etc., are known to those skilled in the art.

Data generated by desorption and detection of markers can be analyzed with the use of a programmable digital computer. The computer program generally contains a readable medium that stores codes. Certain codes can be devoted to memory that include the location of each feature on a biologically active surface, the identity of the adsorbent at that feature and the elution conditions used to wash the adsorbent. Using this information, the program can then identify the set of features on the biologically active surface defining certain selectivity characteristics (e. g. types of adsorbent and eluents used). The computer also contains codes that receive as data (input) on the strength of the signal at various molecular masses received from a particular addressable location on the biologically active surface. This data can indicate the number of biomolecules detected, as well as the strength of the signal and the determined molecular mass for each biomolecule detected.

Data analysis can include the steps of determining signal strength (e. g., height of peaks) of a biomolecule detected and removing "outliers" (data deviating from a predetermined statistical distribution). For example, the observed peaks can be normalized, a process whereby the height of each peak relative to some reference is calculated. For example, a reference can be background noise generated by instrument and chemicals (e. g., energy absorbing molecule), which is set as zero in the scale. Then the signal strength detected for each biomolecule can be displayed in the form of relative intensities in the scale desired (e. g., 100). Alternatively, a standard may be admitted with the sample so that a peak from the standard can be used as a reference to calculate relative intensities of the signals observed for each biomolecule or other biomolecules detected.

The computer can transform the resulting data into various formats for displaying. In one format, referred to as "spectrum view", a standard spectral view can be displayed, wherein the view depicts the quantity of a biomolecule reaching the detector at each particular molecular mass. In another format, referred to as "scatter plot" only the peak height and mass information are retained from the spectrum view, yielding a cleaner image and enabling biomolecules with nearly identical molecular mass to be more visible.

Using any of the above display formats, it can be readily determined from the signal display whether a biomolecule having a particular molecular mass is detected from a sample. Preferred biomolecules of the invention are biomolecules with an apparent molecular mass of about 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ± 70 Da, 15959 Da ± 80 Da, 16164 Da ± 81 Da, 17279 Da ± 86 Da, 17406 Da ± 87 Da, 17630 Da ± 88 Da, or 18133 Da ± 91 Da. Moreover, from the strength of signal, the amount of a biomolecule bound on the biologically active surface can be determined.

### g) Identification of proteins

In case the biomolecules of the invention are proteins, the present invention comprises a method for the identification of these proteins, especially by obtaining their amino acid sequence. This method comprises the purification of said proteins from the complex biological sample (blood, blood serum, plasma, nipple aspirate, urine, semen, seminal fluid, seminal plasma, prostatic fluid, tears, saliva, sweat, ascites, cerebrospinal fluid, milk, lymph, or tissue extract samples) by fractionating said sample using techniques known by the one of ordinary skill in the art, most preferably protein chromatography (FPLC, HPLC).

The biomolecules of the invention include those proteins with a molecular mass selected from 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ± 70 Da, 15959 Da ± 80 Da, 16164 Da ± 81 Da, 17279 Da ± 86 Da, 17406 Da ± 87 Da, 17630 Da ± 88 Da, or 18133 Da ± 91 Da.

Furthermore, the method comprises the analysis of the fractions for the presence and purity of said proteins by the method which was used to identify them as differentially expressed biomolecules, for example two-dimensional gel electrophoresis or SELDI mass spectrometry, but most preferably SELDI mass spectrometry. The method also comprises an analysis of the purified proteins aiming towards the revealing of their amino acid sequence. This analysis may be performed using techniques in mass spectroscopy known to those skilled in the art.

In one embodiment, this analysis may be performed using peptide mass fingerprinting, revealing information about the specific peptide mass profile after proteolytic digestion of the investigated protein.

In another embodiment, this analysis may be preferably performed using post-source-decay (PSD), or MSMS, but most preferably MSMS, revealing mass information about all possible fragments of the investigated protein or proteolytic peptides thereof leading to the amino acid sequence of the investigated protein of proteolytic peptide thereof.

The information revealed by the aforementioned techniques can be used to feed world-wide-web search engines, such as MS Fit (Protein Prospector, http://prospector.ucsf.edu) for information obtained from peptide mass fingerprinting, or MS Tag (Protein Prospector, http://prospector.ucsf.edu) for information obtained from PSD, or mascot (www.matrixscience.com) for information obtained from MSMS and peptide mass fingerprinting, for the alignment of the obtained results with data available in public protein sequence databases, such as SwissProt (http://us.expasy.org/sprot/), NCBI (http://www.ncbi.nlm.nih.govBLAST/), EMBL (http://srs.embl-heidelberg.de:8000/srs5/) which leads to a confident information about the identity of said proteins.

This information may comprise, if available, the complete amino acid sequence, the calculated molecular mass, the structure, the enzymatic activity, the physiological function, and gene expression of the investigated proteins.

### h) Kits

In yet another aspect, the invention provides kits using the methods of the invention as described in the section Diagnostic for the differential diagnosis of epithelial cancers or an acute and chronic inflammation of the epithelium, wherein the kits are used to detect the biomolecules of the present invention.

The biomolecules of the invention include those proteins with a molecular mass selected from 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ± 70 Da, 15959 Da ± 80 Da, 16164 Da ± 81 Da, 17279 Da ± 86 Da, 17406 Da ± 87 Da, 17630 Da ± 88 Da, or 18133 Da ± 91 Da.

For example, the kits can be used to detect one or more of differentially present biomolecules as described above in a test sample of subject. The kits of the invention have many applications. For example, the kits can be used to differentiate if a subject is healthy, having a precancerous lesion, an epithelial cancer, a metastasized epithelial cancer or an acute and chronic inflammation of the epithelium. Thus aiding the diagnosis of epithelial cancers or diseases of epithelial origin. In another example, the kits can be used to identify compounds that modulate expression of said biomolecules.

In one embodiment, a kit comprises an adsorbent on a biologically active surface, wherein the adsorbent is suitable for binding one or more biomolecules of the invention, a denaturation solution for the pre-treatment of a sample, a binding solution, a washing solution or instructions for making a denaturation solution, binding solution, or washing solution, wherein the combination allows for the detection of a biomolecule using gas phase ion spectrometry. Such kits can be prepared from the materials described in other previously detailed sections (e. g., denaturation buffer, binding buffer, adsorbents, washing solutions, etc.).

In some embodiments, the kit may comprise a first substrate comprising an adsorbent thereon (e. g., a particle functionalized with an adsorbent) and a second substrate onto which the first substrate can be positioned to form a probe, which is removably insertable into a gas phase ion spectrometer. In other embodiments, the kit may comprise a single substrate, which is in the form of a removably insertable probe with adsorbents on the substrate.

In another embodiment, a kit comprises a binding molecule that specifically binds to a biomolecule related to the invention, a detection reagent, appropriate solutions and instructions on how to use the kit. Such kits can be prepared from the materials described above, and other materials known to those skilled in the art. A binding molecule used within such a kit may include, but is not limited to, proteins, peptides, nucleotides, nucleic acids, hormones, amino acids, sugars, fatty acids, steroids, polynucleotides, carbohydrates, lipids, or a combination thereof (e.g. glycoproteins, ribonucleoproteins, lipoproteins), compounds or synthetic molecules. Preferably, a binding molecule used in said kit is an antibody.

In either embodiment, the kit may optionally further comprise a standard or control information so that the test sample can be compared with the control information standard to determine if the test amount of a marker detected in a sample is a diagnostic amount consistent with a diagnosis of prostate cancer.

The present invention is further illustrated by the following examples, which should not be construed as limiting in any way. The contents of all cited references (including literature references, issued patents, published patent applications), as cited throughout this application, are hereby expressly incorporated by reference. The practice of the present invention will employ, unless otherwise indicated, conventional techniques of cell biology, cell culture, molecular biology, transgenic biology, microbiology, recombinant DNA, and immunology, which are known to those skilled in the art. Such techniques are explained fully in the literature.

### Examples

### Example 1. Sample collection for gastric cancer evaluation in a subject (Set 1).

Serum samples were obtained from a total of 148 individuals, which included two different groups of subjects. In the first group (group I), sera were drawn from 88 gastric cancer patients, undergoing diagnosis and treatment of gastric cancer at the Departments of Gastroenterology and Surgery of the Universities of Magdeburg and Cottbus. After endoscopy and histological confirmation of gastric cancer, serum samples were collected from the patients before any further treatment. In all cases the diagnosis was confirmed by histological evaluation prior to treatment. Follow-up data for all gastric cancer patients are currently collected and will be available for later studies.

The non-cancer control group consisted of 60 subjects (39 female, 21 male) with dyspeptic symptoms, which were recruited from both primary care physicians and the outpatient clinic of the Department of Gastroenterology. Serum from each subject was taken following gastrointestinal endoscopy, wherein the absence of gastric cancer was confirmed. Furthermore, all subjects denied a personal history of cancer and were otherwise healthy. A follow-up on these patients was available for a maximum of five years, in which none of the patients developed gastric or colon cancer. The average age of the subjects was 57 years (range 40-70 years).

### Example 2. Sample collection for colon cancer evaluation in a subject (Set 2).

Serum samples were obtained from a total of 134 individuals, which included two different groups of subjects. In the first group (group I), sera were drawn from 57 colon cancer patients, undergoing diagnosis and treatment of colon cancer at the Departments of Gastroenterology and Surgery of the Universities of Magdeburg and Cottbus (both Germany). After endoscopy, serum samples were collected from the patients before any further treatment. In all cases the diagnosis was confirmed by histological evaluation prior to treatment. Follow-up data for all colon cancer patients are currently collected and will be available for later studies.

The non-cancer control group consisted of 77 healthy blood donors. Blood donors are considered to be healthy individuals not suffering from severe diseases.

### Example 3. ProteinChip Array analysis.

ProteinChip Arrays of the SAX2-type (strong anion exchanger) were arranged into a bioprocessor (Ciphergen Biosystems, Inc.), a device that contains up to 12 ProteinChips and facilitates processing of the ProteinChips.

The ProteinChips were pre-incubated in the bioprocessor with 200 µl binding buffer (0.1 M Tris-HCl, 0.02% Triton X-100, pH 8.5). 10 µl of serum sample was diluted 1:5 in a buffer (7 M urea, 2 M thiourea, 4% CHAPS, 1% DTT, 2% ampholine) and again diluted 1:10 in the binding buffer. Then, 300 µl of this mixture (equivalent to 6 µl original serum sample) were directly applied onto the spots of the SAX2 ProteinChips. In between dilution steps and prior to the application to the spots, the sample was kept on ice (at 0°C). After incubation for 120 minutes at 20 to 24 °C the chips were incubated with 200 µl binding buffer, before 2 x 0.5 µl EAM solution (20 mg/ml sinapinic acid in 50% acetonitrile and 0.5% trifluoroacetic acid) was applied to the spots.

After air-drying for 10 min, the ProteinChips were placed in the ProteinChip Reader (ProteinChip Biology System II, Ciphergen Biosystems, Inc.) and time-of-flight spectra were generated by laser shots collected in the positive mode at laser intensity 215, with the detector sensitivity of 8. Sixty laser shots per average spectra were performed.

Calibration of mass accuracy was performed by using the following mixture of mass standard calibrant proteins: Dynorphin A (porcine, 209 - 225, 2147.50 Da), Beta-endorphin (human, 61 - 91, 3465.00 Da), Insulin (bovine, 5733.58 Da), and Cytochrome c (bovine, 12230.90 Da) at a concentration of 1.21 pmol/µl, and Myoglobin (equine cardiac, 16951.50 Da) at a concentration of 5.16 pmol/µl.

Zero point five µl of this mixture were applied to a single spot of a H4 ProteinChip array. After air-drying of the drop, 2 x 1 µl matrix solution (a saturated solution of sinapinic acid in 50% acetonitrile 0.5% trifluoracetic acid) was applied to the spot. The drop was allowed to air-dry for 10 min after each application of matrix solution.

The ProteinChip was placed in the ProteinChip Reader (Biology System II, Ciphergen Biosystems, Inc.) and time-of-flight spectra were generated by laser shots collected in the positive mode at laser intensity 210, with the detector sensitivity of 8. Sixty laser shots per average spectra were performed. Subsequently, Time-Of-Flight values were correlated to the molecular masses of the standard proteins, and calibration was performed according to the instrument manual.

### Example 4. Peak detection and data analysis.

The analysis of the data was performed by automatic peak detection and alignment using the operating software of the ProteinChip Biology System II, the ProteinChip Software Version 3.01 (Ciphergen Biosystems, Inc.). Figures 1A and 1B show a comparison of protein mass spectra detected using the above mentioned SAX2 ProteinChip arrays for samples isolated from both gastric and colon cancer, respectively, as compared to samples taken from healthy patients.

Each complete set of patients (Set 1 for gastric cancer and set 2 for colon cancer, see examples 1 and 2) was divided into a training set and a test set. The train set for Set 1 comprised of 70 patients with gastric cancer and 48 patients without gastric cancer. The test set for Set 1 comprised of 18 randomly selected patients with gastric cancer and 12 randomly selected patients without gastric cancer. The train set for Set 2 comprised of 46 patients with colon cancer and 62 patients without colon cancer (blood donors). The test set for Set 2 comprised of 11 randomly selected patients with colon cancer and 15 randomly selected patients without colon cancer (blood donors). Additionally, test sets for the train sets of sets 1 and 2 were compiled comprising of the at each case other set in complete (see details below). This was done in order to test the classification algorithm generated on the basis of the spectra of the subgroup of patients selected for each training set with the corresponding, blinded, test set and the test set of the, at each case, other complete set (see below).

The m/z values of all mass spectra selected for the analysis ranged between 1500 Da and 30000 Da, wherein smaller masses were not used since artefacts with the "Energy Absorbing Molecule, EAM" ("Matrix") could not be excluded, and higher masses were not detected under the chosen experimental conditions. The spectra within the train sets were normalised according to the intensity of the total ion current, followed by baseline subtraction, and automatic peak detection as previously described by Adam et al. (ref.), using the "Biomarker Wizard" tool of the ProteinChip Software Version 3.0 (Ciphergen Biosystem, Inc.). The following settings were chosen for peak detection by "Biomarker Wizard": a) auto-detect peaks to cluster, b) first pass: 3 signal/noise, c) minimum peak threshold: 25% of all spectra, d) deletion of user-detected peaks below threshold, e) cluster mass window: +/- 0.5% of mass. Using these settings, 66 signal clusters were identified for the train set of set 1 and 67 signal clusters were identified for the train set of set 2.

The normalization coefficient generated by normalizing the spectra of the train sets and the cluster information of the train sets generated by the "Biomarker Wizard" tool of the software were saved and used to externally normalize the spectra of the corresponding test sets and to cluster the signals of the corresponding test sets according to the normalization and peak identification of the train sets.

The cluster information for each train and test set (containing sample ID and sample group, cluster mass values and cluster signal intensities for each spectrum within the sets) was transformed into an interchangeable data format (a .csv table) using the "Sample group statistics" function of the "Biomarker Wizard" tool of the ProteinChip Software Version 3.0. In this format, the data can be analysed by a specific software for the generation of regression and classification trees (see examples 5 to 7).

### Example 5. Construction of classifiers.

Five classifiers with binary target variable (cancer versus non-cancer) were constructed: First, as a proof of principle, two classifiers were constructed only on the basis of the two training sets described above. Second, two final classifiers were constructed on the basis of all available gastric or colon cancer data, fusing the corresponding training and test data sets. Third, a 2^{nd} final colon classifier was constructed analogously to the first final colon cancer classifier but excluding the most informative and dominating mass of the first final colon classifier.

Forward variable selection was applied in order to determine highly informative sets of variables ("patterns") for classification. The results of the present invention were generated using the "CART" decision tree approach (classification and regression trees; Breiman et al., 1984). Moreover, bagging of classifiers was applied to overcome typical instabilities of forward variable selection procedures, thereby increasing overall classifier performance (Breiman, 1994).

More precisely, for each training set 50 bootstrap samples were generated (sampling with replacement, maximal 3 sample redraws). For each bootstrap sample a number of classifiers of different complexity using 1, 2, ..., *N* variables were generated (*N* corresponds to classifier complexity allowing vanishing error on the respective training data) and evaluated (by resembling bootstrap samples, or also by cross validation; averaging performance of classifiers of same complexity). Then, classifier complexity was chosen according to minimal re-sampling (or cross validation) error and a classifier of this complexity was generated on the respective bootstrap sample. The such obtained 50 single classifiers, one for each bootstrap sample, were combined to constitute an ensemble of classifiers predicting class membership by plurality vote.

The procedure of classifier construction was conducted five times to obtain two classifiers, a proof-of-principle classifier and a final classifier, for gastric cancer and three classifiers, a proof-of-principle classifier and two final classifiers, for colon cancer.

### Example 6. Classifier structure.

For gastric cancer, the proof-of-principle classifier employed 23 masses out of 66 determined signal clusters. Single decision trees consisted of up to 5 variables (6 end nodes), 3 to 4 variables being typical, see histogram of Figure 5a. Variable importance was roughly deduced by the frequency with which variables appear in the decision tree ensemble (starting with most frequent variables, frequency in brackets): 3946 Da (44), 7001 Da (20), 5491 Da (17), 10665 Da (14), 18133 Da (11), 4477 Da (9), 6449 Da (8), 12471 Da (8), 7969 Da (7), 4154 Da (6), 4104 Da (5), 15959 Da (3), 1516 Da (2), 17905 Da (2), 8711 Da (2), 18380 Da (1), 6876 Da (1), 13989 Da (1), 5113 Da (1), 9210 Da (1), 4298 Da (1), 4867 Da (1), 5650 Da (1), see Figure 6a for the distribution of masses in the gastric cancer classifier ensemble.

The final classifier for gastric cancer employed 28 masses out of 66 determined signal clusters. Single decision trees consisted of up to 6 variables (7 end nodes), 3 to 5 variables being typical, see histogram of Figure 5b. Variable importance was roughly deduced by the frequency with which variables appear in the decision tree ensemble (starting with most frequent variables, frequency in brackets): 3947 Da (48), 5492 Da (20), 5650 Da (13), 8711 Da (12), 1516 Da (11), 10665 Da (11), 18133 Da (10), 6450 Da (8), 13996 Da (7), 7971 Da (7), 4867 Da (7), 15960 Da (5), 4104 Da (5), 4477 Da (5), 4154 Da (3), 4298 Da (3), 8232 Da (3), 2022 Da (3), 12471 Da (3), 16164 Da (1), 22473 Da (1), 17630 Da (1), 4360 Da (1), 17279 Da (1), 2050 Da (1), 6881 Da (1), 17406 Da (1), 7006 Da (1), figures 3A-AC shows the scatter plot clusters for each deduced variable within the decision tree ensemble for gastric cancer. See Figure 6b for the distribution of masses in the final gastric cancer classifier ensemble.

For colon cancer, the proof-of-principle classifier employed 6 masses out of 67 determined signal clusters. Single decision trees consisted of up to 2 variables (3 end nodes), 1 and 2 variables are typical, see histogram of Figure 5c. Variable importance was roughly deduced by the frequency with which variables appear in the decision tree ensemble (starting with most frequent variables, frequency in brackets): 3947 Da (47), 1509 Da (11), 5653 Da (5), 4958 (3), 1535 Da (2), 2020 (1), figures 2A-F shows the scatter plot clusters for each of the deduced variables within the decision tree ensemble for colon cancer. See Figure 6c for the distribution of masses in the colon cancer classifier ensemble.

The first final classifier for colon cancer employed 6 masses out of 67 determined signal clusters. Single decision trees consisted of up to 3 variables (4 end nodes), 1 and 2 variables are typical, see histogram of Figure 5d. Variable importance was roughly deduced by the frequency with which variables appear in the decision tree ensemble (starting with most frequent variables, frequency in brackets): 3947 Da (47), 1509 Da (17), 5653 Da (7), 4958 Da (5), 1535 Da (2), 2020 Da (1), figures 2A-F shows the scatter plot clusters for each of the deduced variables within this decision tree. See Figure 6d for the distribution of masses in the colon cancer classifier ensemble.

The second final classifier for colon cancer employed 12 masses out of 67 determined signal clusters. Single decision trees consisted of up to 3 variables (4 end nodes), 2 and 3 variables are typical. Variable importance was roughly deduced by the frequency with which variables appear in the decision tree ensemble (starting with most frequent variables, frequency in brackets): 4958 Da (40), 5653 Da (29), 4158 Da (23), 1509 Da (12), 2020 Da (4), 12504 Da (4), 1535 Da (2), 12669 Da (2), 13808 Da (1), 7977 Da (1), 5114 Da (1), 5474 Da (1).

The final classifiers for gastric as well as colon cancer consist of more complex decision trees in accordance with the larger data set for classifier construction, compare Figure 5a with 5b and Figure 5c with 5d. For the final gastric classifier, the set of masses is increased from 23 masses (proof-of-principle classifier) to 28 masses. The final gastric classifier comprises additionally the masses 2022 Da (3), 2050 Da (1), 4360 Da (3), 8232 Da (3), 16164 Da (1), 17279 Da (1), 17406 Da (1), 17630 Da (1), 22473 Da (1) while not using the masses 5113 Da (1), 92 10 Da (1), 17905 Da (1), 183 80 Da (1) of the proof-of-principle gastric classifier, respective frequencies are given in brackets. The first final colon classifier consists of the same masses as the proof-of-principle colon classifier. The second final colon cancer classifier comprises additionally the masses 4158 Da (23), 5114 Da (1), 5474 Da (1), 7977 Da (1), 12504 Da (4), 12669 Da (2), 13808 Da (1) while not using the one explicitly removed mass 3947 Da (47) of the proof-of-principle gastric classifier, respective frequencies are given in brackets.

The classifiers do not contain all differentially expressed bio-molecules / proteins found in this study. Those not included in the classifier may gain high significance in classifier construction when larger sample sets are examined. Candidates for such masses are competitors and surrogates on the first level of variable selection.

Competitors of mass 3947 Da for the final gastric classifier are (ordered according to importance) 12471 Da, 5492 Da, 5650 Da, 4154 Da, 4968 Da, of which only the mass 4968 Da is not yet included in our current classifiers. Surrogates of mass 3947 Da for the final gastric classifier are 5492 Da, 4154 Da, 12471 Da, 4968 Da, 5650 Da, of which again only the mass 4968 Da is not yet included in our current gastric classifiers.

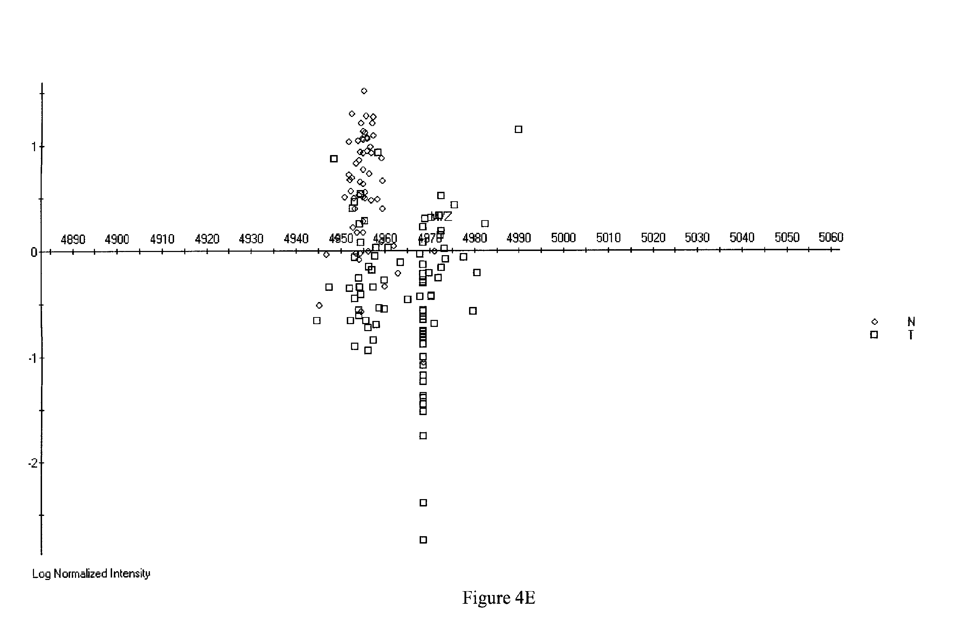

Competitors of mass 3947 Da for the first final colon classifier are 4958 Da, 5653 Da, 12504 Da, 12669 Da, 1535 Da, of which only the masses 12504 Da, 12669 Da are not yet included in our current classifiers. Surrogates of mass 3947 Da for the first final colon classifier are (ordered according to importance) 4958 Da, 5653 Da, 12504 Da, 5474 Da, 12669 Da, of which only the masses 12504 Da, 5474 Da, 12669 Da are not yet included in our current classifiers. See Figure 4 for all differentially expressed biomolecules not included in the current classifiers.

### Example 7. Classification performance.

Classification performance is determined for the two proof-of-principle classifiers.
The gastric cancer classifier was evaluated on 3 test sets: 1. a gastric cancer test set consisting of 18 gastric cancer and 12 non-gastric cancer patients, 2. a colon cancer test set consisting of all 57 colon cancer and 77 remaining non-cancer patients, and 3. a combined gastric-colon cancer test set combining test set 1 and 2 consisting of 18 gastric cancer, 57 colon cancer, and 89 non-cancer patients. Classifier performance was as follows:

| | gastric cancer | colon cancer | combined |
|---|---|---|---|
| sensitivity | 94,4 % | 100 % | 98,7 % |
| specificity | 91,7 % | 83,1 % | 84,3 % |
| positive predictive value | 94,4 % | 81,4 % | 84,1 % |
| negative predictive value | 91,7 % | 100 % | 98,7 % |
| misclassifications | 6,7 % | 9,7 % | 9,1 % |

The colon cancer classifier was also evaluated on 3 test sets: 1. a colon cancer test set consisting of all 11 colon cancer and 15 non-cancer patients, 2. a gastric cancer test set consisting of 88 gastric cancer and 60 non-gastric cancer patients, and 3. a combined gastric-colon cancer test set combining test set 1 and 2 consisting of 11 colon cancer, 88 gastric cancer, and 75 non-cancer patients. Classifier performance was as follows:

| | colon cancer | gastric cancer | combined |
|---|---|---|---|
| sensitivity | 100 % | 80,7 % | 82,8 % |
| specificity | 100 % | 100 % | 100 % |
| positive predictive value | 100 % | 100 % | 100 % |
| negative predictive value | 100 % | 77,9 % | 81,5 % |
| misclassifications | 0 % | 11,5 % | 9,8 % |

### Example 8. Text from a corresponding publication (not yet submitted)

Despite its decreasing incidence gastric cancer remains the second most common cause of cancer-related deaths in certain parts of the world, wherein more than 1 million individuals die from this disease every year. This poor prognosis is based on poor therapeutic options and the late diagnosis of the disease in advanced stages. The identification of gastric cancer in its early stages and the screening of individuals with an increased risk of developing gastric cancer would improve this prognosis dramatically. Unfortunately to date, no valid serum markers for gastric cancer have been identified. Using serum samples from 110 patients (50 patients with histologically confirmed gastric cancer at different clinical stages and from 60 non-cancer individuals undergoing upper GI endoscopy for dyspepsia), we screened for protein patterns to differentiate gastric cancer from non-cancer individuals by surface enhanced laser desorption ionization (SELDI™) mass spectrometry using ProteinChip™ technology coupled with a pattern-matching algorithm. In total 71 clusters were identified, from which a panel of 26 was selected to generate an ensemble of 50 classifiers which separated cancerous samples from non-cancerous samples. This classifier was able to correctly classify all gastric cancers and all non-cancerous individuals. A blind test set comprising of 9 stage I cancers and 11 randomly selected sera from non-cancerous controls was used to determine the sensitivity and specificity of these markers. Interestingly, 8 out of 9 cancers and all 11 non-cancerous samples were correctly classified, thus the sensitivity and specificity of the classifier was 88.9% and 100%, respectively. In addition, a further independent test set of 29 serum samples taken from gastric cancer patients treated in 2 different hospitals was correctly classified as cancer in all cases. Finally, we applied the classifier to a set of 30 presumably healthy blood donors of which 29 were classified as non-cancerous. Serum protein fingerprinting by SELDI mass spectrometry allows for the separation of serum from gastric cancer patients from non-cancerous individuals and may identify early gastric cancers, indicating that proteome analysis in conjunction with bioinformatics may facilitate the identification of biomarkers that could be used for the early detection of cancer, which would improve the overall poor prognosis of human cancers.

Using the ProteinChip™ Software, we first analysed the peaks in the mass range of 1200 to 30000 Da of 111 serum samples taken from patients of group I who were either diagnosed with gastric cancer or who presented with dyspeptic symptoms for exclusion of gastric cancer (Figure 7). Representative protein spectra of two patients with and two patients without cancer are presented in figure 8. In order to assess reproducibility of the SELDI spectra, we determined the mass location and signal intensity of each sample on a single chip (intra-assay) and between chips (inter-assay) using all 111 pooled spectra. From these spectra we chose three peaks in the range of 1200 to 30000 Da, i.e. 2020, 8483, 13778 Da in order to determine standard deviation and the coefficient of variance (Figure 9). Analysis of the inter-assay reproducibility revealed the following mean mass, SD and coefficient of variance: protein 1: 2020.5 ± 0.89 Da (0.044%), protein 2: 8483.5 ± 5.81 Da (0.068%), protein 3: 13779.6 ± 5.59 Da (0.04%). The intra-assay reproducibility was assessed by the following proteins: protein 1: 2020.7 ± 0.33 Da (0.016%), protein 2: 8479.5 ± 2.56 Da (0.03%) and protein 3: 13779.6 ± 5.28 Da (0.038%). For normalized intensity (peak height or relative concentration) the intra-assay coefficients of variance were 9.93% (2020 Da), 17.7% (8483 Da) and 12.5% (13779 Da), while the inter-assay coefficients of variance were 15.1% (2020 Da), 21% (8483 Da) and 21.1% (13779 Da), respectively.

Although eighty peaks were identified using the 'Biomarker Wizard' tool of the ProteinChip™ Software Version 3.01., none of the peaks were able to distinguish all cancer patients from non-cancer individuals. The sensitivity of identifying cancer patients ranged from 39 to 95.1%, whereas the specificity of the various markers ranged from 47.8 to 100%. Among these 13 markers, four markers with the apparent molecular weights of 12470, 3946, 5649 and 3503, exhibited a sensitivity and specificity above 80%. While some markers showed a sensitivity up to 95.1%, this was usually accompanied by a sharp decline in specificity and vice versa (Table 3). Since none of the markers alone were able to separate all cancers from non-cancer serum samples, a bioinformatical approach using the Biomarker Pattern Software™ (BPS) was employed. Thus, these 80 peaks were then tested in a training set which comprised of 46 patients without gastric cancer (referred to as 'normal') and of 41 patients with gastric cancer (referred to as 'cancer'). The Biomarker Pattern Software™ identified three masses, i.e. 12470, 2610 and 11537 Da to generate four terminal nodes, which correctly separated the cancers in 40 of 41 cases (sensitivity 97.5%) and 45 of 46 normal (specificity 97.8%) (Figure 7, 10). The positive and negative predictive values were 97.6% and 97.8%, respectively. The three markers, alone, demonstrated poor sensitivities and specificities (Table 1), however, taken together, their ability to distinguish cancer from non-cancerous samples rose above 97% and was far better than any other single marker. Interestingly, a combination of the four best markers, which all exhibited sensitivities and specificities above 80%, yielded a sensitivity and specificity of 97.5% and 91.3%, respectively, which was, in turn, less efficient than the three markers picked by the BPS™. Furthermore, while the single best marker 12470 demonstrated a sensitivity and specificity of 87.8 and 89.1%, respectively, every other possible combination of these three markers (12470, 2610, 11537 Da) yielded sensitivities and specificities far below the combination of all three biomarkers (Table 2). Interestingly, the various biomarkers were either absent or increased in cancer sera, indicating that these markers do not only represent tumour-derived proteins, but may also reflect changes in the protein profile secondary to the presence of cancer. Thus, the markers 12470 and 2610 were increased in normal controls and absent, or significantly lower, in cancer sera, while the marker 11537 was increased only in a small subset of gastric cancers (Figure 7).

The generated ensemble of decision trees employed 26 masses out of 71 determined signal clusters. Single decision trees consisted of up to 5 masses (6 end nodes) with 3 and 4 masses being typical, see Figure 11. Each decision tree can be read as a specific pattern of masses valuable for classifying cancers versus non-cancers. All such patterns of our classifier are summarized in Table 3. Variable importance was roughly deduced by the frequency with which variables appear in the decision tree ensemble (starting with most frequent variables, frequency in brackets): 3946 Da (35), 12471 Da (19), 1510 Da (17), 3503 Da (13), 2610 Da (12), 4198 Da (10), 3654 Da (8), 15958 Da (8), 5650 Da (6), 5492 Da (6), 6879 Da (5), 4478 Da (5), 7966 Da (5), 6647 Da (5), 2048 Da (3), 18137 Da (3), 6449 Da (3), 8791 Da (3), 11537 Da (3) 8233 Da (2), 8938 Da (2), 4103 Da (1), 12663 Da (1), 17409 Da (1), 4158 Da (1), 9435 Da (1), see Figure 12 for the distribution of masses within the ensemble on specific (a-e) and all hierarchical levels (f).

Since identifying early cancers is the best approach to improve the overall poor prognosis of gastric cancer patients, we then tested the overall classifier on 9 stage I cancers and 11 randomly selected normal, all of which were not included in the training set used for classifier generation. 8 of the 9 cancers were correctly classified as cancers, as well as all 11 normal controls. The performance of an exemplary single decision tree consisting of the three masses 3946 Da, 3503 Da, and 15958 Da is shown in Figure 10. However, 8 of the 9 gastric cancers, which were T1 and T2 cancers without local or distant metastasis, were correctly classified, thereby underscoring the efficacy of the pattern, especially for the diagnosis of early gastric cancer. In order to assure that the different spectra were indeed related to the diverse diagnosis of the cancer patients and the normal controls, we also tested our pattern against a test set of 29 gastric cancer patients from other hospitals (group II). These patients also had histologically confirmed gastric cancer, albeit the mode of serum sampling and processing was not standardized as in the train set or the first test set (group I). However, this collection of sera reflects more closely the actual clinical condition of various sera obtained at different time points from various individuals. Nonetheless our algorithm correctly classified all 29 gastric cancer samples as cancer, thus supporting the efficacy of our biomarker pattern. In addition, we also tested the decision tree algorithm with a further group of 30 healthy individuals who were blood donors and otherwise healthy. Interestingly, 26 of 30 healthy individuals (group III) were correctly classified as non-cancer individuals (Figure 10). Even more interesting, the extent of the primary tumour (pT stage), presence of lymph nodes (pN stage) or distant metastases (M stage) in cancer patients was independent of the markers mentioned above.

Classification performance is determined for 3 classifiers of different complexity: 1. the best single mass, 2. the most frequent decision tree considering 3 masses, and 3. the ensemble of 50 decision trees. The results are summarized in Table 4.

Despite all advances in diagnostic and therapeutic approaches in the clinical management of gastric cancer, the prognosis of this disease still remains dismal and less than 10% of patients with gastric cancer survive 5 years following its initial diagnosis. Thus, besides prevention, the development of new diagnostic tools is of major importance for the improvement of this clinical dilemma. Despite great efforts in the development of serum-based markers, to date, no valid serum markers for gastric cancer have been identified. Recent studies indicate that the sensitivity of the most frequently used markers, such as CEA, Ca19-9 and Ca 72-4 lies between 20.9 and 56%, which does not qualify for routine screening or diagnostic purposes. Moreover, the potential curatively treated stage I cancers are detected in less than 23% of gastric cancers (Table 5), thus the identification and development of serum markers specific for gastric cancers has been rather disappointing. Recently, new proteomic approaches, including SELDI™, have been developed. This technique is based on the detection of proteins affinity-bound to a ProteinChip™. Based on this technique several groups have analysed sera from patients with various cancers, including prostate, breast, bladder, ovary and pancreatic cancer (Table 6). Using this technique, coupled to a pattern-matching algorithm, various biomarker patterns have been identified. These markers were able to correctly classify cancer and non-cancer individuals with high sensitivity and specificity. In fact, the combination of eight biomarkers correctly classified all ovarian cancers, and specificity was 95 % in this group of patients (Petricoin 2002). Furthermore, other biomarker patterns were identified in breast, prostate and bladder cancer, which correctly classified these cancers in 87 to 95% (Vlahou, Li, Qu). Using a similar approach, we analysed a large set of well characterized gastric cancers patients, wherein all cases gastric cancer were confirmed by histology. We used this set of patients, together with a further group of individuals without gastric cancer as assessed by upper GI endoscopy and without a personal history of cancer, in order to screen for protein patterns by SELDI-TOF™ using the ProteinChip™ technology coupled to a pattern-matching algorithm. Eighty biomarkers were identified which exhibited large variations in sensitivity and specificity in distinguishing cancer from non-cancer sera. However, with the help of three selected biomarkers, our train set of cancer and non-cancer individuals was correctly classified in all but one cancer patient, and all but one non-cancer individual. This accounts for a sensitivity and specificity of 97.5% and 97.8%, respectively. Next, we tested our algorithm with a group of patients diagnosed with stage I gastric cancer (test set). We chose this group of patients since these patients could undergo a potential curative resection and therefore are the primary focus of potential serum based screening markers. Our biomarker pattern correctly identified 8 of these 9 patients, whereas a group of 15 non-cancer individuals was correctly classified in 12 cases. None of these cases was used for the generation of the decision tree. We then challenged our biomarker pattern with a further independent set of patients, i.e. 29 patients with gastric cancer. Again, histological verification of the cancers was obtained in all cases. These test sets are of considerable interest since these sera were collected in other hospitals under non-standardized conditions, and, therefore, truly reflect the clinical situation in the management of cancer patients. Again our decision tree algorithm correctly classified all gastric cancer patients.

Our study demonstrates that changes of serum protein profiles in cancer patients may not only result from proteins produced by a given tumour, but may also result from a secondary reaction e.g. of the immune system against the tumour or tumour-specific proteins. This hypothesis is supported by the finding that two of the biomarkers identified by the Biomarker Pattern Software™, were not present, or increased, in cancer patients (i.e. 2610 and 12470 Da), but rather in individuals without cancer. The loss of certain proteins in cancer patients, as detected by SELDI™ analysis, would explain why the extent of the primary tumour (pT), the presence of lymph node metastasis (pN) and/or distant metastasis (pM) was independent of the biomarkers and/or the biomarker pattern, a fmding that has already been reported by other groups (Li). The loss or reduced expression of these serum proteins is most likely a secondary effect to cancer, whereby the loss of expression reflects the presence of cancer and, thus, cannot predict the extent of cancers. This would also indicate that this secondary loss of serum proteins may be a very early event in the pathogenesis of cancers, which is supported by our findings in 9 patients with stage I gastric cancer, wherein 8 were correctly classified as cancer patients. Thus, those proteins which are present in non-cancer individuals and absent, or reduced, in cancer patients are most likely not tumour-derived or tumour-specific, but rather present proteins that are either lost or down-regulated in cancer patients as a secondary reaction to the presence of cancer. The results from our study may indicate a novel concept of identifying serum markers in human cancers. Since the search and identification of tumour-specific serum markers for gastric cancer has not led to the development of efficient serum markers in the last decades, the loss of serum proteins, which we term **negative serum protein profiling**, as a secondary reaction against the tumour as identified in our analysis may be a universal hallmark of various, if not all, cancers. A novel approach towards the identification and development of serum markers should, therefore, focus on serum proteins that are lost in sera of patients with cancer since these changes may be present in multiple cancers. Furthermore, such changes may even lead to the serum-based identification of premalignant conditions and screening of high-risk individuals which would not only dramatically improve the prognosis of cancer patients, but may also prevent these malignant diseases.

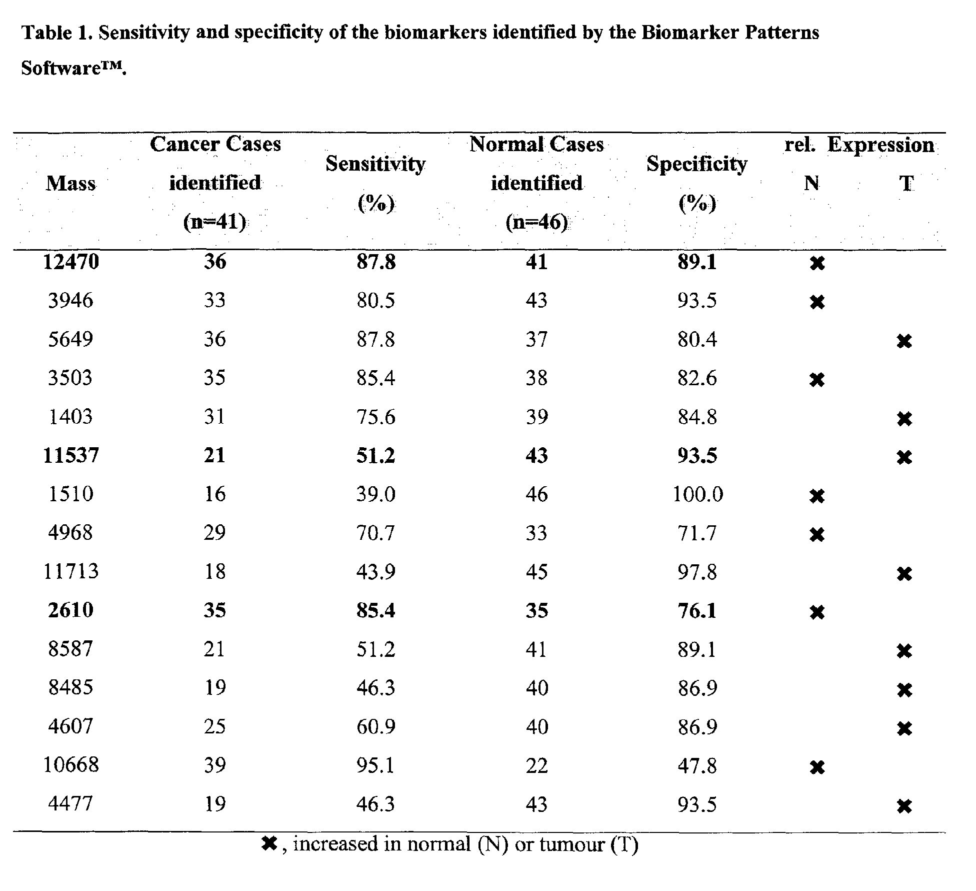

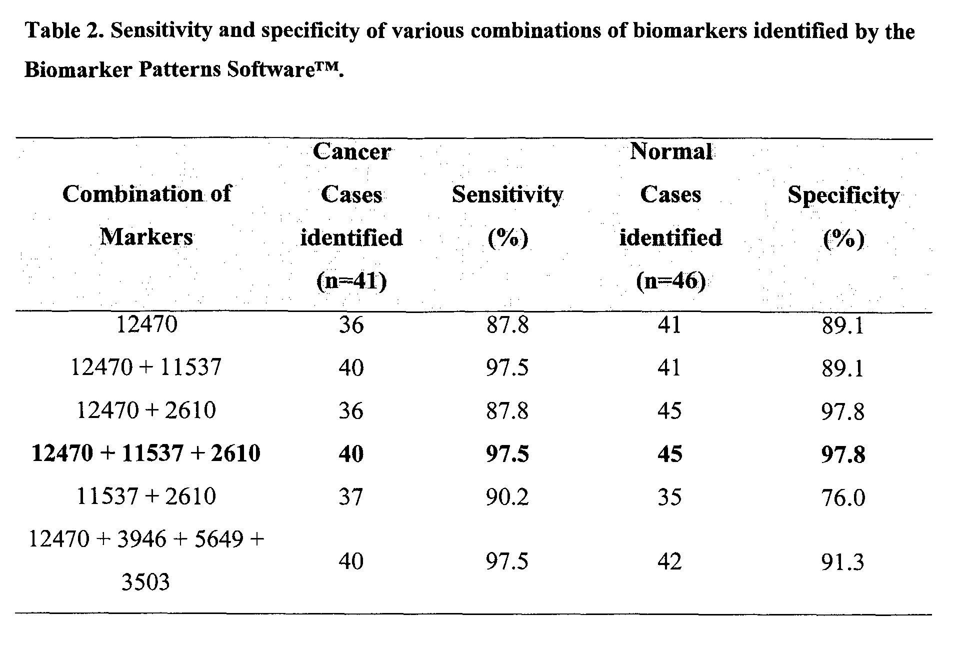

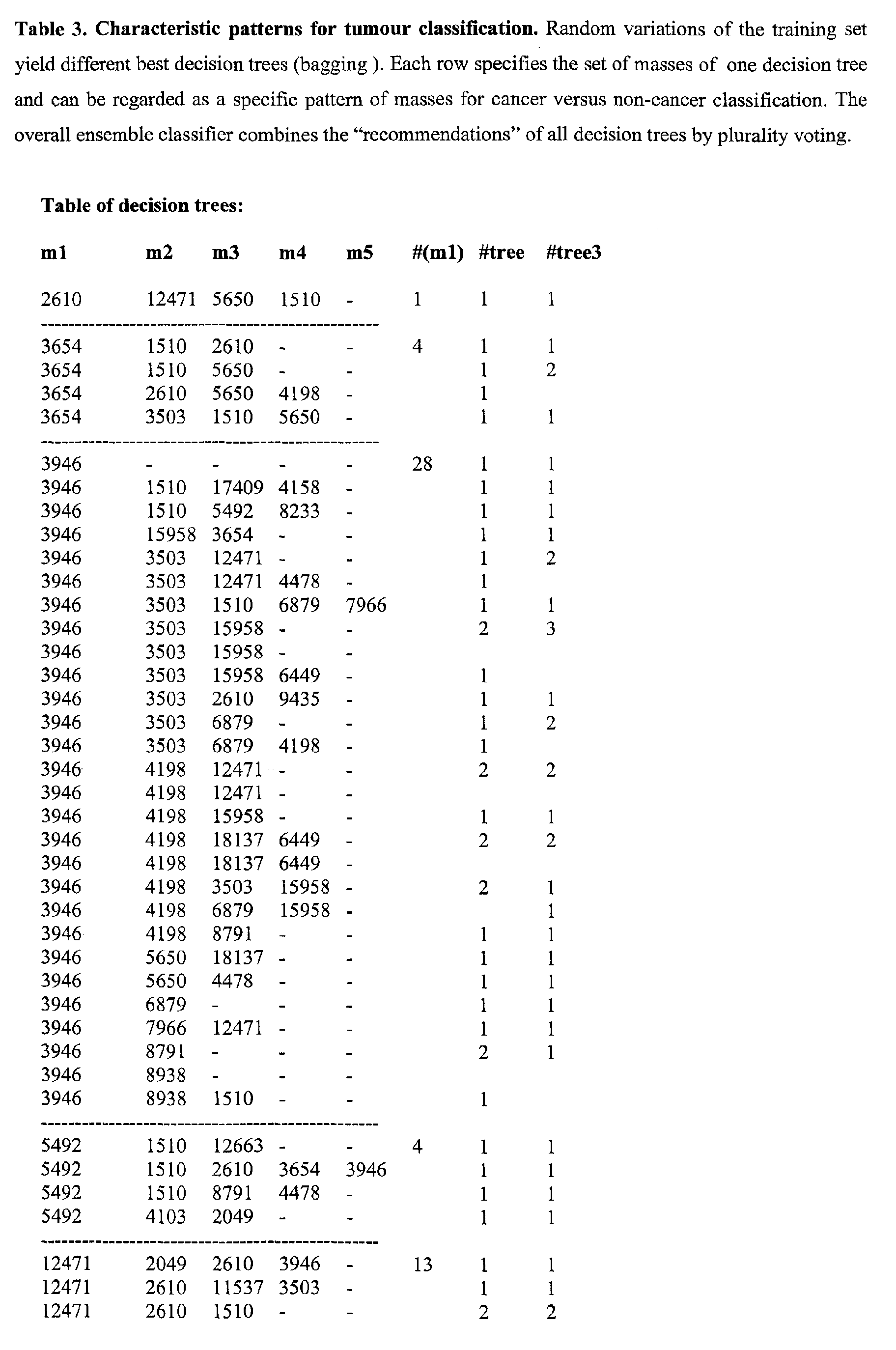

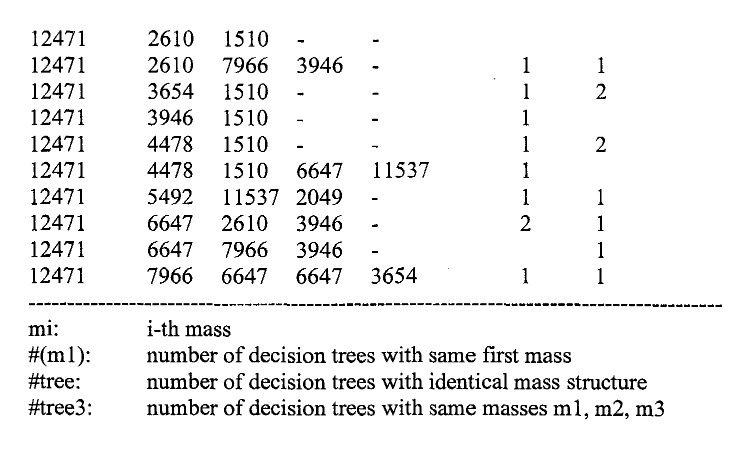

**Table 4.**

| **Summary of Classification Performance** | | | | | | | |
|---|---|---|---|---|---|---|---|
| classifier | characterization | training data | | test data: group 1 | | test data: gastric cancers | test data: blood donors |
| | | sens | spec | sens | spec | sens | spec |
| best single mass | mass 3946 | 85.4 | 91.8 | 89.9 | 100 | 100 | 90 |
| best single tree | masses 3946, 3508, 15958 | 92.7 | 94.1 | 89.9 | 90.9 | 93.1 | 86.7 |
| ensemble | 50 trees | 100 | 100 | 89.9 | 100 | 88.9 | 96.7 |
| sens : sensitivity, spec: specificity | | | | | | | |

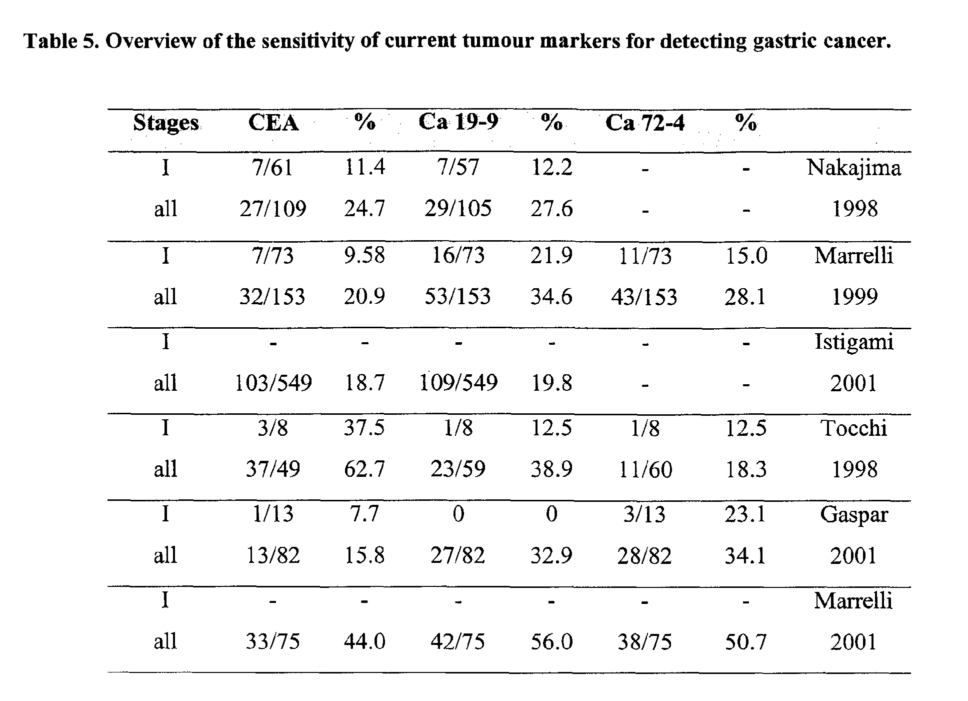

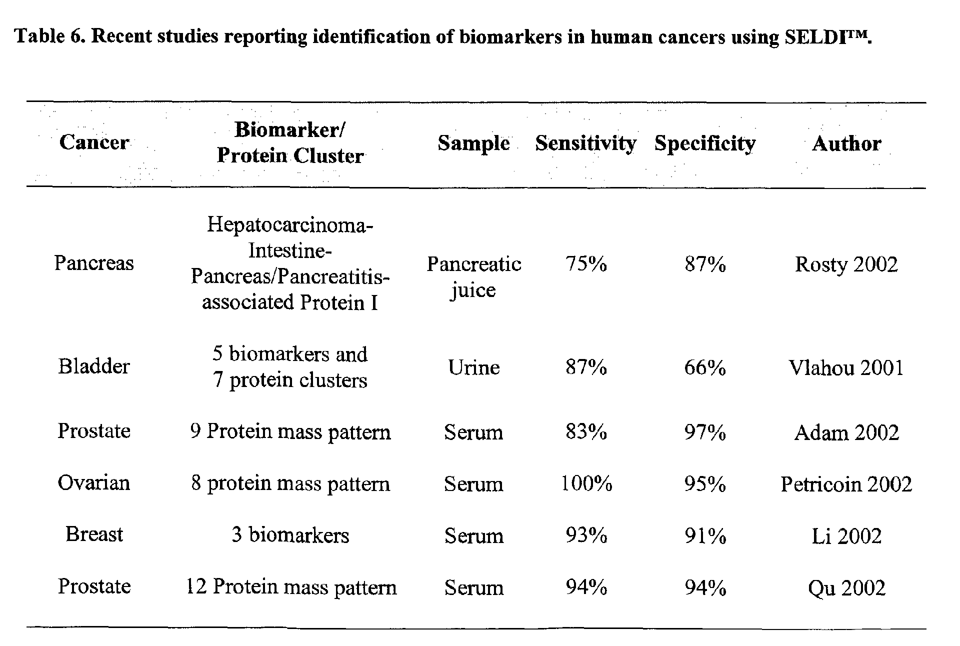

## Claims

1. A method for the differential diagnosis of an epithelial cancer and/or an acute and chronic inflammation of the epithelium, *in vitro,* comprising:
a) obtaining a test sample from a subject,
b) contacting test sample with a biologically active surface under specific binding conditions
c) allowing the biomolecules within the test sample to bind said biologically active surface,
d) detecting bound biomolecules using a detection method, wherein the detection method generates a mass profile of said test sample,
e) transforming the mass profile into a computer readable form, and
f) comparing the mass profile of e) with a database containing mass profiles specific for healthy subjects, subjects having precancerous lesions, subjects having epithelial cancer, subjects having metastasised epithelial cancers, or subjects having an acute and chronic inflammation of the epithelium,
wherein said comparison allows for the differential diagnosis of a subject as healthy, having a precancerous lesion, having an epithelial cancer, having a metastasised epithelial cancer and/or an acute and chronic inflammation of the epithelium.

2. The method of claim 1, wherein the database is generated by
a) obtaining biological samples from healthy subjects, subjects having precancerous lesions, subjects having epithelial cancer, subjects having metastasised epithelial cancers, and subjects having an acute and chronic inflammation of the epithelium,
b) contacting said biological samples with a biologically active surface under specific binding conditions,
c) allowing the biomolecules within the biological samples to bind to said biologically active surface,
d) detecting bound biomolecules using a detection method, wherein the detection method generates mass profiles of said biological samples,
e) transforming the mass profiles into a computer-readable form,
f) applying a mathematical algorithm to classify the mass profiles in e) as specific for healthy subjects, subjects having precancerous lesions, subjects having epithelial cancer, subjects having metastasised epithelial cancers, and subjects having an acute and chronic inflammation of the epithelium.

3. The method of claim 1, wherein the biomolecules are **characterized by**:
a) diluting a sample 1:5 in a denaturation buffer consisting of 7 M urea, 2 M thiourea, 4% CHAPS, 1% DTT, 2% Ampholine, at 0° to 4°
b) further diluting said sample 1:10 with a binding buffer consisting of 0.1 M Tris-HCl, 0.02% Triton X-100, pH 8.5 at 0° to 4°
c) contacting the sample with a biologically active surface comprising positively charged quaternary ammonium groups
d) incubating of the treated sample with said biologically active surface for 120 minutes under temperatures between 20 and 24°C at pH 8.5,
e) and analysing the bound biomolecules by gas phase ion spectrometry.

4. The method of claim 1, wherein the detection method is mass spectrometry.

5. The method of claim 4 wherein the method of mass spectrometry is selected from the group of matrix-assisted laser desorption ionization/time of flight (MALDI-TOF), surface enhanced laser desorption ionisation/time of flight (SELDI-TOF), liquid chromatography, MS-MS, or ESI-MS.

6. The method of claims 1, wherein the biologically active surface comprises an adsorbent selected from the group of quaternary ammonium groups, carboxylate groups, groups with alkyl or aryl chains, groups such as nitriloacetic acid that immobilize metal ions, or proteins, antibodies, or nucleic acids.

7. The method of claim 1, wherein the mass profiles comprise a panel of one or more differentially expressed biomolecules.

8. The method of claim 7, wherein, wherein the biomolecules are selected from a group having the apparent molecular mass of 1516 Da ± 8 Da, 1535 Da ± 8 Da, 2020 Da ± 10 Da, 2022 Da ± 10 Da, 2050 Da ± 10 Da, 3946 Da ± 20 Da, 4104 Da ± 21 Da, 4154 Da ± 21 Da, 4298 Da ± 21 Da, 4360 Da ± 22 Da, 4477 Da ± 22 Da, 4867 Da ± 24 Da, 4958 Da ± 25 Da, 4968 Da ± 25 Da, 5474 Da ± 27 Da, 5491 Da ± 27 Da, 5650 Da ± 28 Da, 6449 Da ± 32 Da, 6876 Da ± 34 Da, 7001 Da ± 35 Da, 7969 Da ± 40 Da, 8232 Da ± 41 Da, 8711 Da ± 44 Da, 10665 Da ± 53 Da, 12471 Da ± 62 Da, 12504 Da ± 63 Da, 12669 Da ± 63 Da, 13989 Da ± 70 Da, 15959 Da ± 80 Da, 16164 Da ± 81 Da, 17279 Da ± 86 Da, 17406 Da ± 87 Da, 17630 Da ± 88 Da, or 18133 Da ± 91 Da.

9. A method for the identification of differentially expressed biomolecules wherein the biomolecules of any of claims 1-8 are proteins, comprising:
a) chromatography and fractionation,
b) analysis of fractions for the presence of said differentially expressed proteins and/or fragments thereof, using a biologically active surface,
c) further analysis using mass spectrometry to obtain amino acid sequences encoding said proteins and/or fragments thereof, and
d) searching amino acid sequence databases of known proteins to identify said differentially expressed proteins by amino acid sequence comparison.

10. The method of claim 9, wherein the method of chromatography is selected from high performance liquid chromatography (HPLC) or fast protein liquid chromatography (FPLC).

11. The method of claim 9, wherein the mass spectrometry used is selected from the group of matrix-assisted laser desorption ionization/time of flight (MALDI-TOF), surface enhanced laser desorption ionisation/time of flight (SELDI-TOF), liquid chromatography, MS-MS, or ESI-MS.

12. A method for the differential diagnosis of an epithelial cancer and/or an acute and chronic inflammation of the epithelium, *in vitro,* comprising detection of one or more differentially expressed biomolecules wherein the biomolecules are polypeptides, comprising:
a) obtaining a test sample from a subject,
b) contacting said sample with a binding molecule specific for a differentially expressed polypeptide identified in claims 9-11,
c) detecting the presence or absence of said polypeptide(s),
wherein the presence or absence of said polypeptide(s) allows for the differential diagnosis of a subject as healthy, having a precancerous lesion, having an epithelial cancer, having a metastasised epithelial cancer and/or an acute and chronic inflammation of the epithelium.

13. A kit for the diagnosis of an epithelial cancer comprising the method of claim 1 and further comprising a denaturation solution, a binding solution, a washing solution, a biologically active surface comprising an adsorbent, and instructions to use the kit.

14. A kit for the diagnosis of an epithelial cancer comprising the method of claim 12, and further comprising a solution, binding molecule, detection substrate, and instructions to use the kit.

15. The method of any one of claims 1-14, wherein the epithelial cancer is of breast, lung, gastrointestinal, prostate, ovarian, cervical, endometrial, and/or other cancers of epithelial origin.

16. The method of any one of claims 1-14, wherein the test sample is a blood, blood serum, plasma, nipple aspirate, urine, semen, seminal fluid, seminal plasma, prostatic fluid, excreta, tears, saliva, sweat, biopsy, ascites, cerebrospinal fluid, milk, lymph, or tissue extract sample.

17. The method of any one of claims 1-14, wherein the biological sample is a blood, blood serum, plasma, nipple aspirate, urine, semen, seminal fluid, seminal plasma, prostatic fluid, excreta, tears, saliva, sweat, biopsy, ascites, cerebrospinal fluid, milk, lymph, or tissue extract sample.

18. The method of any one of claims 1-14, wherein the subject is of mammalian origin.

19. The subject of claim 18, wherein the subject is of human origin.
